(19) Europäisches Patentamt
European Patent Office
Office européen des brevets

(11) Publication number: **0 332 104 A2**

(12) **EUROPEAN PATENT APPLICATION**

(21) Application number: 89103888.7

(51) Int. Cl.4: C12N 15/00

(22) Date of filing: 06.03.89

The applicant has filed a statement in accordance with Rule 28 (4) EPC (issue of a sample only to an expert). Accession numbers of the deposits: ATCC 67628, 40426, 40427, 40326, 40526, 40527, 40528 and 40535.

(30) Priority: 08.03.88 US 165667
06.02.89 US 305566

(43) Date of publication of application:
13.09.89 Bulletin 89/37

(84) Designated Contracting States:
AT BE CH DE ES FR GB GR IT LI LU NL SE

(71) Applicant: CIBA-GEIGY AG
Klybeckstrasse 141
CH-4002 Basel(CH)

(72) Inventor: Ryals, John
415 Dunhill Circle
Durham, NC 27713(US)
Inventor: Montoya, Alice

deceased(US)
Inventor: Harms, Christian
1412 Gray Bluff Trail
Chapel Hill, NC 27514(US)
Inventor: Duesing, John
Gatternweg 18
CH-4125 Riehen(CH)
Inventor: Sperisen, Christoph
Landskronstrasse 4
CH-4056 Basel(CH)
Inventor: Meins, Fred
Leimgrubenweg 52
CH-4125 Riehen(CH)
Inventor: Payne, George
804 B7 Park Ridge Road
Durham, NC 27713(US)

(74) Representative: Zumstein, Fritz, Dr. et al
Bräuhausstrasse 4
D-8000 München 2(DE)

(54) **Chemically regulatable DNA sequences and genes and uses thereof.**

(57) The present invention provides chemically regulatable DNA sequences capable of regulating transcription of an associated DNA sequence in plants or plant tissues, chimeric constructions containing such sequences, vectors containing such sequences and chimeric constructions, and transgenic plants and plant tissues containing these chimeric constructions. Also provided are a novel signal peptide sequence, genes which code for this sequence, and newly identified PR protein genes. The chimeric constructions and transgenic plants and plant tissues may be used in an assay for new chemical regulators.

EP 0 332 104 A2

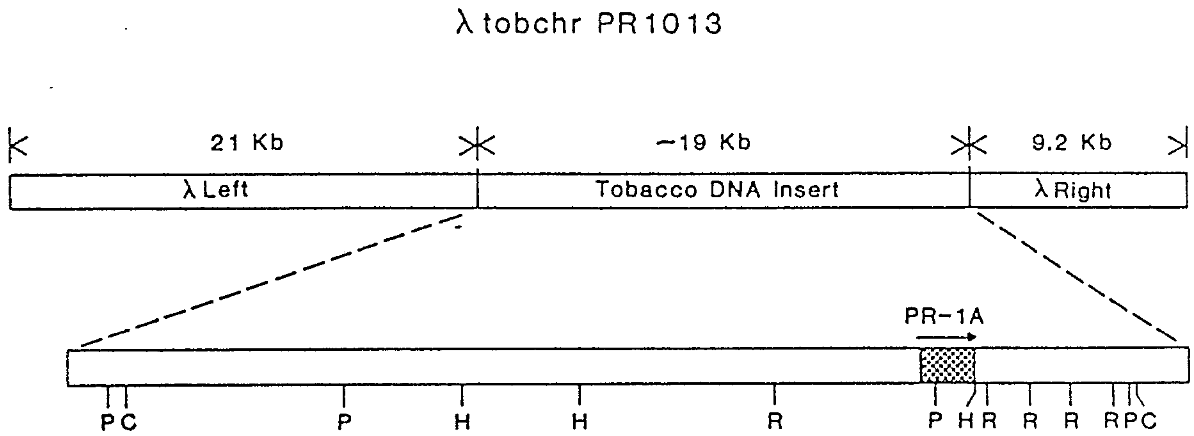
λ tobchr PR1013
21 Kb
~19 Kb
9.2 Kb
λ Left
Tobacco DNA Insert
λ Right
PR-1A
P C
P
H
H
R
P H R
R
R
R P C

FIGURE 1

# CHEMICALLY REGULATABLE DNA SEQUENCES AND GENES AND USES THEREOF

## FIELD OF THE INVENTION

The present invention relates to the chemical regulation of genetic materials and to novel materials that can be so regulated. In particular, it relates to non-coding DNA sequences which, in the presence of chemical regulators, regulate the transcription of other DNA sequences in plants.

## BACKGROUND OF THE INVENTION

Advances in recombinant DNA technology coupled with advances in plant transformation and regeneration technology have made it possible to introduce new genetic material into plant cells, plants or plant tissue, thus introducing new traits, e.g., phenotypes, that enhance the value of the plant or plant tissue. Recent demonstrations of genetically engineered plants resistant to pathogens (EP-A 240 332 and EP-A 223 452) or insects (Vaeck, M. et al., Nature 328, 33 (1987)) and the production of herbicide tolerant plants (DeBlock, M. et al., EMBO J. 6, 2513 (1987)) highlight the potential for crop improvement. The target crops can range from trees and shrubs to ornamental flowers and field crops. Indeed, it is clear that the "crop" can be a culture of plant tissue grown in a bioreactor as a source for some natural product.

In some cases it will be desirable to control the time and/or extent of the expression of introduced genetic material in plants, plant cells or plant tissue. An ideal situation would be the regulation of expression of an engineered trait at will via a regulating intermediate that could be easily applied to field crops, ornamental shrubs, bioreactors, etc. This situation can now be realized by the present invention which is directed to, among other things, a chemically regulatable chimeric gene expression system comprising a chemically regulatable, non-coding DNA sequence coupled, for example, to a gene encoding a phenotypic trait, such that the expression of that trait is under the control of the regulator, e.g. such that expression from the regulated gene is determined by the presence or absence of a chemical regulator. This system is the first demonstration of the concept of chemical regulation of chimeric gene expression in plants or plant tissue. As such it enables the production of transgenic plants or plant tissue and the control of traits expressed as a function of a chemical regulator.

### A. GENERAL OVERVIEW OF PLANT TRANSFORMATION TECHNOLOGY

Various methods are known in the art to accomplish the genetic transformation of plants and plant tissues (i.e., the stable introduction of foreign DNA into plants). These include transformation by *Agrobacterium* species and transformation by direct gene transfer.

#### 1. *Agrobacterium*-mediated Transformations

*A. tumefaciens* is the etiologic agent of crown gall, a disease of a wide range of dicotyledons and gymnosperms, that results in the formation of tumors or galls in plant tissue at the site of infection. *Agrobacterium*, which normally infects the plant at wound sites, carries a large extrachromosomal element called the Ti (tumor-inducing) plasmid.

Ti plasmids contain two regions required for tumorogenicity. One region is the T-DNA (transferred-DNA) which is the DNA sequence that is ultimately found stably transferred to plant genomic DNA. The other region required for tumorogenicity is the *vir* (virulence) region which has been implicated in the transfer mechanism. Although the *vir* region is absolutely required for stable transformation, the *vir* DNA is not actually transferred to the infected plant. Transformation of plant cells mediated by infection with *Agrobacterium tumefaciens* and subsequent transfer of the T-DNA alone have been well documented. See, for example, Bevan, M.W. and Chilton, M-D., Int. Rev. Genet. 16, 357 (1982).

After several years of intense research in many laboratories, the *Agrobacterium* system has been developed to permit routine transformation of a variety of plant tissue. Representative tissues transformed in this manner include tobacco, tomato, sunflower, cotton, rapeseed, potato, soybean, and polplar. While the host range for Ti plasmid transformation using *A. tumefaciens* as the infecting agent is known to be very large, tobacco has been a host of choice because of its ease of manipulation.

Agrobacterium rhizogenes has also been used as a vector for plant transformation. That bacterium, which incites hairy root formation in many dicotyledonous plant species, carries a large extrachromosomal element called an Ri (root-inducing) plasmid which functions in a manner analogous to the Ti plasmid of A. tumefaciens. Transformation using A. rhizogenes has developed analogously to that of A. tumefaciens and has been successfully utilized to transform, for example, alfalfa, Solanum nigrum L., and polplar.

2. Direct Gene Transfer

Several so-called direct gene transfer procedures have been developed to transform plants and plant tissues without the use of an Agrobacterium intermediate. In the direct transformation of protoplasts the uptake of exogenous genetic material into a protoplast may be enhanced by use of a chemical agent or electric field. The exogenous material may then be integrated into the nuclear genome. The early work was conducted in the dicot tobacco where it was shown that the foreign DNA was incorporated and transmitted to progeny plants, see e.g. Paszkowski, J. et al., EMBO J. 3, 2717 (1984); and Potrykus, I. et al., Mol. Gen. Genet. 199, 169 (1985).

Monocot protoplasts have also been transformed by this procedure in, for example, Triticum monococcum, Lolium multiflorum (Italian ryegrass), maize, and Black Mexican sweet corn.

Alternatively exogenous DNA can be introduced into cells or protoplasts by microinjection. A solution of plasmid DNA is injected directly into the cell with a finely pulled glass needle. In this manner alfalfa protoplasts have been transformed by a variety of plasmids, see e.g. Reich, T.J. et al., Bio/Technology 4, 1001 (1986).

A more recently developed procedure for direct gene transfer involves bombardment of cells by microprojectiles carrying DNA, see Klein, T.M. et al., Nature 327, 70 (1987). In this procedure tungsten particles coated with the exogenous DNA are accelerated toward the target cells, resulting in at least transient expression in the example reported (onion).

B. REGENERATION OF TRANSFORMED PLANT TISSUE

Just as there is a variety of methods for the transformation of plant tissue, there is a variety of methods for the regeneration of plants from plant tissue. The particular method of regeneration will depend on the starting plant tissue and the particular plant species to be regenerated. In recent years it has become possible to regenerate many species of plants from callus tissue derived from plant explants. The plants which can be regenerated from callus include monocots, such as corn, rice, barley, wheat and rye, and dicots, such as sunflower, soybean, cotton, rapeseed and tobacco.

Regeneration of plants from tissue transformed with A. tumefaciens has been demonstrated for several species of plants. These include sunflower, tomato, white clover, rpeseed, cotton, tobacco, and polplar. The regeneration of alfalfa from tissue transformed with A. rhizogenes has also been demonstrated. Plant regeneration from protoplasts is a particularly useful technique, see Evans, D.A. et al., in: Handbook of Plant Cell Culture, Vol. 1, MacMillan Publ. Co., 1983, p. 124. When a plant species can be regenerated from protoplasts, then direct gene transfer procedures can be utilized, and transformation is not dependent on the use of A. tumefaciens. Regeneration of plants from protoplasts has been demonstrated for rice, tobacco, rapeseed, potato, eggplant, cucumber, poplar, and corn.

The technologoical advances in plant transformation and regeneration technology highlight the potential for crop improvement via genetic engineering. There have been reports of genetically engineered tobacco and tomato plants which are resistant to infections of, for example, tobacco mosaic virus (TMV) and resistant to different classes of herbicides. Insect resistance has been engineered in tobacco and tomato plants.

C. CELL CULTURES

The potential for genetic engineering is not limited to field crops but includes improvements in ornamentals, forage crops and trees. A less obvious goal for plant biotechnology, which includes both genetic engineering and tissue culture applications, is the enhanced production of a vast array of plant-derived chemical compounds including flavors, fragrances, pigments, natural sweeteners, industrial feedstocks, antimicrobials and pharmaceuticals. In most instances these compounds belong to a rather broad metabolic group, collectively denoted as secondary products. Plants may produce such secondary products to ward off potential predators, attract pollinators, or combat infectious diseases.

Plant cell cultures can be established from an impressive array of plant species and may be propagated in a bioreactor. Typical plant species include most of those that produce secondary products of commercial interest. It has been clearly demonstrated in a number of agriculturally important crop plants that stable genetic variants arising from the tissue culture of plant somatic cells (somaclonal variation) can be induced and selected. Numerous advantages flow from plant tissue culture production of secondary compounds. These include (1) the possibility of increased purity of the resultant product, (2) the conversion of inexpensive precursors into expensive end products by biotransformation, and (3) the potential for feeding substrate analogs to the culture to create novel compounds.

D. ADVANTAGES OF CONTROLLED GENE EXPRESSION

Whether the target of genetic engineering of plants is a field crop, ornamental shrub, flower, tree or a tissue culture for use in a bioreactor, a principal advantage to be realized is the control of expression of the chimeric gene so that it is expressed only at the appropriate time and to the appropriate extent, and in some situations in particular parts of the plant. For example, in order to achieve a desirable phenotype the chimeric gene may need to be expressed at levels of 1% of the total protein or higher. This may well be the case for fungal resistance due to chimeric chitinase expression or insect resistance due to increased proteinase inhibitor expression. In these cases the energy expended to produce high levels of the foreign protein may result in a detriment to the plant whereas, if the gene were expressed only when desired, for instance when a fungal or insect infestation is imminent, the drain on energy, and therefore yield, could be reduced.

Alternatively, the phenotype expressed by the chimeric gene could result in adverse effects to the plant if expressed at inappropriate times during development. For example, if the chimeric gene product were a plant hormone that induced pod abscission, early expression could bring about abscission of the fruit from the plant before the seed had matured, resulting in decreased yield. In this case it would be much more advantageous to induce the expression of this type of gene to a time when pod abscission is preferred, or least injurious to the plant.

For tissue in culture or in a bioreactor the untimely production of a secondary product could lead to a decrease in the growth rate of the culture resulting in a decrease in the yield of the product. Therefore, it would be advantageous to allow the culture to grow without expressing the secondary product and then induce the chimeric gene at an appropriate time to allow for an optimized expression of the desired product.

In view of considerations like these, as well as others, it is clear that control of the time, extent and/or site of expression of the chimeric gene in plants or plant tissues would be highly desirable. Control that could be exercised easily in a field, a greenhouse or a bioreactor would be of particular commercial value.

E. KNOWN REGULATABLE GENE EXPRESSION SYSTEMS IN PLANTS

Several plant genes are known to be induced by various internal and external factors including plant hormones, heat shock, chemicals, pathogens, lack of oxygen and light. While few of these systems have been described in detail, in the best characterized, an increased accumulation of mRNA leads to an increased level of specific protein product.

As an example of gene regulation by a plant hormone, abscissic acid (ABA) has been shown to induce the late embryogenesis abundant mRNAs of cotton, see Galau, G.A. et al., Plant Mol. Biol. 7, 155 (1986). In another example, gibberellic acid (GA3) induces malate synthase transcripts in castor bean seeds and alpha-amylase isozymes in barley aleurone layers, see Rodriguez, D. et al., Plant Mol. Biol. 9, 227 (1987); Nolan, R.C. et al., Plant Mol. Biol. 8, 13 (1987).

The regulation of heat shock protein genes of soybean has been studied in detail. Treatment of plants for several hours at 40° C results in the de novo synthesis of several so-called heat shock proteins (Key, J. et al., Proc. Natl. Acad. Sci. USA, 78, 3526 (1981)). Several such genes have been isolated and their regulation studied in detail. The expression of these genes is primarily controlled at the level of transcription. The promoter of the hsp70 gene has been fused to the neomycin phosphotransferase II (NptII) gene and the chimeric gene has been shown to be induced by heat shock (Spena, A. et al., EMBO J. 4, 2736 (1985)) albeit at a lower level than the endogenous heat shock genes.

Another class of inducible genes in plants include the light regulated genes, most notably the nuclear encoded gene for the small subunit of ribulose 1,5-bisphosphate carboxylase (RUBISCO). Morelli, G. et al. (Nature 315, 200 (1985)) and Hererra-Estrella, L. et al. (Nature 310, 115 (1984)) have demonstrated that the 5′ flanking sequences of a pea RUBISCO gene can confer light inducibility to a reporter gene when attached in a chimeric fashion. This observation has been extended to other light inducible genes such as the chlorophyll a/b binding protein.

The alcohol dehydrogenase (adh) genes of maize have been extensively studied. The adh1-s gene from maize was isolated and a portion of the 5′ flanking DNA was shown to be capable of inducing the expression of a chimeric reporter gene (e.g., chloramphenicol acetyl transferase, CAT) when the transiently transformed tissue was subjected to anaerobic conditions (Howard, E. et al., Planta 170, 535 (1987)).

A group of chemicals known as safeners have been developed to protect or "safen" crops against potentially injurious applications of herbicides. While a general mechanism for the action of such compounds has not been fully developed, regulation of naturally regulatable genes by such compounds is one possible mechanism. It has recently been reported that higher levels of a glutathione-S-transferase (GST) are induced in maize treated with the safener 2-chloro-4-(trifluoromethyl)-5-methyl-thiazolecarboxylic acid benzyl ester, see Wiegand, R.C. et al., Plant Mol. Biol. 7, 235 (1986). Although the level of GST mRNA is elevated upon treatment with the safener, the mechanism leading to the elevation was not reported.

Many plants, when reacting hypersensitively toward various pathogens, are stimulated to produce a group of acid-extractable, low molecular weight pathogenesis-related (PR) proteins (Van Loon, L.C., Plant Mol. Biol. 4, 111 (1985)). Of particular interest, however, is the observation that these same PR proteins accumulate to high levels in plants treated with chemicals such as polyacrylic acid and acetylsalicylic acid (Gianinazzi, S. et al., J. Gen. Virol. 23, 1 (1974); White, R.F., Virology 99, 410 (1979)). The presence of PR proteins has been correlated with the induction of both a local and systemic resistance against a broad range of pathogens. An interspecific tobacco hybrid resistant to tobacco mosaic virus (TMV) was shown to express the PR-proteins constitutively (Ahl, P. et al., Plant Sci. Lett. 26, 173 (1982)). Furthermore, immunoprecipitation of in vitro translation products using mRNA from either TMV-infected or chemically treated tobacco (Cornelissen, B.J.C. et al., EMBO J. 5, 37 (1986); Carr, J.P. et al., Proc. Natl. Acad. Sci. USA 82, 7999 (1985)) indicated that the increased level of PR-protein was a result of RNA accumulation. Therefore, induction of PR protein genes by chemicals or pathogens provides a method to address the problem of chemically regulating gene expression in plants and plant tissue.

## SUMMARY OF THE INVENTION

The present invention includes (a) chemically regulatable DNA sequences, preferably in substantially pure form; (b) one or more chemically regulatable DNA sequences in combination with one or more parts but not all of any coding DNA sequences with which the regulatable sequences are associated in naturally occurring genes; (c) chimeric genes containing one or more chemically regulatable DNA sequences; (d) vectors containing sequences or genes of (a), (b) and/or (c); (e) plants, seeds, and plant tissue containing the chemically regulatable chimeric genes; and (f) chemical regulation of chemically regulatable chimeric genes in plant tissue. The invention further includes a signal peptide, a DNA sequence coding for the signal peptide, and the substantially pure forms of several naturally occurring chemically inducible genes.

The present invention further embraces several uses of the chemically regulatable DNA sequences: (a) regulation of chimeric genes in cells propagated in a bioreactor, (b) an assay for chemical regulators, (c) developmental regulation of the plant, (d) regulation of plant sterility and (e) regulation of chimeric and/or heterologous gene expression in a transformed plant. Other uses and advantages will be apparent from the following detailed description of the invention.

## BRIEF DESCRIPTION OF THE FIGURES

FIG. 1. Partial restriction endonuclease map of lambda tobchrPR1013. The 49 kb recombinant phage genome is depicted with the right and left arms of lambda designated. The 19 kb tobacco insert is enlarged below the lambda map. The location of the PR-1a gene (crosshatched box) and the direction of transcription (arrow) are designated. P = Pst, H = HindIII, C = ClaI, R = EcoRI.

FIG. 2. The construction of pBS-PR1013Cla from lambda tobchrPR1013 is shown. The 19 kb DNA fragment between the two ClaI sites was subcloned into the bluescript plasmid. C = ClaI, LGT agarose = low-gelling temperature agarose.

FIG. 3. The construction of pBS-PR1013Eco from lambda tobchrPR1013 is depicted. The 3.6 kb EcoRI fragment containing the PR-1A gene from lambda tobchrPR1013 is subcloned into Bluescript. R = EcoRI, LGT agarose = low-gelling temperature agarose.

FIG. 4. The construction of pBS-PR1013Eco from pBS-PR1013Cla is shown. The 3.6 kb EcoRI fragment containing the PR-1a gene is subcloned into the EcoRI site of the bluescript plasmid, C = ClaI, R = EcoRI, LGT = low-gelling temperature agarose.

FIG. 5. The construction of pBS-PR1013EcoΔPst from pBS-PR1013Eco is shown. The 600 bp PstI fragment is deleted from pBS-PR1013Eco. P = PstI, R = EcoRI, X = XhoI, S = SalI, LGT agarose = low-gelling temperature agarose.

FIG. 6. The construction of pBS-PR1013EcoΔPstΔXho from pBS-PR1013EcoΔPst is shown. The 2 kb XhoI fragment is deleted from the plasmid pBS-PR1013EcoΔPst. R = EcoRI, P = PstI, X = XhoI, S = SalI, LGT agarose = low-gelling temperature agarose.

FIG. 7. The construction of pCIB270 from pBI101.3 and pBS-PR1013EcoΔPstΔXho is illustrated. The PstI-XhoI fragment containing part of the PR-1a gene and 5′ flanking sequence is subcloned into SalI-BamHI digested pBI101.3. The XhoI and SalI sites are compatible and when ligated destroy both restriction sites. The PstI site is adapted to a BamHI site using a molecular adapter. X = XhoI, P = PstI, (X/S) = site of XhoI and SalI fusion.. Neither enzyme will now cut at this (X/S) site. LB = T-DNA left border, RB = T-DNA right border. Direction of transcription from the PR-la inducible region is shown by the arrow on pCIB270. The crosshatched area on pCIB270 represents the 3′-processing site of the NOS gene. The shaded area of pCIB270 represents the beta-glucuronidase gene. The stippled area of pCIB270 represents the neomycin phosphotransferase II gene. The striped area of pCIB270 represents the NOS promoter.

FIG. 8. The construction of M13mp18-PR1013EcoΔPstΔXho and M13mp19-PR1013EcoΔPstΔXho is shown. The PstI-Asp718 fragment containing part of the PR-1a coding sequence and the 5′ flanking sequence is subcloned from pBS-PR1013EcoΔPstΔXho into Asp718-PstI digested M13mp18 or 19. R = EcoRI, H = HindIII, P = PstI, K = KpnI (Asp718 is an isoschizomer of KpnI), LGT agarose = low-gelling temperature agarose.

FIG. 9. Flow diagram depicting the conversion of the ATG codon of PR-1a to a NcoI site. The single stranded DNA of M13mp18-PR1013EcoΔPstΔXho is shown with a solid line. The sequence TCATGG is converted to CCATGG by site-directed mutagenesis. K = KpnI, X = XhoI, B = BstEII, P = PstI.

FIG. 10. The construction of pCIB268 is shown. The BstEII-PstI fragment derived from the replicative form of M13mp18-PR1013EcoΔPstΔXho.Nco is subcloned into BstEII-PstI digested pBS-PR1013EcoΔPstΔXho to form pCIB268. X = XhoI, B = BstEII, N = NcoI, P = PstI.

FIG. 11. The construction of pCIB269 from pBS-GUS1.2 and pCIB268 is shown. X = XhoI, N = NcoI, P = PstI. The crosshatched box of pCIB269 depicts the GUS gene sequences, and the shaded box depicts the sequences derived from the PR-1a gene.

FIG. 12. The construction of pBS-GUS1.2 is shown. pBS-GUS1.2 is made via a three-way ligation from fragments derived from pRAJ265, pBI221.1 and pBluescript. S = SalI, R = EcoRI, N = NcoI.

FIG. 13. The construction of pCIB271 from pCIB269 and pCIB200 is shown. X = XhoI, N = NcoI, R = EcoRI, S = SalI, (S/X) = fusion of SalI and XhoI sites, LGT agarose = low-gelling temperature agarose.

FIG. 14. Restriction endonuclease map of pCIB219. This plasmid is constructed by adding an EcoRI/XhoI adapter to the pCIB269 XhoI/EcoRI fragment containing the PR-1 and GUS gene and ligating it into SalI restricted pCIB712.

FIG. 15. Restriction endonuclease map of pCIB272. This plasmid is constructed by ligating an Asp718I/BamHI fragment from pCIB282 containing a PR-1/GUS gene (-833 to +1 of PR-1a) into Asp718I/BamHI restricted pCIB200.

FIG. 16. Restriction endonuclease map of pCIB273. This plasmid is constructed by ligating an Asp718I/BamHI fragment from pCIB283 containing a PR-1/GUS gene (-603 to +1 of PR-1a) into Asp718I/BamHI restricted pCIB200.

FIG. 17. Restriction endonuclease map of pCIB1004. This plasmid is constructed by ligating a XhoI/NcoI fragment from pCIB269 (containing the PR-1a promoter) with the BT gene excised from pCIB10/35Bt(607) as a NcoI/BamHI fragment and SalI/BamHI digested pCIB710.

FIG. 18. Restriction endonuclease map of pCIB200/PR1-BT. This plasmid is constructed from pCIB1004 and pCIB200.

FIG. 19. Restriction endonuclease map of pCIB1207. A 5.8 kb XbaI fragment of the lambda genomic clone containing the Arabidopsis AHAS gene is cloned into XbaI restricted Bluescript.

FIG. 20. Restriction endonuclease map of pCIB1216. A 3.3 kb NcoI/XbaI fragment from pCIB1207 is cloned into pCIB269 which had been restricted with NcoI and XbaI to remove the GUS gene.

FIG. 21. Restriction endonuclease map of pCIB1233. A 4.2 kb KpnI/XbaI fragment is isolated from pCIB1216 and ligated into pCIB200 which had been restricted with KpnI and XbaI.

FIG. 22. Restriction endonuclease map of pBSGluc39.1/GUS. A 1462 bp fragment of pBSGluc39.1 is cloned into pBS-GUS1.2 which had been restricted with NcoI and KpnI.

FIG. 23. Restriction endonuclease map of pCIB200/Gluc39.1-GUS. A KpnI/XbaI fragment containing the $\beta$-glucanase promoter and the GUS gene is isolated from pBSGluc39.1/GUS and ligated into pCIB200 restricted with KpnI and XbaI.

FIG. 24. Restriction endonuclease map of pCIB200/Gluc39.1-BT. A KpnI/NcoI fragment from pCIB1004 containing the BT gene, a KpnI/NcoI fragment from pBSGluc39.1/GUS, and pCIB200 restricted with KpnI and treated with calf thymus alkaline phosphatase are ligated.

FIG. 25. Restriction endonuclease map of pBSGluc39.1/AHAS, constructed from a NcoI/XbaI fragment of pBSGluc39.1/GUS and a 3.3 kb NcoI/XbaI fragment from pCIB1207 containing the AHAS gene.

FIG. 26. Restriction endonuclease map of pCIB200/Gluc39.1-AHAS. A KpnI/XbaI fragment containing the $\beta$-glucanase promoter and the AHAS gene is isolated from pBSGluc39.1/AHAS and ligated into pCIB200 restricted with KpnI and XbaI.

FIG. 27. Restriction endonuclease map of pBSGluc39.3/GUS. A 1677 bp fragment of pBSGluc39.3 is cloned into pBS-GUS1.2 which had been restricted with NcoI and KpnI.

FIG. 28. Restriction endonuclease map of pCIB200/Gluc39.3-GUS. A KpnI/XbaI fragment containing the $\beta$-glucanase promoter and the GUS gene is isolated from pBSGluc39.3/GUS and ligated into pCIB200 restricted with KpnI and XbaI.

FIG. 29. Restriction endonuclease map of pCIB200/Gluc39.3-BT. A KpnI/NcoI fragment from pCIB1004 containing the BT gene, a KpnI/NcoI fragment from pBSGluc39.3/GUS, and pCIB200 restricted with KpnI and treated with calf thymus alkaline phosphatase are ligated.

FIG. 30. Restriction endonuclease map of pBSGluc39.3/AHAS, constructed from a NcoI/XbaI fragment of pBSGluc39.3/GUS and a 3.3 kb NcoI/XbaI fragment from pCIB1207 containing the AHAS gene.

FIG. 31. Restriction endonuclease map of pCIB200/Gluc39.3-AHAS. A KpnI/XbaI fragment containing the $\beta$-glucanase promoter and the AHAS gene is isolated from pBSGluc39.3/AHAS and ligated into pCIB200 restricted with KpnI and XbaI.

FIG. 32. Restriction endonuclease map of pCIB1208. A 5.8 kb XbaI fragment of the lambda genomic clone containing a mutated <u>Arabidopsis</u> AHAS gene is cloned into XbaI restricted Bluescript.

FIG. 33. Restriction endonuclease map of pCIB1230. A 3.3 kb NcoI/XbaI fragment from pCIB1208 is cloned into pCIB269 which had been restricted with NcoI and XbaI to remove the GUS gene.

FIG. 34. Restriction endonuclease map of pCIB1232. A 4.2 kb KpnI/XbaI fragment is isolated from pCIB1230 and ligated into pCIB200 which had been restricted with KpnI and XbaI.

FIG. 35. Restriction endonuclease map of pBSGluc39.1/AHAS-SuR, constructed from a NcoI/XbaI fragment of pBSGluc39.1/GUS and a 3.3 kb NcoI/XbaI fragment from pCIB1208 containing the AHAS gene.

FIG. 36. Restriction endonuclease map of pCIB200/Gluc39.1-AHAS-SuR. A KpnI/XbaI fragment containing the $\beta$-glucanase promoter and the AHAS gene is isolated from pBSGluc39.1/AHAS-SuR and ligated into pCIB200 restricted with KpnI and XbaI.

FIG. 37. Restriction endonuclease map of pBSGluc39.3/AHAS-SuR, constructed from a NcoI/XbaI fragment of pBSGluc39.3/GUS and a 3.3 kb NcoI/XbaI fragment from pCIB1208 containing the AHAS gene.

FIG. 38. Restriction endonuclease map of pCIB200/Gluc39.3-AHAS-SuR. A KpnI/XbaI fragment containing the $\beta$-glucanase promoter and the AHAS gene is isolated from pBSGluc39.3/AHAS-SuR and ligated into pCIB200 restricted with KpnI and XbaI.

## BRIEF DESCRIPTION OF THE SEQUENCES

Sequence 1 discloses the genomic DNA sequence of the 2 kb pair fragment between the XhoI and BglII sites of the tobacco PR-1a gene. The arrow at about nucleotide 903 indicates the transcriptional start site, and the vertical arrows marked 207 and 313 identify the ends of two independent cDNA clones. The polypeptide sequence encoded by the coding portion of the gene is also disclosed.

Sequence 2 discloses the genomic DNA sequence of the tobacco PR-1′ gene and the amino acid sequence of the polypeptide which is coded by the coding region of the gene.

Sequence 3 discloses the cDNA sequence of the cucumber PR chitinase and the amino acid sequence of the polypeptide which is coded by the coding region of the gene.

Sequence 4 discloses the cDNA sequence of the major form of the tobacco PR-R gene and (4a) discloses both, the sequence (4) and the amino acid sequence of the polypeptide that is coded by the coding region of the gene.

Sequence 5 discloses the genomic DNA sequence of the tobacco basic β-1,3 glucanase gene contained in the clone pBSGluc39.1.

Sequence 6 discloses the genomic DNA sequence of the tobacco basic β-1,3-glucanase gene contained in the clone pBSGluc39.3.

Sequence 7 discloses the cDNA sequence of the tobacco PR-Q contained in the plasmid pBScht15.

Sequence 8 discloses the DNA sequence of an isolated cDNA contained in the plasmid pBSGL6e. The cDNA encodes the acidic form of β-1,3-glucanase.

## DETAILED DESCRIPTION OF THE INVENTION

The present invention describes the isolation, cloning and identification of DNA sequences which are capable of regulating the transcription in plant tissue of an associated DNA sequence where the regulation is dependent upon a chemical regulator. These DNA sequences can be utilized to construct chimeric genes in which the expression of the gene can be regulated by such regulators. The ability to regulate the expression of a chimeric gene in a transgenic plant by a chemical method is useful to obtain suitable expression of the phenotypic trait with minimal adverse effect on the growth and development of the plant. This regulation is important in the production of secondary products or other cloned products in plant tissue in culture or bioreactors. The regulation of the cloned sequence is also important in the regulation of other gene products by an anti-sense mechanism.

The expression of a given coding sequence at any specific time can be regulated by use of a chemical regulator, typically applied to the plant tissue. Genes involved in the control of distinct developmental transition stages can also be regulated by associating a chemically regulatable DNA sequence with an appropriate coding DNA sequence. In this manner the development of a plant can be halted at a specific stage until, or accelerated at a specific rate when, the level of a chemical regulator is increased or reduced.

The chemically regulatable DNA sequences can be utilized to drive the expression of foreign genes which, for example, confer herbicide resistance or tolerance (e.g., atrazine tolerance in soybean), confer insect resistance (e.g., Bacillus thuringiensis crystal protein in cotton) or require selective expression (such as male or female sterility). The chemically regulatable DNA sequences can also be utilized to drive the transcription of a DNA sequence which will control the expression of a second coding sequence by an anti-sense mechanism.

Accordingly, the present invention addresses a number of objectives:

a. As a principal objective, a chimeric gene whose expression in plant tissue is regulated by a chemical regulator.

b. Substantially pure chemically regulatable DNA sequences capable of controlling genetic activity in plant tissue of other DNA sequences in response to a chemical regulator.

c. Chemically regulatable DNA sequences in combination with part but not all of any coding DNA sequence with which they are associate in naturally occurring genes.

d. Vectors containing the chemically regulatable DNA sequences with or without other parts of naturally occurring genes in which they may occur.

e. Vectors containing the chimeric genes that are the principal object of the invention.

f. Plant tissue, plants and seeds derived from cells transformed by these vectors.

g. Process of chemically regulating the expression of DNA coding sequences associated with the chemically regulatable DNA sequences, for example to regulate a phenotypic trait.

h. Process of identifying new chemical regulators using chimeric genes of this invention and plant tissue containing them.

i. Signal peptide sequences in proteins encoded by chemically regulated genes.

j. Substantially pure pathogenesis-related protein genes.

Other objects are also discernible from the following description of the invention.

### Definitions

In order to provide a clear and consistent understanding of the specification and the claims, including the scope given to such terms, the following definitions are provided:

Anti-sense Mechanism:

A mechanism for gene regulation based on controlling the rate of translation of mRNA to protein due to the presence in a cell of a RNA molecule complementary to at least a portion of the mRNA being translated.

Associated DNA Sequence:

A DNA sequence whose cellular activity either (l) regulates the activity of another DNA sequence or (2) is regulated by another DNA sequence. This definition specifically embraces, but is not limited to, sequences which are physically adjacent in a continuous DNA strand or which are physically separated. Physical separation includes, for example, separation within the same DNA strand, location within different DNA strands, or discontinuous interspersed sequences (e.g., alternating regulatable and coding sequences) in one strand.

Chemically Regulatable DNA Sequence:

A DNA sequence which is capable of regulating the transcription of an associated DNA sequence where the regulation is dependent on a chemical regulator. The sequences may be of natural or synthetic origin.

Chemically Regulatable Gene:

A gene containing at least one non-coding chemically regulatable DNA sequence and at least one associated coding DNA sequence. The genes may be of natural, synthetic or partially natural/partially synthetic origin.

Chemical Regulator (for a chemically regulatable DNA sequence):

An elemental or molecular species which controls (e.g., initiates, terminates, increases or reduces), by direct or indirect action, the activity of a chemically regulatable DNA sequence in a system in which the chemical regulator is not normally found in an active form in an amount sufficient to effect regulation of transcription, to the degree and at the time desired, of a transcribable DNA sequence associated with the chemically regulatable DNA sequence. This terminology is intended to embrace situations in which no or very little regulator is present at the time transcription is desired or in which some regulator is present but increased or decreased regulation is required to effect more or less transcription as desired.

Thus, if the system containing the chemically regulatable DNA sequence is a plant, for example a transgenic plant, a chemical regulator is a species not naturally found in the plant in an amount sufficient to effect chemical regulation, and thus transcription of an associated gene, to the desired degree at the time desired.

By "direct action" it is intended that the chemical regulator action result from the direct interaction between the chemical regulator and the DNA sequence. By "indirect action" it is meant that the regulator action results from the direct interaction between the chemical regulator and some other endogenous or exogenous component in the system, the ultimate result of that direct interaction being activation or suppression of the activity of the DNA sequence. By "active form" it is intended that the chemical regulator be in a form required to effect control.

Chimeric Sequence or Gene:

A DNA sequence containing at least two heterologous parts, e.g., parts derived from naturally occurring DNA sequences which are not associated in their naturally occurring states, or containing at least one part that is of synthetic origin and not found in nature.

Coding DNA Sequence:

A DNA sequence which, when transcribed and translated, results in the formation of a cellular polypeptide.

Gene:

A discrete chromosomal region which is responsible for a discrete cellular product.

Non-coding DNA Sequence:

A DNA sequence which is not transcribed and translated resulting in the formation of a cellular polypeptide when associated with a particular coding DNA sequence. Thus, for example, a sequence that is non-coding when associated with one coding sequence may actually be coding when associated with another coding or non-coding sequence.

Phenotypic Trait:

An observable property resulting from expression of a gene.

Plant Tissue:

Any tissue of a plant in planta or in culture. This term includes, but is not limited to, whole plants, plant cells, plant organs, plant seeds, protoplasts, callus, cell cultures and any groups of plant cells organized into structural and/or functional units. The use of this term in conjunction with, or in the absence of, any specific type of plant tissue as listed above or otherwise embraced by this definition is not intended to be exclusive of any other type of plant tissue.

PR, or Pathogenesis-Related Proteins:

Proteins expressed in plants reacting hypersensitively towards pathogens. This term embraces, but is not limited to, the acidic and basic forms of tobacco PR-1a, PR-1b, PR-1c, PR-1', PR-2, PR-N, PR-O, PR-P, PR-Q, PR-S, PR-R proteins, the chitinase which is a basic counterpart of PR-P or PR-Q, and the beta-1,3-glucanase (glucan endo-1,3-β-glucosidase, EC 3.2.1.39) which is a basic counterpart of PR-2, PR-N or PR-O, and the pathogen-inducible chitinase from cucumber. A hypersensitive reaction is characterized by a local necrosis of the tissues immediately surrounding the infection site of the pathogen and a subsequent localization of the pathogen, which is in contrast to a sensitive reaction wherein the pathogen spreads throughout the plant. Pathogens are, for example, viruses or viroids, e.g. tobacco or cucumber mosaic virus, ringspot virus or necrosis virus, pelargonium leaf curl virus, red clover mottle virus, tomato bushy stunt virus, and like viruses, fungi, e.g. Phythophthora parasitica or Peronospora tabacina, bacteria, e.g. Pseudomonas syringae or Pseudomonas tabaci, or aphids, e.g. Myzus persicae. This list is not limiting in any respect.

Regulation:

The increasing (inducing) or decreasing (repressing) of the level of expression of a gene or the level of transcription of a DNA sequence. The definition is not intended to embrace any particular mechanism.

Substantially Pure DNA Sequence:

A DNA sequence isolated in substantially pure form from a natural or non-natural source. Such a sequence may occur in a natural system, for example, in bacteria, viruses or in plant or animal cells, or may be provided, for example, by synthetic means or as a cDNA sequence. Substantially pure DNA sequences are usely isolated in form of a vector comprising the intended DNA. Substantially pure means that other DNA sequences than the ones intended are present only in marginal amounts, for example less than 5%, less than 1%, or preferably less than 0.1%. Substantially pure DNA sequences and vectors containing them may be, and typically are, provided in solution, for example in aqueous solution containing buffers or in the usual culture media.

Substantial Sequence Homology:

Substantial sequence homology means close structural relationship between sequences of nucleotides or amino acids. For example, substantially homologous DNA sequences may be 80% homologous, preferably 90% or 95% homologous, and substantially homologous amino acid sequences may typically be 50% homomlogous, or more. Homology also includes a relationship wherein one or several subsequences of nucleotides or amino acids are missing, or subsequences with additional nucleotides or amino acids are interdispersed.

A. CHEMICALLY REGULATABLE DNA SEQUENCES

The present invention is concerned with non-coding DNA sequences which are capable of regulating, under the influence of a chemical regulator, the transcription of an associated DNA sequence in a plant or plant tissue. Specifically, the present invention embraces a non-coding DNA sequence capable of regulating the transcription of an associated DNA sequence in a plant or plant tissue wherin this regulation is dependent upon a chemical regulator. Preferably the DNA sequences exist in substantially pure form relative to the gene and genome in which they occur if they come from a natural source, or relative to a DNA mixture if they occur in a synthetic mixture.

Preferably the non-coding chemically regulatable DNA sequences of this invention are those which, when associated with a coding DNA sequence, regulate the expression of the coding sequence in plant tissue, the extent of regulation being dependent upon a chemical regulator. Such sequences can be synthesized de novo or derived (for example, isolated or cloned) from a naturally occurring chemically regulatable gene. The occurrence of chemically regulatable DNA sequences of the invention is not limited to plant tissue; i.e., the chemically regulatable DNA sequences may be derived from a variety of natural sources, e.g., bacterial, viral or animal sources. It may be isolated, for example, from the 5′ flanking region or from the 3′ flanking region of a naturally occurring chemically regulatable gene. Alternatively, the non-coding DNA sequence may be chemically synthesized or enzymatically synthesized as cDNA identical to, or having substantial sequence homology to, the isolated sequence. By cloning the chemically regulatable, non-coding DNA sequence, the sequence can be separated from other sequences which are adjacent to it in the naturally occurring gene. In this manner a substantially pure DNA sequence can be obtained.

In the context of the present invention, regulation embraces chemical regulators which are either inducing or repressing regulators. Examples of chemically repressible genes, that is, genes which are repressed by a repressing chemical, include, for example, genes like TrpR or AroH where the addition of the tryptophan repressor or tryptophan itself represses the expression from these genes. Many other genes exist that are regulated by this type of end-product repression and in each case the end-product acts as a chemical regulator that can be used to repress the expression of the gene. The present invention embraces the regulatable sections of such genes by themselves or as part of chimeric constructions which can be chemically regulated for transcription of an associated DNA sequence in a plant or plant tissue.

Examples of chemically inducible genes, that is, genes which are induced by an inducing chemical regulator, are the PR protein genes, especially the tobacco PR protein genes, for example the PR-1a, PR-1b, PR-1c, PR-1′, PR-Q, PR-R and PR-S genes, the cucumber chitinase gene, and the basic and acidic tobacco β-1,3-glucanase genes. In a particular aspect the present invention comprises a substantially pure DNA sequence which is, or has substantial sequence homology to, a non-coding chemically regulatable DNA sequence which is part of a naturally occurring chemically regulatable gene, for example of a naturally occurring chemically regulatable gene in a plant or plant tissue from a monocot, dicot or gymnosperm.

Preferably such a DNA sequence is capable of regulating the transcription of an associated DNA sequence in a plant or plant tissue wherein said associated DNA sequence is a coding sequence. The transcription of said DNA sequence may be regulated by a repressing chemical regulator or an inducing chemical regulator. DNA sequences particularly considered are those wherein the chemically regulatable gene is a PR protein gene, for example from a dicotyledonous plant, e.g. tobacco or cucumber. Most preferred are DNA sequences wherein the chemically regulatable gene is a tobacco PR-1a, PR-1b, PR-1c, PR-1′, PR-Q or PR-R gene, a cucumber chitinase gene, or a basic or acidic β-1,3-glucanase gene, in particular the tobacco PR-1a and PR-1′ gene, but also the cucumber chitinase gene and the basic and acidic tobacco β-1,3-glucanase genes. Foremost considered are DNA sequences wherein the chemically regulatable gene is a tobacco PR-1a or basic β-1,3-glucanase gene.

The chemically inducible DNA sequences of the preferred PR and related genes of the invention apparently occur in the non-coding sequences of the adjacent region 5′ flanking to the coding sequences. As a representative example, in a sequence isolated from an approximately 6500 base pair fragment of the tobacco PR-1a gene containing part of the coding region, the region with about 900 to about 1200 base pairs, naturally adjacent to the transcriptional start site, has been found to be chemically inducible. Inducibility is retained in a fragment thereof containing about 500 to about 700 base pairs naturally adjacent to the transcriptional start site. Most preferred are therefore DNA sequences which are located in the 5′ flanking region of said PR and related genes, for example in the 1200 base pairs adjacent to the transcriptional start site.

The invention further embraces a process for the preparation of a non-coding DNA sequence capable of regulating the transcription of an associated DNA sequence in a plant or plant tissue wherein this regulation is dependent upon a chemical regulator, and of DNA sequences having substantial homology to said non-coding sequences, characterized in that the DNA is isolated from a naturally occurring gene in substantially pure form or is synthesized chemically or enzymatically.

In particular, the invention embraces a process for the preparation of a substantially pure chemically regulatable DNA sequence from a chemically regulatable gene in a naturally occurring system containing that gene, which process comprises the steps of

(a) activating expression in said system of RNA from the chemically regulatable gene;

(b) isolating said RNA;

(c) differentially screening a genomic library for RNA generated from RNA isolated from said activated system as well as RNA generated from RNA isolated from a control system that is not activated;

(d) isolating a genomic clone;

(e) subcloning the chemically regulatable gene from said genomic clone; and

(f) isolating the desired chemically regulatable DNA sequence.

Moreover the invention embraces such a process wherein said system is a plant, which process comprises the steps of

(a) activating expression in said plant of polyA+ RNA from the chemically regulatable gene;

(b) isolating said polyA+ RNA;

(c) constructing a cDNA library from said polyA+ RNA;

(d) differentially screening said cDNA library with cDNA generated from RNA in a control plant that is not activated;

(e) isolating cDNA clones that are chemically regulatable;

(f) isolating a genomic clone from a genomic library of said plant using as a probe the cDNA clone of step (e);

(g) subcloning the chemically regulatable gene from said genomic clone; and

(h) isolating the desired chemically regulatable DNA sequence.

Preferred is such a process wherein said chemically regulatable gene is a chemically inducible gene, for example a PR protein gene, e.g. a tobacco PR-1a, PR-1b, PR-1c, PR-1′, PR-Q or PR-R gene, a cucumber chitinase gene, or a tobacco basic or acidic β-1,3-glucanase gene. Further preferred is such a process wherein said PR protein gene is activated in step (a) by a chemical inducer or a pathogen.

The invention further embraces substantially pure DNA prepared by the mentioned processes.

## B. CHEMICALLY REGULATABLE DNA SEQUENCES WITH PARTS OF NATURALLY OCCURRING CODING SEQUENCES

In addition to the entirely non-coding chemically regulatable DNA sequences described above in Part A, this invention also provides for the non-coding DNA sequence of a naturally occurring chemically regulatable DNA sequence in combination with part but not all of a coding sequence with which the regulatable sequence is associated in a naturally occurring gene. More specifically, the present invention embraces, preferably in substantially pure form, a DNA sequence which comprises a first DNA component sequence which is, or has substantial sequence homology to, a non-coding chemically regulatable DNA sequence of a naturally occurring chemically regulatable gene, this first component sequence being capable of regulating the transcription of an >iõ_öoassociated DNA sequence in a plant or plant tissue, wherein this regulation is dependent upon a chemical regulator, and a second DNA component sequence which is, or has substantial sequence homology, to part but not all of a transcribable DNA sequence with which the first component is associated in the naturally occurring chemically regulatable gene. The naturally occurring chemically regulatable gene may be of plant origin, for example occurring in a monocotyledonous or dicotyledonous plant, and may be regulated by a repressing or an inducing chemical regulator. The second DNA component sequence will typically be a coding sequence. A preferred second DNA sequence for the present invention is a sequence which codes for the signal peptide of any protein expressed by the naturally occurring chemically regulatable gene.

In particular the invention embraces such a DNA sequence comprising a first non-coding chemically regulatable DNA sequence and a second coding DNA sequence from a PR protein gene, for example a PR protein gene from a dicotyledonous plant, preferably from tobacco or cucumber such as a PR-1a, PR-1b, PR-1c, PR-1′, PR-Q, or PR-R gene, a chitinase gene, or a basic or acidic β-1,3-glucanase gene. Preferred is a DNA sequence from a PR protein gene wherein the transcription is regulated by an inducing chemical regulator. In particular the invention embraces a DNA sequence wherein the first non-coding DNA sequence is located in the 5′ flanking region of one of the mentioned PR protein genes, for example located in the approximately 2000 base pairs adjacent to the transcriptional start site of said PR protein gene. Most preferred is a DNA sequence comprising a preferred first non-coding sequence as mentioned above and a second coding DNA sequence coding for a signal peptide as mentioned above.

Most preferred are substantially pure DNA sequences as shown in "Sequence 1", "Sequence 2", "Sequence 5", and "Sequence 6", and substantially pure DNA sequences having substantial sequence homology to these "Sequences" 1, 2, 5 and 6. These "Sequences" are examples of chemically regulatable DNA sequences comprising a first non-coding and a second coding DNA sequence and are derived from the PR protein genes tobacco PR-1a, PR-1′, and from two forms of basic tobacco β-1,3-glucanase, respectively. "Sequence 1" shows the sequence of the representative PR-1a gene from tobacco. Nucleotides 1 to about 1150 are the non-coding, 5′ flanking chemically inducible DNA sequence and part of the PR-1a coding sequence which is naturally occurring in a tobacco plant. In this case the chemically inducible component sequence is adjacent to the coding sequence. Those nucleotides which code for the first thirty amino acids constitute the coding sequence for the signal peptide of the PR-1a protein. The coding sequence can be removed from the non-coding sequence to generate the non-coding, chemically inducible DNA sequence free of any coding sequence described above in Part A. Such removal can be accomplished by conventional techniques, such as restriction enzyme digestions.

The invention further embraces a method for the preparation of a DNA sequence which comprises a first DNA component sequence which is, or has substantial sequence homology to, a non-coding chemically regulatable DNA sequence of a naturally occurring chemically regulatable gene, this first component sequence being capable of regulating the transcription of an associated DNA sequence in a plant or plant tissue, wherein this regulation is dependent upon a chemical regulator, and a second DNA component sequence which is, or has substantial sequence homology, to part but not all of a transcribable DNA sequence with which the first component is associated in the naturally occurring chemically regulatable gene, characterized in that the DNA is isolated from a naturally occurring gene in substantially pure form or is synthesized chemically or enzymatically. Preferred are the particular processes mentioned in Part A, above, and the substantially pure DNA sequences obtained thereby.

## C. CHIMERIC GENES CONTAINING CHEMICALLY REGULATABLE DNA SEQUENCES

A further aspect of the present invention is a chimeric DNA sequence (chimeric gene) containing at least one chemically regulatable DNA sequence. Two types of such chimeric sequences are provided as examples. The simpler, or two-part type chimeric DNA sequence comprises a chemically regulatable DNA sequence and a transcribable DNA sequence such that the chimeric gene is capable of being expressed in plant tissue under the proper conditions of chemical regulation. More specifically, the invention embraces a chemically regulatable chimeric DNA sequence comprising a first non-coding, chemically regulatable DNA component sequence which is capable of regulating the transcription of an associated DNA sequence in a plant or plant tissue, wherein this regulation is dependent upon a chemical regulator, and a second DNA component sequence capable of being transcribed in a plant or plant tissue. The DNA component sequences may be derived from natural sources or be prepared synthetically.

The second DNA sequence may be transcribed as an RNA which is capable of regulating the expression of a phenotypic trait by an anti-sense mechanism. Alternatively, the second DNA sequence in the chimeric DNA sequence may be transcribed and translated, i.e. coded, in the plant tissue to produce a polypeptide resulting in a phenotypic trait. The chimeric gene is constructed such that the second DNA sequence is properly associated with, typically in an adjacent orientation to, the chemically regulatable DNA sequence to ensure transcription. Association is effected with conventional techniques.

Preferred is a chimeric DNA sequence comprising a first non-coding chemically regulatable DNA component and a second transcribable DNA component wherein the first non-coding DNA sequence is one of the preferred DNA sequences mentioned above under part A. The first DNA component sequence may be regulated by a repressing or by an inducing chemical regulator. A particular sequence of the invention is a chimeric DNA sequence wherein the first DNA component sequence has substantial sequence homology to a chemically regulatable DNA sequence in a naturally occurring chemically regulatable gene from a plant.

Preferred is a chimeric DNA sequence wherein the first DNA component sequence is, or has substantial sequence homology to, a chemically regulatable DNA sequence in a naturally occurring chemically regulatable gene from a plant and the second DNA component sequence is a coding sequence that is capable of being transcribed and translated to produce a polypeptide resulting in a phenotypic trait. Preferably this second DNA component sequence is adjacent to the first DNA coding sequence. Particularly preferred is such a chimeric DNA sequence wherein the first DNA component sequence is, or has substantial sequence homology to, the chemically inducible DNA sequence in a PR protein gene, for example a PR protein from a dicotyledonous plant, such as tobacco or cucumber. Examples thereof are mentioned above in Part A.

A second exemplified type of chimeric DNA sequence contains three DNA component sequences, originating from two or more sources. In the simplest embodiment this chimeric DNA sequence comprises the two-part DNA sequence as described above in Part B and a third DNA sequence originating from at least one different source. More specifically, this type of chimeric DNA sequence comprising a first DNA component sequence which is, or has substantial sequence homology to, a non-coding, chemically regulatable DNA sequence of a naturally occurring chemically regulatable gene, this first DNA component sequence being capable of regulating the transcription of an associated DNA sequence in a plant or plant tissue, wherein this regulation is dependent upon a chemical regulator, a second DNA component sequence which is, or has substantial sequence homology to, part but not all of a transcribable DNA sequence with which the first component is associated in the naturally occurring chemically regulatable gene, and a third DNA component sequence capable of being transcribed in a plant or plant tissue. Preferably, the naturally occurring chemically regulatable gene is a plant gene.

The second and third DNA component sequences will typically be coding sequences. The third DNA component may be derived from more than one natural or synthetic source. The first two DNA components will typically be natural in origin. If the origin is a plant, it may be a monocot, a dicot or a gymnosperm. In a preferred embodiment the second DNA sequence will include the nucleotide sequence which codes for the signal peptide of the chemically regulatable gene in which the first two DNA sequences occur. If the chemically regulatable DNA sequence is associated with part of the coding sequence of the gene from which it was derived, the third DNA sequence must not only be in the proper orientation, but must also be in the proper reading frame with the second DNA sequence. This orientation can be achieved by techniques well known in the art.

Preferred are chimeric DNA sequences wherein the first and the second DNA sequence components are those mentioned as preferred in Part B above. For example a preferred chimeric DNA sequence is one wherein the first DNA component sequence is, or has substantial sequence homology to, the chemically inducible DNA sequence in a PR protein gene from a dicotyledonous plant, fot example from tobacco or cucumber. Examples of PR protein genes are mentioned above.

The chimeric genes described above embrace a variety of possible constructions. A chemically regulatable non-coding sequence can be associated with a gene controlling flowering or fruit ripening; a gene effecting tolerance or resistance to herbicides or to many types of pests, for example fungi, viruses, bacteria, insects, nematodes, or arachnids; a gene controlling production of enzymes or secondary metabolites; male or female sterility; dwarfness; flavor; nutritional qualities; and the like. Using the present invention such traits can be enhanced by the farmer and gardener, which is no longer dependent on natural factors alone. A phenotypic trait of particular interest for control is the production of metabolites in tissue culture or a bioreactor.

A preferred chimeric DNA sequence is a two or three component sequence wherein the coding DNA component sequence codes for a phenotypic trait, for example a trait selected from the group consisting of tolerance or resistance to a herbicide, fungus, virus, bacterium, insect, nematode or arachnid; production of secondary metabolites; male or female sterility; and production of an enzyme or other reporter compound. Particularly preferred is a two or three component chimeric DNA sequence wherein the coding component sequence codes for tolerance or resistance to herbicides, for example codes for wild-type or herbicide resistant acetohydroxyacid synthase (AHAS), or wherein the coding component sequence codes for resistance to insects, for example codes for Bacillus thuringiensis endotoxin (BT).

If the chimeric sequence is to be used as an assay for chemical regulators, the phenotypic trait is preferably an assayable marker. Suitable markers include, but are not limited to, luciferase (LUX), chloramphenicol acetyltransferase (CAT), neomycin phosphotransferase (NPT), nopaline synthase (NOS), octopine synthase (OCS), beta-l,3-glucuronidase (GUS), acetohydroxyacid synthase (AHAS), and Bacillus thuringiensis endotoxin (BT). Preferred markers are beta-l,3-glucuronidase (GUS), acetohydroxyacid synthase (AHAS), and Bacillus thuringiensis endotoxin (BT).

A representative example of such a chimeric DNA sequence, described in detail in the examples, is a two-part chimeric DNA sequence which contains the 5′ flanking, non-coding sequence of the PR-1a gene. While one of the exemplified marker is the coding sequence for the GUS gene, any of the above mentioned markers could be used. The analogous three-part chimeric sequence contains part of the coding sequence of the PR-1a gene. These constructions are particularly useful because the effect of the chemical induction, i.e. beta-glucuronidase enzyme activity, is easily detectable in plant cells or extracts thereof. Other particular embodiments, for example those which comprise the non-coding sequence of one of the tobacco $\beta$-1,3-glucanase genes and those which comprise the coding sequence for wild-type or herbicide resistant acetohydroxyacid synthase or for Bacillus thuringiensis endotoxin, are described in Part L, Examples.

Preferred chimeric DNA sequence are two component or three component chimeric DNA sequences wherein the first DNA component sequence is the 5′ flanking region of the tobacco PR-1a gene and contains more than about 300, for example between 300 and 2000, preferably between 600 and 1000, base pairs adjacent to the transcriptional start site.

Further preferred is a chimeric DNA sequence comprising three components wherein the second DNA component sequence codes for a signal peptide, for example wherein the second DNA component sequence codes for a peptide which is, or has substantial sequence homology to, the signal peptide from a PR protein gene, preferably the PR-1a gene.

The invention further embraces a method for the preparation of a chemically regulatable chimeric DNA sequence comprising a first non-coding, chemically regulatable DNA component sequence which is capable of regulating the transcription of an associated DNA sequence in a plant or plant tissue, wherein this regulation is dependent upon a chemical regulator, and a second DNA component sequence capable of being transcribed in a plant or plant tissue, characterized in that the DNA component sequences are ligated. Likewise, the invention embraces a method for the prepartion of a chimeric DNA sequence comprising a first DNA component sequence which is, or has substantial sequence homology to, a non-coding, chemically regulatable DNA sequence of a naturally occurring chemically regulatable gene, this first DNA component sequence being capable of regulating the transcription of an associated DNA sequence in a plant or plant tissue, wherein this regulation is dependent upon a chemical regulator, a second DNA component sequence which is, or has substantial sequence homology to, part but not all of a transcribable DNA sequence with which the first component is associated in the naturally occurring chemically regulatable gene, and a third DNA component sequence capable of being transcribed in a plant or plant tissue, characterized in that the DNA component sequences are ligated concurrently or consecutively.

## D. VECTORS

Vectors, produced by standard techniques and comprising either the chemically regulatable DNA sequences described in part A or B or the chimeric DNA sequences described in Part C above, represent an additional feature of the invention. Vectors are recombinant DNA sequences which may be used for isolation and multiplication purposes of the mentioned DNA sequence and for the transformation of suitable hosts with these sequences. Preferred vectors for isolation and multiplication are plasmids which can be propagated in a suitable host microorganism, for example in E. coli. Preferred vectors for transformation are those useful for transformation of plant cells or of Agrobacteria. More particularly, if plant cells other than protoplasts are being transformed, then the preferred vector is a Ti-plasmid derived vector. For the direct DNA transfer into protoplasts, any of the mentioned vectors may be used. Appropriate vectors which can be utilized as starting materials are known in the art. Suitable vectors for transforming plant tissue and protoplasts have been described by deFramond, A. et al., Bio/Technology 1, 263 (1983); An, G. et al., EMBO J. 4, 277 (1985); Potrykus, I. et al., supra; Rothstein, S.J. et al., Gene 53, 153 (1987). In addition to these, many other vectors have been described in the art which are suitable for use as starting materials in the present invention.

The vectors which contain only the chemically regulatable DNA sequence as described in Part A or B above can be used as intermediates for the preparation of a vector containing the chimeric DNA sequence as described in Part C above. The insertion of an appropriate sequence, which is capable of transcription, into such an intermediate vector results in a vector comprising a chimeric DNA sequence of the invention that can then be used to transform the desired plant tissue, protoplast or other host. Alternatively, a chimeric DNA sequence can be prepared and inserted into a suitable vector which is then used to transform the desired plant tissue or other host.

The construction and multiplication of the vectors can be performed in a suitable host, for example, in E. coli. Suitable E. coli strains include HB101, JM83, DH1, DH5α, LE392 and the like. The vectors of the invention may be used as such in a direct gene transfer or a micro-injection technique. In certain instances it may be preferable to linearize the vector before use. Alternatively the vectors may be transferred to an Agrobacterium host. This transfer is accomplished by conventional techniques including biparental mating (Simon, R. et al., Bio/Technology 1, 74 (1983)), triparental mating (Ditta, G. et al., Proc. Natl. Acad. Sci. USA 77, 7347 (1980)) or transformation (Holsters, M. et al., Mol. Gen. Genet. 163, 181 (1978)). Suitable strains of Agrobacterium include but are not limited to A. tumefaciens LBA4404, CIB542 and C58Z707.

Preferred vectors are those comprising the preferred DNA sequences mentioned in Parts A, B and C above. Furthermore a preferred vector is one that is functional in plant cells or in Agrobacterium. Particularly preferred are the vectors described in Part L, Examples.

## E. PLANT TISSUES, PLANTS AND SEEDS

A further aspect of the invention are plant tissue, plants or seeds containg the chimeric DNA sequences described above. Preferred are plant tissues, plants or seeds containing those chimeric DNA sequences which are mentioned as being preferred.

The cells of plant tissue are transformed with the vectors described above by any technique known in the art, including those described in the references discussed above and by techniques described in detail in the examples which follow. These techniques include direct infection or co-cultivation of plants or plant tissue with Agrobacterium. A very suitable technique is the leaf disk transformation described by Horsch, R.B. et al., Science 225, 1229 (1985). Alternatively, the vector can be transferred directly, for example by electroporation, by microinjection or by transformation of protoplasts in the presence of polyethylene glycol (PEG) calcium chloride or in an electric field, as more fully described above.

The cells transformed may originate from monocotyledenous or dicotyledonous plants and may contain one or more of the chemically regulatable chimeric genes of this invention. Thus, genes which, for example, code for resistance or tolerance to herbicides and a variety of insect, viral, bacterial, fungal and other pests, for sterility, for size, for flowering and fruit ripening, are introduced in the plant tissue, and these cells or protoplasts ultimately regenerated into plants in which these traits can be controlled by manipulations with a chemical regulator. Alternatively cells can be propagated in tissue culture or in a bioreactor to produce enzymes or secondary matabolites. If an enzyme assay is desired, the coding section of the chimeric gene may, for example, comprise a LUX, CAT, NPT, NOS, OCS, GUS, AHAS or BT gene, as identified previously. Such chimeric genes containing a chemically inducible sequence from a PR gene are a preferred embodiment of the invention because of the ease of application of the regulator and the ease of detection of the enzyme product.

Following transformation, the transformed cell or plant tissue is selected or screened by conventional techniques. The transformed cell or plant tissue contains the chimeric DNA sequence discussed above and is then regenerated, if desired, by known procedures, including those described in the reference discussed above and in the examples which follow for both monocot and dicot plants. The species which can be regenerated by these techniques include, but are not limited to, maize, sunflower, rapeseed, clover, tobacco, carrot, cotton, alfalfa, rice, potato, eggplant and cucumber. The regenerated plants are screened for transformation by standard methods. Progeny of the regenerated plants is continuously screened and selected for the continued presence of the integrated DNA sequence in order to develop improved plant and seed lines. The DNA sequence can be moved into other genetic lines by a variety of techniques, including classical breeding, protoplast fusion, nuclear transfer and chromosome transfer.

F. ADVANTAGES AND USES

The present invention offers a number of advantages and uses stemming from the easily controlled regulatable expression in plants or plant tissue of the chimeric genes containing chemically regulatable DNA sequences. Regulation of genes acting by an anti-sense mechanism or of genes whose expression results in the production of a phenotypic trait is possible. Of particular importance are the ability to control the time and rate of gene expression and the ease of effecting this control, either uniformly throughout the plant or in localized parts of the plant.

Effecting the control may be accomplished simply by applying the chemical regulator to the plant tissue, or to the plant or part of the plant in such a manner and in such an amount to regulate the chimeric gene(s) whose expression in plant cells, plant tissues or plants is desired. For example, if the trait to be expressed is preferably expressed only in the leaves, then spraying or dusting the leaves at a time which optimizes that expression in the leaves, and before any migration to other parts of the plant, may accomplish that objective easily and efficiently.

Alternatively uniform expression throughout that part of the plant above ground may result from application to the entire plant (i.e., stem and both sides of the leaves). If expression in the roots is desired, application to the seeds or the soil around the seeds or roots is a possible method of regulation. Expression in a bioreactor is accomplished quite easily, for example, by applying the chemical regulator to the medium contacting the cells.

The ability to control the time, rate and/or gene expression of novel phenotypic traits in transgenic plants offers a number of useful advantages. For example, if tolerance by a detoxification mechanism to a herbicide or other pesticide is introduced into a plant, that trait can be maximized by proper timing of the the application of the chemical regulator. Thus, the regulator can be applied before, with or after application of a herbicide or other pesticide, depending on which method gives optimal tolerance.

It is also possible now to regulate the production of compounds whose biosynthesis is controlled by endogenous or foreign genes. Upon chemical induction the production of such products can be started at the desired time. The induced process could be a multi-step biosynthesis or a one-step conversion of a metabolite.

Another advantage of the present invention arises from the ability to regulate the developmental processes in plants at a desired time by the application of a regulating chemical. For example, the synchronization of plant development (germination, tillering, sprouting, flower formation, anthesis, fruit ripening, dry down, abscission etc.) can be accomplished. In addition, normal plant development can be prevented. This can be accomplished by introducing, for example, a toxin gene which would interfere with the desired developmental stage, a DNA sequence which would block the developmental stage through an anti-sense mechanism, or a gene whose expression blocks the transition to a new developmental stage and which can be chemically repressed to allow development to proceed. One suitable utility is the induction of male or female sterility for the controlled hybridization of crops.

Additional advantages of the present invention are novel assay procedures, preferably enzyme assay procedures. For example, identification of new chemical regulators can now be accomplished quite easily. Such assay methods involve the use of transformed plants or plant cells which contain a chimeric DNA sequence of this invention. A chemical is applied to the transformed host and the transcription of the transcribable DNA sequence is measured against a control; transcription is usually detected in the form of a translation product or as an effect of such a product. The assay may be performed in a variety of ways, but is typically carried out using whole, regenerated plants (by applying the chemical to the plant) or to cultured cells or tissue (by applying the chemical to the cells or tissue) and subsequently supplying a substrate for the enzyme activity. The product of enzyme activity is then detected by standard methods, e.g., spectrophotometric measurement.

In particular the invention embraces a process for identifying a chemical regulator which comprises transforming a host with a chimeric DNA sequence as described above in Part C or with a vector containing said chimeric DNA sequence, applying a putative chemical regulator to the transformed host, and measuring the expression of the phenotypic trait. A preferred process is such a process wherein the transformed host is plant tissue or plant cells. Further preferred is a process wherein the chimeric DNA sequence is a sequence mentioned above as being preferred, for example wherein the first DNA component sequence of said chimeric DNA is a chemically inducible sequence which is, or has substantial sequence homology to, a chemically inducible sequence of a PR protein gene, and wherein the phenotypic trait coded for by the chimeric DNA is an assayable enzyme marker.

Another feature of the invention is the development of novel methods to identify other chemically regulatable DNA sequences. A vector containing a putative chemically regulatable DNA sequence is prepared, for example, from a host-selectable marker and a selectable or assayable marker. Suitable selectable markers include antibiotic resistance genes and herbicide resistance genes. Representative antibiotic resistance genes includes those for hygromycin, kanamycin, chloramphenicol, bleomycin, puromycin, lincomycin, G418 and methotrexate. A particularly suitable gene for resistance to hygromycin is aminoglycoside phosphotransferase IV. Suitable vectors for the transformation of plants containing the hygromycin resistance gene have been described by Rothstein, S.J. et al., Gene 53, 153 (1987). Examples of herbicide resistance genes encode resistance to, for example, sulfonylureas, glyphosate, phosphinotricin and atrazine.

Suitable assayable markers include genes for enzymes, antigens, immunoglobulins and the like, as well as antibiotic resistance genes. Suitable enzyme markers include LUX, CAT, NPT, NOS, OCS, GUS, AHAS and BT. A particularly suitable enzyme is beta-1,3-glucuronidase (GUS) because of the ease of enzyme assay. The DNA sequence coding for the assayable marker can be inserted into the vector using conventional techniques.

The putative regulatable DNA sequence is typically inserted adjacent to the assayable marker gene so that the expression of the assayable marker is under the control of the putative DNA sequence. The vector is then used to transform plant tissue or another appropriate host. Transformed plant tissue or host is selected on the basis of the plant selectable marker, typically antibiotic resistance. A chemical regulator is then applied to the plant tissue or other host following selection or following regeneration of the transformed tissue. Induction or repression of the assayable marker following the application of the chemical regulator identifies the putative DNA sequence as one which is capable of regulating the transcription of an adjacent DNA sequence wherin the regulation is dependent on a chemical regulator. The assay can be performed on whole, regenerated or transformed plants, for example, by applying the chemical regulator to the leaves or other plant tissue, and measuring the expression of the assayable marker. Alternatively the assay can be performed on transformed callus tissue or other host in cell culture, by applying the putative chemical regulator to the callus or other culture and measuring the expression of the assayable marker.

In particular the invention embraces a process for identifying a chemically regulatable DNA sequence which comprises the steps of

(a) transforming a host with a putative chemically regulatable DNA sequence or a vector containing said sequence, a second DNA sequence which is a host-selectable gene marker and a third DNA sequence which codes for a phenotypic trait;

(b) applying a chemical regulator to said transformed host; and

(c) measuring the expression or selecting for change in expression of the phenotypic trait coded for by the third DNA sequence.

Preferred is such a process wherein said third DNA sequence is a host-selectable or an assayable gene marker, and a process wherein the second or third DNA sequence is a gene marker for herbicide resistance or antibiotic resistance, for example selected from the group consisting of hygromycin, kanamycin, chloramphenicol, bleomycin, puromycin, lincomycin and methotrexate resistance genes, e.g. an aminoglycoside phosphotransferase IV hygromycin resistance gene.

Also preferred is such a process wherein the putative chemically regulatable DNA sequence is associated with, and preferably adjacent to, the third DNA sequence. Further preferred is a process wherein the transformed host is plant tissue or plant cells.

Further preferred is such a process wherein the third DNA sequence codes for an assayable enzyme marker selected from the group consisting of luciferase (LUX), chloramphenicol acetyltransferase (CAT), neomycin phosphotransferase (NPT), nopaline synthase (NOS), octopine synthase (OCS), beta-l,3-glucuronidase (GUS), acetohydroxyacid synthase (AHAS), and Bacillus thuringiensis endotoxin (BT).

A further aspect of the invention are substantially pure chemically regulatable DNA sequence identified by the mentioned processes.

The present invention also provides a method for selecting transformed plant material. Plant material exposed to an exogenous DNA sequence containing a chemically regulatable DNA sequence for which a chemical regulator is known and a second DNA sequence for a phenotypic trait is treated with the regulator and expression of the phenotypic trait sought. Observation of the trait confirms that transformation of the putative transformants has indeed occurred. Typical phenotypic traits for purposes of this method include the host selectable markers and assayable markers previously described.

In particular the invention embraces a method for selecting transformed plant cells or tissue which have been subjected to transforming DNA comprising a first DNA component sequence that is chemically regulatable by a known chemical regulator and an associated second DNA sequence coding for a phenotypic marker, which process comprises the steps of (a) treating putative transformants with said known chemical regulator and (b) selecting transformants according to expression of the phenotypic trait coded for by the second DNA sequence. A preferred method is such a method wherein said phenotypic trait is antibiotic resistance, for example resistance to hygromycin, kanomycin, chloroamphenicol, bleomycin, puromycin, lincoymcin, G418 and methotrexate, or an assayable enzyme marker, for example selected from the group consisting of luciferase (LUX), chloramphenicol acetyltransferase (CAT), neomycin phosphotransferase (NPT), nopaline synthase (NOS), octopine synthase (OCS), beta-l,3-glucuronidase (GUS), acetohydroxyacid synthase (AHAS), and Bacillus thuringiensis endotoxin (BT).

## G. SIGNAL PEPTIDES

A signal peptide or a signal sequence is a special N-terminal amino acid sequence in a protein entering the endoplasmic reticulum. Such a sequence in eukaryotic cells typically contains about 15 to 40 amino acid residues, many of which are hydrophobic. The sequence is eventually cleaved from the mature protein.

In the context of the present invention the signal peptide of a chemically regulatable gene is useful in the construction of three-part chimeric constructions as described above. Such chimeric constructions contain a first chemically regulatable sequence, a second DNA sequence coding for a signal peptide in proteins, and a third sequence which codes for a phenotypic trait. Preferably the phenotypic trait is one that is easily detected, for example in an assay procedure. Inclusion in the chimeric construction of the DNA sequence which codes for a signal peptide allows the product expressed by the third DNA sequence to be directed away from the endoplasmic reticulum of the host cell to its ultimate target site.

Therefore, an additional feature of the invention is a signal peptide from the tobacco PR-1a protein, and a protein with an amino acid sequence having substantial sequence homology to this peptide. The amino acid sequence for that peptide is as follows:

MetGlyPheValLeuPheSerGlnLeuProSerPheLeuLeuValSerThrLeuLeuLeuPheLeuValIleSerHisSerCysArgAla.

The present invention also embraces the DNA sequence which codes for this peptide (see Sequence 1) and DNA sequences having substantial sequence homology to this sequence. As noted previously, the invention also embraces (1) DNA sequences containing the chemically regulatable sequence in combination with the sequence coding for the signal peptide and (2) three-part chimeric DNA sequences containing the chemically regulatable component, the coding section for a signal peptide and a DNA sequence that is transcribed, preferably with translation.

## H. NOVEL PR GENES

The present invention also embraces newly isolated and identified PR genomic DNA and cDNA sequences from genes denoted herein as PR-1a, PR-1′ and PR-R from tobacco, the pathogen-inducible chitinase from cucumber, PR-Q from tobacco, basic β-1,3-glucanase 39.1 and 39.3 from tobacco, acidic β-1,3-glucanase from tobacco, and DNA sequences having substantial sequence homology to these genes. The DNA sequences of these genes are presented in Sequences 1 through 8. The novel genes of Sequences 1, 2, 5 and 6 (PR-1a, PR-1′, and β-1,3-glucanase 39.1 and 39.3, respectively) also represent particular embodiments of non-coding chemically regulatable DNA sequences of the invention as mentioned above. The novel Sequences 3, 4, 7 and 8 code for the PR proteins cucumber chitinase, tobacco PR-R, tobacco PR-Q and the acidic form of tobacco β-1,3-glucanase. Details of the isolation and characterization of the genes are given in the examples.

## I. CHEMICAL REGULATORS

A chemical regulator is defined as a substance which regulates expression of a gene through a chemically regulatable DNA sequence under certain circumstances as indicated in the definition of the term. The substance, in ionic or neutral form, with or without solvating or other complexing molecules or anions, will usually be exogenous relative to the system containing the chemically regulatable gene at the time regulation is desired. The use of exogenous chemical regulators is preferred because of the ease and convenience of controlling the amount of regulator in the system. However, the invention also includes the use of endogenous regulators, e.g., chemicals whose activities or levels in the system are artificially controlled by other components in, or acting on, the system.

Chemical regulators include chemicals known to be inducers for PR proteins in plants, or close derivatives thereof. These include benzoic acid, salicylic acid, polyacrylic acid and substituted derivatives thereof; suitable substituents include lower alkyl, lower alkoxy, lower alkylthio and halogen. When applied to plant tissue, typically to the leaves of whole plants, increased levels of mRNA and PR proteins develop in the plant tissue.

An additional group of regulators for the chemically regulatable DNA sequences and chimeric genes of this invention is based on the benzo-1,2,3-thiadiazole structure and includes, but is not limited to, the following types of compounds: benzo-1,2,3-thiadiazolecarboxylic acid, benzo-1,2,3-thiadiazolethiocarboxylic acid, cyanobenzo-1,2,3-thiadiazole, benzo-1,2,3-thiadiazolecarboxylic acid amide, benzo-1,2,3-thiadiazolecarboxylic acid hydrazide, and derivatives thereof.

A preferred group of regulators includes, but is not limited to, benzo-1,2,3-thiadiazole-7-carboxylic acid, benzo-1,2,3-thiadiazole-7-thiocarboxylic acid, 7-cyanobenzo-1,2,3-thiadiazole, benzo-1,2,3-thiadiazole-7-carboxylic acid amide, benzo-1,2,3-thiadiazole-7-carboxylic acid hydrazide, and derivatives thereof.

Suitable derivatives encompass but are not limited to representatives of said types of compounds wherein the benzo-1,2,3-thiadiazole moiety is unsubstituted or substituted by small substituents normally used in aromatic ring systems of agrochemicals such as lower alkyl, lower alkoxy, lower haloalkyl, lower haloalkoxy, lower alkylthio, cyano, nitro and halogen. Suitable derivatives further encompass, but are not limited to, representatives of said benzo-1,2,3-thiadiazole compounds wherein either the carboxylic acid, the thiocarboxylic acid, the carboxylic acid amide or the carboxylic acid hydrazide functional group is unsubstituted or substituted by aliphatic, araliphatic or aromatic residues. Suitable residues encompass, but are not limited to, alkyl (especially lower alkyl), alkoxy (especially lower alkoxy), lower alkoxyalkyl, alkoxyalkoxyalkyl, cycloalkyl, cycloalkylalkyl, phenylalkyl (especially benzyl), naphthylalkyl, phenoxyalkyl, alkenyl, and alkinyl, wherein the alkyl part of the substituent is unsubstituted or substituted by hydroxy, halogen, cyano or nitro, and the aromatic part of the substituent is unsubstituted or substituted by small substituents normally used in aromatic ring systems in agrochemicals such as lower alkyl, lower alkoxy, lower haloalkyl, lower haloalkoxy, lower alkylthio, cyano, nitro and halogen.

Regulators based on the benzo-1,2,3-thiadiazole structure encompass all molecular systems capable of releasing the molecule actually acting as the regulator.

A preferred group of regulators based on the benzo-1,2,3-thiadiazole structure includes benzo-1,2,3-thiadiazolecarboxylic acid, alkyl benzo-1,2,3-thiadiazolecarboxylate in which the alkyl group contains one to six carbon atoms, and substituted derivatives of these compounds. Suitable substituents include lower alkyl, lower alkoxy, lower alkylthio and halogen. In particular, benzo-1,2,3-thiadiazole-7-carboxylic acid and its alkyl esters, e.g. methyl benzo-1,2,3-thiadiazole-7-carboxylate, are preferred inducers for the chimeric DNA sequences comprising chemically regulatable DNA sequences isolated from PR protein genes. The syntheses of the mentioned chemical regulators and their utility as biocides may be discerned from British Patent 1,176,799 and Kirby, P. et al., J. Chem. Soc. C 2250 (1970).

Derivatives of benzo-1,2,3-thiadiazole that may further be used as regulators according to the present invention are described in U.S. patent application Serial No. 234,241 filed August 18, 1988, which is hereby incorporated by reference.

Among the preferred species based on the benzo-1,2,3-thiadiazole structure there may be mentioned, for example, benzo-1,2,3-thiadiazole-7-carboxylic acid, methyl benzo-1,2,3-thiadiazole-7-carboxylate, n-propyl benzo-1,2,3-thiadiazole-7-carboxylate, benzyl benzo-1,2,3-thiadiazole-7-carboxylate, benzo-1,2,3-thiadiazole-7-carboxylic acid sec-butylhydrazide, and the like.

An additional group of regulators for the chemically regulatable DNA sequences of this invention is based on the pyridine carboxylic acid structure, such as the isonicotinic acid structure and preferably the haloisonicotinic acid structure. Preferred are dichloroisonicotinic acids and derivatives thereof, for example the lower alkyl esters. Suitable regulators of this class of compounds are, for example, 2,6-dichloroisonicotinic acid, and the lower alkyl esters thereof, especially the methyl ester.

A further aspect of the invention is therefore a process for regulating transcription of a chemically regulatable gene, which process comprises applying such a chemical regulator to plant tissue, plant or seed containing a chemically regulatable DNA sequence as described in Part A, B or C above. Preferred is such a process wherein the plant tissue, plant or seed contains a chemically regulatable DNA sequence mentioned above as being preferred.

The chemical regulators may be applied in pure form, in solution or suspension, as powders or dusts, or in other conventional formulations used agriculturally or in bioreactor processes. Such formulations may include solid or liquid carriers, that is, materials with which the regulator is combined to facilitate application to the plant, tissue, cell or tissue culture, or the like, or to improve storage, handling or transport properties. Examples of suitable carriers include silicates, clays, carbon, sulfur, resins, alcohols, ketones, aromatic hydrocarbons, and the like. If formulated as a conventional wettable powder or aqueous emulsion, the regulator formulation may include one or more conventional surfactants, either ionic or non-ionic, such as wetting, emulsifying or dispersing agents.

The regulators may also be applied to plants in combination with another agent which is desired to afford some benefit to the plant, a benefit related or unrelated to the trait controlled by any chimeric gene which is regulated by the regulator. For example, a regulator can be admixed with a fertilizer and applied just before the expression of a transgenic trait unrelated to fertilization is desired. Or it can be combined with a herbicide and applied to mitigate the effect of the herbicide at the time when such effect would otherwise be at a maximum.

As a liquid formulation the regulator may be applied as a spray to plant leaves, stems or branches, to seeds before planting or to the soil or other growing medium supporting the plant. Regulators can also be used in bioreactor systems, regulation being achieved by a single addition of regulator formulation to the reaction medium or by gradual addition over a predetermined period of time.

The regulator is applied in an amount and over a time sufficient to effect the desired regulation. A preferred regulator is one which shows no, or only minimal phytotoxic or other deleterious effect on the plant, plant tissue or plant cells to which it is applied in the amount applied.

Preferred DNA sequences among those described above in Part A or B and preferred chimeric DNA sequences among those described above in Part C are in particular those wherein the transcription is regulated by a chemical regulator mentioned above, for example selected from the group consisting of benzoic acid, salicylic acid, acetylsalicylic acid, polyacrylic acid and substituted derivatives thereof, or selected from the group consisting of benzo-1,2,3-thiadiazolecarboxylic acid, benzo-1,2,3-thiadiazolethiocarboxylic acid, cyanobenzo-1,2,3-thiadiazole, benzo-1,2,3-thiadiazolecarboxylic acid amide, benzo-1,2,3-thiadiazolecarboxylic acid hydrazide, and derivatives thereof, or dichloroisonicotinic acid or a derivative thereof. Most preferred are those DNA sequences wherein the transcription is regulated by the mentioned preferred chemical regulators, for example by benzo-1,2,3-thiadiazole-7-carboxylic acid, methyl benzo-1,2,3-thiadiazole-7-carboxylate, n-propyl benzo-1,2,3-thiadiazole-7-carboxylate, benzyl benzo-1 2,3-thiadiazole-7-carboxylate, benzo-1,2,3-thiadiazole-7-carboxylic acid sec-butylhydrazide, 2,6-dichloroisonicotinic acid, or methyl 2,6-dichloroisonicotinate, in particular methyl benzo-1,2,3-thiadiazole-7-carboxylate.

## J. DEPOSITS WITH ATCC

The following bacteria, plasmids and suspension culture have been deposited with the American Type Culture Collection:

E. coli HB101/pTJS75-Km, ATCC number 67628, deposited February 12, 1988;
plasmid pBS-PR1013Cla, ATCC number 40426, deposited on February 12, 1988;
plasmid pBS-PR1019, ATCC number 40427, deposited on February 12, 1988;
Zea mays suspension culture 6-2717-GC, ATCC number 40326, deposited on May 20, 1987;
plasmid pBS-Gluc39.1, ATCC number 40526, deposited on December 29, 1988;
plasmid pBS-Gluc39.3, ATCC number 40527, deposited on December 29, 1988;
plasmid pBScucchi/chitinase, ATCC number 40528, deposited on December 29, 1988;
plasmid pBSGL6e, ATCC number 40535, deposited on January 18, 1989.

## K. SUMMARY OF EXPERIMENTAL PROTOCOLS

The chimeric genes of the present invention contain chemically regulatable DNA sequences from any source, natural or synthetic, in combination with one or more transcribable DNA sequences; usually at least part of the transcribable sequence will be translated into a phenotypic trait, although the invention also embraces chimeric genes which operate through an anti-sense mechanism. While the following description and many of the examples are directed to chemically regulatable DNA sequences found naturally in a plant genome, this invention applies equally to such sequences which occur in other natural systems, for example, bacterial, viral and animal systems, since techniques are well known to isolate such sequences from their natural systems. Furthermore, sequences derived from synthetic or other non-natural systems are similarly embraced.

A chemically regulatable DNA sequence is isolated from the source in which it exists naturally or synthetically and, if necessary, characterized by conventional methods. If the DNA sequence is isolated from a naturally occurring gene, this gene is activated by an appropriate regulator, either by a chemical or other known regulator for the gene. RNA resulting from that activation is isolated and is used to construct a cDNA library. This library is used for differential screening using radiolabelled cDNA generated both from (1) RNA isolated from the activated system and (2) RNA isolated from a second system not activated by the regulator. This differential screening is a particular aspect of the present invention, as mentioned above. The clones containing the chemically regulated cDNA are then isolated and are preferably sequenced at this point. The cDNA clones are then used to identify and isolate a genomic clone from a genomic library which genomic clone comprises the chemically regulatable DNA sequence. This gene is preferably sequenced and the chemically regulatable DNA sequence is functionally mapped by subcloning fragments of this gene, ligating them to a reporter gene and evaluating their activity in transgenic plant material or in transient plant expression systems.

For the PR protein genes of tobacco a lambda genomic library is constructed using standard techniques, and clones comprising chemically regulatable DNA sequences are identified and purified. One of these clones is characterized. Restriction fragments carrying the chemically regulatable DNA sequence are identified; for the PR-1a gene these gene fragments include a ClaI fragment of about 20,000 base pairs, a 6500 base pair HindIII fragment, and a 3600 base pair EcoRI fragment. The HindIII fragment contains about 500 coding base pairs and about 6000 non-coding bases in the 5′ flanking region adjacent to the transitional start site. Some of these fragments are subcloned into plasmids for use as sources of DNA for further subcloning and full characterization, i.e., restriction mapping, DNA sequencing and identification of the transcriptional start site of the gene. For the PR-1a gene the 3600 base pair EcoRI fragment is directly subcloned from a lambda genomic clone and subsequently subcloned to a final clone of about 1200 base pairs flanked by XhoI and PstI sites. This clone contains a chemically inducible DNA sequence from the PR-1a gene and a portion of the adjacent coding region for that gene; this portion includes the coding sequence for the signal peptide of the PR-1a protein gene.

Likewise genomic clones are isolated which code for the basic form of beta-1,3-glucanase. Two clones named 39.1 and 39.3 are characterized, and restriction fragments comprising chemically regulatable DNA sequences identified.

Using the vectors of this invention, these clones can then be used for the preparation of chimeric genes containing three parts as discussed previously. These chimeric DNA sequences contain the chemically regulatable sequence, part of the transcribable DNA and a third DNA sequence from a foreign source. Alternatively the clones can be further manipulated, e.g., by site directed mutagenesis, to remove all or most of any coding fragment from the parent gene prior to attachment to a coding sequence from a foreign source to prepare a two-part chimeric gene as described above. In a preferred embodiment that part of the chimeric gene which is not the chemically regulatable sequence constitutes a reporter gene for an easily observed or detected phenotypic trait. The following examples illustrate the genes for beta-1,3-glucuronidase, wild-type and herbicide resistant acetohydroxyacid synthase, and Bacillus thuringiensis endotoxin, but a variety of other reporter genes can be envisioned as described above. In a further preferred embodiment the coding component DNA sequence of the chimeric gene codes for tolerance or resistance to herbicides or for resistance to insects. This embodiment is exemplified by the mentioned genes for acetohydroxyacid synthase and for Bacillus thuringiensis endotoxin.

The chimeric genes and vectors containing these genes can be introduced into plant cells by a variety of techniques which give rise to transformed cells, tissue and plants or to cell cultures useful in bioreactors. Several techniques are described in detail in the examples which follow, directed to both monocotyledons and dicotyledons. Some of these methods, included here for enabling purposes, are the subject of other patent applications, in particular the U.S. patent application Serial No. 056,506, filed May 29, 1987, and the U.S. patent application Serial No. 165,665, filed March 8, 1988.

The transformed plant material can then be treated with a chemical regulator and the expression of the phenotypic reporter gene observed and/or measured. This kind of system provides an easy screening device to identify the regulatory activity of particular chemicals. The invention also concerns an improved assay of beta-1,3-glucuronidase (GUS) enzymatic activity which allows the screening of a large number of samples with high precision and in a short period of time. In particular this assay comprises reaction of plant tissue extracts in samples of less than 0.5 ml containing 1.5 to 3 mM 4-methyl umbelliferyl glucuronide at around 37°C for 1 to 5 hours and determining the concentration of the fluorescent indicator released.

The quantitative determination of steady-state levels of RNA is an essential step in the analysis of gene expression. To that end a primer extension assay has been developed. This method of the invention involves labeling a gene specific oligonucleotide to high specific radioactivity, hybridizing the oligonucleotide to an RNA sample and extending the hybrid with reverse transcriptase. The extension products are separated on denaturing acrylamide gels and visualized by autoradiography. The benefits of this method over previous assays include ease of probe preparation, simplicity of the assay protocol and the time required to carry out the assay. This primer extension assay is as sensitive and quantitative as S1 mapping.

The primer extension assay as applied under the particular reaction conditions described below in the examples is optimized for the determination of the level of PR-1 mRNA in total RNA extracted from TMV infected tobacco leaves. The PR-1 mRNA is expressed as 1% of the mRNA in these leaves based on quantitative analysis of the primer extension data and the frequency of cDNA clones in a cDNA library derived from this RNA. The improvements of the method of the invention relative to those previously described comprise the labelling of the oligonucleotide to a high specific activity, the decreased molar amount of probe used in the assay (typically about 0.01 to 0.05 pmol), optimized hybridization and elongation time, and optimized nucleotide triphopsphate concentrations.

The invention therefore embraces a method for the determination of the amount of a specific RNA in a solution of RNA comprising

(a) labeling a RNA specific primer oligonucleotide of a length of between 12 and 18 nucleotides to high specific radioactivity,

(b) hybridizing the RNA with the labeled oligonucleotide at a concentration of between 0.1 and 20 nM for between 2 minutes and 24 hours at a temperature around 37° C,

(c) elongating the primer oligonucleotide with reverse transcriptase in the presence of nucleotide triphosphates at a concentration of between 0.003 and 1 mM for between 1 and 120 minutes, and

(d) separating and visualizing the elongation products with autoradiography.

## L. EXAMPLES

The following examples further illustrate the present invention. They are in no way to be construed as a limitation in scope and meaning of the claims.

Enzyme reactions are conducted in accordance with the manufacturer's recommended procedures unless otherwise indicated. The chimeric genes and vectors of the present invention are constructed using techniques well known in the art. Suitable techniques have been described in Maniatis, T. et al., Molecular Cloning, Cold Spring Harbor Laboratory, New York (1982); Methods in Enzymology, Volumes 68, 100, 101 and 118 (1979, 1983, 1983 and 1986); and DNA Cloning, Glover, D.M.. Ed. IRL Press, Oxford (1985). Medium compositions have been described in Miller, J.H., Experiments in Molecular Genetics, Cold Spring Harbor Laboratory, New York (1972), as well as the references previously identified.

| ABBREVIATIONS | |
|---|---|
| ATP | adenosine triphosphate |
| bp | base pair |
| CETAB | hexadecyltrimethylammonium bromide |
| 2,4-D | 2,4-dichlorophenoxyacetic aci |
| DTT | dithiothreitol |
| dicamba | 3,6-dichloro-2-methoxybenzoic acid |
| EDTA | ethylendiamine N,N,N′,N′-tetraacetic acid |
| kb | kilo base pair |
| MES | 2-(N-morpholino)ethanesulfonic acid |
| MU | 4-methyl umbelliferyl glucuronide |
| PEG | polyethylene glycol |
| picloram | 4-amino-3,5,6-trichloropicolinic acid |
| SDS | sodium dodecyl sulfate |
| TFA | trifluoroacetic acid |
| TMV | tobacco mosaic virus |
| Tris-HCl | tris(hydroxymethyl)methylamine hydrochloride |

MEDIA

SH-0 medium:

Medium of Schenk, R.U. et al., Can. J. Bot., 50 (1972) 199-204; without hormones. SH medium can be liquid or solidified with 0.8% agar or with 0.5% GelRite®. The medium is normally sterilized by heat in an autoclave at 230-250° F for 15-20 mins.

SH-30 medium:

SH-0 medium containing 30 µm Dicamba.

SH-45 medium:

SH-0 medium containing 45 µm Dicamba.

RY-2 medium:

Medium of Yamada, Y. et al., Plant Cell Reports, 5, 85-88 (1986).

OMS medium:

Medium of Murashige, T. and Skoog, F., Physiologia Plantarum 9, 473 (1968). The medium can be solidified with 0.8% agar or agarose or with 0.5% GelRite®.

TABLE I Composition of Media Used

| Media: | KM-8p [b,c,d] | N6 |
|---|---|---|
| Macroelements [a], microelements [a] and Fe-EDTA are as given in the literature: KM medium according to Kao, K.N. et al., Planta 126, 105-110 (1965); N6 medium according to Chu et al., Scientia Sinica 18, 659 (1975) | | |
| Organics and vitamins [e] [mg/l]: | | |
| biotin | 0.01 | |
| pyridoxine-HCl | 1.00 | 0.5 |
| thiamine-hcl | 10.00 | 0.1 |
| nicotinamide | 1.00 | |
| nicotinic acid | 0.10 | 0.5 |
| folic acid | 0.40 | |
| D-Ca-pantothenate | 1.00 | |
| p-aminobenzoic acid | 0.02 | |
| choline chloride | 1.00 | |
| riboflavin | 0.20 | |
| Vitamin B 12 | 0.02 | |
| glycine | 0.10 | 2.0 |
| Sugars and sugar alcohols [g/l]: | | |
| sucrose | 0.25 | 30.0 |
| glucose | 68.40 | |
| mannitol | 0.25 | |
| sorbitol | 0.25 | |
| cellobiose | 0.25 | |
| fructose | 0.25 | |
| mannose | 0.25 | |
| rhamnose | 0.25 | |
| ribose | 0.25 | |
| xylose | 0.25 | |
| myo-inositol | 0.10 | |

| | | |
|---|---|---|
| Final pH | 5.8 | 5.6 |
| Sterilization | filter | autoclaved |

Footnotes:

a Macroelements are usually made up as a 10 X concentrated stock solution, and microelements as a 1000 X concentrated stock solution.

b Citric, fumaric and malic acid (each 40 mg/l final concentration) and sodium pyruvate (20 mg/l final concentration) are prepared as a 100 X concentrated stock solution, adjusted to pH 6.5 with $NH_4OH$, and added to this medium.

c Adenine (0.1 mg/l final concentration), and guanine, thymidine, uracil, hypoxanthine and cytosine (each 0.03 mg/l final concentration) are prepared as a 1000 X concentrated stock solution, adjusted to pH 6.5 with $NH_4OH$, and added to this medium.

d The following amino acids are added to this medium using a 10 X stock solution (pH 6.5 with $NH_4OH$) to yield the given final concentrations: glutamine (5.6 mg/l), alanine, glutamic acid (each 0.6 mg/l), cysteine (0.2 mg/l), asparagine, aspartic acid, cystine, histidine, isoleucine, leucine, lysine, methionine, phenylalanine, proline, serine, threonine, tryptophan, tyrosine and valine (each 0.1 mg/l).

e Vitamin stock solution is normally prepared 100 X concentrated.

MATERIALS

Agarose:

Preparation and purification of agarose are described by Guiseley and Renn, "The Agarose Monograph", Marine Colloids Division FMC Corp., 1975. Agarose is one of the constituents of agar. Commercially available agar normally consists of a mixture of neutral agarose and ionic agaropectin with a large number of side groups. Usually a certain number of of side chains remains intact and determines the physicochemical properties of the agarose such as gel formation and melting temperature. Low-melting agarose, especially SeaPlaque® agarose, is a preferred solidifying agent.

Casein hydrolysate:

Casein Hydrolysate - Enzymatic Hydrolysate from bovine milk, Type 1, Sigma Co., PO. Box 14508, St. Louis, MO 63178, USA.

Cellulase RS:

Cellulase RS, Yakult Honsha Co. Ltd., 1.1.19 Higashi-Shinbashi, Minato-ku, Tokyo, 105 Japan.

GelRite®:

GelRite Gellan Gum, Scott Laboratories Inc., Fiskerville, R.I. 02823, USA.

GeneScreen Plus®:

Cat. No. NEF 976, NEN Research Products, 549 Albany St., Boston, MA 02118, USA.

IBI Random primer kit:

'Prime Time' random primer kit, International Biotechnologies Inc., PO. Box 1565, New Haven, CT 07606, USA.

Nalgene® filter:

Nalge Co., Division of Sybron Corp. Rochester, N.Y. 14602, USA.

Pectolyase Y-23®:

Seishin Pharmaceutical Co. Ltd., 4-13 Koami-cho, Nihonbashi, Tokyo, Japan.

Parafilm®:

Parafilm® laboratory film - American Can Co. Greenwich, CT 06830, USA.

SSC:

1.54 mM NaCl, 0.154 mM sodium citrate.

Spin column:

Sephadex® G25 prepacked column, Cat.No. 100402, Boehringer Mannheim Biochemicals, Piscataway, NJ, USA.

TAE buffer and TBE buffer:

Tris-acetate buffer and Tris-borate buffer, respectively - common buffers for electrophoresis, see Maniatis et al., supra.

1. GENERAL TECHNIQUES

This group of examples describes general manipulations used to carry out the following detailed examples.

EXAMPLE 1 Ligation in Agarose

Following restriction digestion of plasmid DNA and electrophoretic separation of the fragments on a low melting TAE gel, the bands containing appropriate fragments are precisely excised and heated to 65° C to melt the agarose. 2-5 µl are added to 15 µl water and the solution is left at 65° C for 10 minutes. This solution is cooled to 37° C and left for five minutes to equilibrate to temperature. 2 µl of 10 X ligase buffer (200 mM Tris, pH 8.0, 100 mM $MgCl_2$, 100 mM DTT, 10 mM ATP) are added along with 1 µl T4 DNA ligase (New England BioLabs), and this solution is allowed to solidify and incubate at 15° C overnight.

EXAMPLE 2 Transformation From Agarose

The agarose containing the appropriate DNA is melted by incubating at 65° C for 10 minutes. 10 µl of this solution are added to 30 µl of TE buffer (10 mM Tris pH 7.5, 1 mM EDTA), mixed and allowed to stand at room temperature. Frozen competent cells (E. coli strain DH5α) are placed on wet ice to thaw. The diluted DNA solution is added to 200 µl of cells and allowed to stand on ice for 20 minutes. The cells containing the DNA are then heat-shocked for 90 seconds at 41° C. The cells are then left at room temperature for 10 minutes. 0.8 ml of SOC medium (Hanahan, D., J. Mol. Biol. 166, 557-580 (1983)) is added and the culture is incubated at 37° C for one hour. 100 µl of the culture is plated on LB plates (Miller, supra) containing 100 µg/ml ampicillin (L-amp) and the plates are incubated overnight at 37° C. Positive colonies are picked and restreaked to a second L-amp plate and the plates are incubated overnight at 37° C.

EXAMPLE 3 Southern Blotting

3 µg of tobacco DNA is digested with various restriction enzymes under the conditions suggested by the supplier. The DNA is extracted with phenol, precipitated with ethanol and then resuspended in gel loading buffer (15% ficoll, 0.025% bromophenol blue, 10 mM EDTA, pH 8). Samples are loaded and electrophoresed on a 0.5% agarose gel at 5 V/cm until the bromophenol blue dye reaches the end of the gel. The DNA is transferred to Gene-Screen Plus (Dupont) using the alkaline transfer procedure as described by the supplier. Prehybridization, hybridization and washing are according to the manufacturer's recommendation. Hybridization is detected by autoradiography.

EXAMPLE 4 Moleoular Adaptors

A typical molecular adaptor is the sequence
5′-GGGATCCCTGCA-3′
for the conversion of a PstI site to a BamHI site. This molecule is synthesized on an Applied Biosystems Synthesizer using B-cyanoethylphosphoramidite chemistry and purified by reverse-phase HPLC. About 2 µg of this oligonucleotide is kinased according to Maniatis et al., supra p. 125. The oligonucleotide solution is heated to 65° C in a water bath and allowed to cool to room temperature over a period of about 30 minutes. An approximately 10-fold molar excess of this annealed adapter is added to the digested DNA along with 10 X ligase buffer, T4 DNA ligase, and an appropriate amount of water. A typical reaction is:

| | |
|---|---|
| DNA to be adapted: | 1-2 µl (~ 1 pmol) |
| Adapter: | 1 µl (~10 pmol) |
| 10 X ligase buffer: | 1 µl |
| T4 DNA ligase: | 1 µl |
| Water: | 5-6 µl |

This solution is incubated at 12-15° C for 30 minutes, and heated to 65° C for 30 minutes to inactivate the ligase. The salt concentration and volume are adjusted for the appropriate restriction digest and the adapted DNA is digested to expose the adapted "sticky end." Unincorporated adapters are removed either by electrophoresis on an agarose gel or by sequential isopropanol precipitations.

EXAMPLE 5 Primer Extension Mapping

A. Synthesis and 5′ End Labeling of Primers for Primer Extension

The following primer oligomers are synthesized using an Applied Biosystems Synthesizer and β-cyanoethylphosphoramidite chemistry:

PR-1: 5′-ATAGTCTTGTTGAGAGTT-3′

GUS: 5′-TCACGGGTTGGGGTTTCTAC-3′

AHAS: 5′-AGGAGATGGTTTGGTGGA-3′

BT: 5′-ATACGTTCTACTATCATAGT-3′

The oligonucleotides are purified by reverse-phase high pressure liquid chromatography (HPLC). 5 pmol of each oligo is kinased (Maniatis, T. et al., supra, at p. 125) using 200 µC of $^{32}$P-ATP (6000 Ci/mmol, 10 µCi/µl). After incubation at 37° C for 30 minutes, the reaction is diluted to 100 µl, extracted with phenol/chloroform and then precipitated three times with 50 µg carrier RNA. The final precipitate is resuspended in 1 X reverse- transcriptase buffer (50 mM Tris-HCl, pH 7.5, 40 mM KCl, 3 mM $MgCl_2$) at a concentration of 2 nM. The specific activity of the labeled oligonucleotide is determined to be about 3 X $10^6$ Cvcpm/pmol.

B. Total RNA Preparation

Total RNA is prepared essentially as described by Lagrimini, L.M. et al., Proc. Natl. Acad. Sci. USA 84, 7542 (1987). Tissue is ground under liquid nitrogen in a mortar and pestle. The ground tissue is added to grinding buffer Lagrimini et al., supra) using 2.5 ml per gram tissue. An equal volume of phenol is then added and the emulsion is homogenized in a Brinkman polytron. A one-half volume of chloroform is added and the emulsion is gently mixed for 15 minutes. The phases are separated by centrifugation and the aqueous phase is removed. RNA is precipitated by the addition of sodium acetate to 0.3 M and 2.5 volumes ethanol. The precipitate is collected by centrifugation and resuspended in 2 ml sterile water. Lithium chloride is added to a final concentration of 3 M and left at 4° C overnight. The precipitate is collected by centrifugation and the pellet is washed with ice-cold 80% ethanol. The pellet is dried and resuspended in 500 µl sterile water. The concentration of this total RNA preparation is determined spectrophotometrically. Alternatively, RNA is extracted from callus as described above except that the callus tissue is cut into cubes approximately 3 mm in size, and added to pre-chilled mortars and pestles for grinding in liquid nitrogen prior to the polytron step.

C. Primer Extension

50 µg of total RNA is lyophilized in a 500 µl Eppendorf tube. The RNA is resuspended in 30 µl of radiolabeled probe solution and heated to 70° C for 10 minutes. The tube is slowly cooled to 37° C and allowed to incubate overnight. Without removing the tube from the 37° C water bath, 2 µl of 10 X reverse-transcriptase buffer (500 mM Tris-HCl, pH 7.5, 400 mM KCl, 30 mM $MgCl_2$), 1 µl 5 mg/ml bovine serum albumin, 5 µl 100 mM dithiothreitol, 5µl 10 X dNTPs (10 mM of each dNTP in $H_2O$), 3 µl $H_2O$, 2 µl RNasin (80 units), and 2 µl reverse transcriptase (400 units) are added and the reaction is incubated at 37° C for 30 minutes. To stop the reaction, 5 µl of 3 M sodium acetate, pH 5, and 150 µl absolute ethanol are added. The tube is left at -20° C for 30 minutes, the precipitate is collected by centrifugation, washed with 80% ethanol and allowed to air-dry. The precipitate is resuspended in 10-20 µl of loading dye (90% formamide, 0.05% bromophenol blue, 0.05% xylene cyanol, 1 mM EDTA) and the extension products are separated on a 6% sequencing gel (Maniatis, T. et al., supra). Extension products are visualized by autoradiography.

EXAMPLE 6 S1 Nuclease Mapping

The plasmid pBS-PR1013Cla is digested with SfaNI, dephosphorylated with calf intestinal phosphatase and kinased with $^{32}P$-ATP. Following phenol extraction and ethanol precipitation, the DNA is digested with BstEII and the 300 bp fragment from 750 to 1035 of Figure 1 is isolated after electrophoresis on a low gelling temperature agarose gel. The probe is resuspended in formamide hybridization buffer (Berk, A.J. et al., Cell 12, 721 (1977)) at a concentration of about 2 nM. The specific activity of the probe is about 5 X 10 Cvcpm/pmol.

Lyophilized, total RNA (50 µg) is dissolved in 30 µl of the probe solution, and the tubes are heated to 65° C for 10 minutes, then allowed to hybridize overnight at 48° C. S1 nuclease treatment and gel electrophoresis are essentially as described, using an S1 concentration of 400 units/ml and an incubation temperature of 30° C. The appropriate S1 nuclease concentration is determined in pilot experiments.

EXAMPLE 7 Mapping the Transcriptional Start Site

The transcriptional start site for the PR-1a gene is determined by a combination of S1 nuclease mapping and primer extension analysis. An autoradiogram of a primer extension experiment using either RNA isolated from TMV-infected leaves or an mpl9 subclone of the XhoI-PstI fragment as a template and a 17 base oligonucleotide complementary to positions 1025 to 1042 of the PR-1a sequence as a specific primer is examined. The primer itself is labeled at the 5′ phosphate, therefore the size of the extension product will be identical to the size of the corresponding band in the sequencing lanes. The appearance of two strong bands corresponding to positions 902 and 903 and a weak band at position 901 of the genomic clone suggests transcription initiating at either of these positions. However, primer extension analysis alone cannot be used to identify the 5′ end of a mRNA. For instance, the mRNA may contain a 5′ end that has been spliced from an upstream location.

To determine conclusively the 5′ end, high resolution S1 nuclease mapping is used in conjunction with primer extension. An SfaNI fragment is labeled at the 5′ end and digested with BstEII to yield a strand specific probe extending from position 759 to 1040. This probe is used to map the 5′ end of PR-1a transcripts in RNA isolated from TMV-infected tobacco leaves. A major band of 137 ±2 bases is found which corresponds to positions 901 to 905 of the genomic clone. In high resolution S1 experiments, where the digestion products are electrophoresed along with a size standard of sequencing reactions performed on the probe, three bands are visualized corresponding to positions 901, 902 and 903. These results confirm the primer extension analysis and place the 5′ end of the PR-1 mRNA at either position 901, 902 or 903. With regard to transcription initiation, one possible interpretation of these results is that RNA polymerase begins transcription at either base 901, 902 or 903 with more or less equal probability. However, since eukaryotic transcription favors initiation at an A, a more likely explanation for the apparent multiple 5′ ends is that the PR-1a mRNA begins at position 903 (an A) and the PR-1b and -1c mRNAs begin each at one of the other positions on their corresponding genes.

2. PROTEIN IDENTIFICATION AND CHARACTERIZATION

The PR proteins relevant to these examples are isolated, purified and sequenced, for the first time in some cases and in accordance with literature procedures in other, for the purpose of ultimately confirming the identities of their chemically inducible genes.

EXAMPLE 8 Purification of PR-1a and PR-1b Protein

Plants of Nicotiana tabaccum cv. Xanthi are grown in a glasshouse and infected when eight weeks old by gently rubbing the leaves with a suspension of a common strain (Ul) of TMV (0.5 µg/ml). Leaves are harvested seven days after infection and the intracellular fluid (ICF) is washed out of the leaves and collected according to Parent, J.G. et al., Can. J. Bot. 62, 564 (1984). 250 ml of ICF are concentrated to 50 ml by lyophilization and loaded on an Ultragel ACA54 column equilibrated with Tris-HCl, pH 8.0, and 1 mM EDTA. Eluates are analyzed by electrophoresis on 10% polyacrylamide gels. Fractions containing PR-1 proteins are pooled, lyophilized, resuspended in 3 ml water and then dialyzed overnight against water. This preparation is further purified by HPLC anion exchange chromatography on a TSK-DEAE 5PN column. The column is eluted with a 0-0.6 M NaCl gradient in 50 mM Tris-HCl, pH 8.0, 1 mM EDTA. Fractions are analyzed by polyacrylamide gel electrophoresis (PAGE). PR-1b elutes first from the column at 0.18 M NaCl and PR-1a elutes at 0.28 M NaCl. Fractions containing each protein are pooled, dialyzed against water and lyophilized. The purity of the PR-1a and PR-1b protein preparation is confirmed by reverse-phase HPLC using an Aquapore phenyl column (2.1 x 100 mm, Brownlee) and eluting with a linear acetonitrile/isopropanol (1:1) gradient (5-80%) in 0.1% TFA.

EXAMPLE 9 Protein Sequence Determination

Purified, lyophilized PR-1a protein is dissolved in 6 M guanidine-HCl containing 1 M Tris-HCl, pH 8.6, 10 mM EDTA. Dithiothreitol is added to 20 mM and 4-vinylpyridine is added to a final concentration of 50 mM. The sample is then incubated for 1.5 hours under nitrogen. The pyridylethylated material is desalted on HPLC using an Aquapore phenyl column (2.1 x 10 cm, Brownlee). The column is eluted with a linear, 5-80% gradient of acetonitrile/isopropanol (1:1) in 0.1% trifluoroacetic acid (TFA).

Automated Edman degradations are performed with an Applied Biosystems 470A gas-phase sequencer. Phenylthiohydantoin (PTH) amino acids are identified by an Applied Biosystems 120A PTH analyzer.

Cyanogen bromide cleavage is performed in situ according to Simpson, R.J. et al., Biochem. Intl. 8, 787 (1984). Digestion of PR-1 with pyroglutamate aminopeptidase (Boehringer Mannheim) is carried out according to Allen, G., Plant Sci. Lett. 26, 173 (1982).

PR-1a is digested with endoproteinase Lys-C (Boehringer Mannheim) in 0.1 M Tris-HCl, pH 8.5, for 24 hours at room temperature using an enzyme to substrate ratio of 1:10. Peptides are isolated by HPLC using an Aquapore C-8 column (1 x 22 cm, Brownlee) by a linear acetonitrile/isopropanol (1:1 ratio) gradient (0 to 60%) in 0.1% TFA.

Digestion of the N-terminal Lys-C peptide with trypsin (Cooper) is performed in 0.1 M ammonium bicarbonate, pH 8.2, containing 0.1 M calcium chloride for five hours at 37° C using an enzyme to substrate ratio of 1:100. The two peptides generated are separated on HPLC using the same conditions as with the Lys-C peptides.

EXAMPLE 10 Protein Sequence of PR-1a Peptides

A correlation of the DNA sequence of three cDNA clones with the PR-1a, -1b and -1c proteins is originally made by Cornelissen, B.J.C. et al., supra, based on a comparison of the published protein sequence data of three peptides derived from PR-1a (Lucas, J. et al., EMBO J. 4, 2745 (1985)) and the primary structure of the protein deduced from the cDNA clones. However, the cDNA clone designated as PR-1a is truncated at the 5′ end and can be compared to only two of the three peptides with a mismatch of one residue. The encoded amino acid sequence deduced from cDNA as prepared and analyzed above and the PR-1a cDNA sequence from Pfitzner, U.M. et al., supra, not only mismatch the published protein sequence at the tryptophan residue as reported, but also mismatch at three other positions of the amino terminal protein sequence. This anomaly places the identification of this clone as PR-1a in question. Therefore, the identity of the tobchrPR1013 clone as the PR-1a gene is verified by determining a large portion of the primary structure of the purified PR-1a protein by amino acid sequencing.

The amino acid sequence data for the PR-1a protein is compared to the amino acid sequences deduced from cDNA clones for PR-1a, -1b and -1c. The cDNA sequence data used in the analysis is from cDNA clones isolated above and sequences from Pfitzner, U.M. et al., supra, which agree at every position. Over 80% of the mature PR-1a protein sequences are determined and this sequence matches the deduced protein sequence for tobchrPR1013. When compared to the deduced amino acid sequence derived from putative PR-1b and PR-1c cDNA clones (Cornelissen, B.J.C. et al., supra), there are seven and six mismatches, respectively. Thus, the evidence clearly demonstrates that tobchrPR1013 is indeed a clone of the PR-1a gene.

## 3. PRODUCTION OF CLONES

This group of examples describes clones prepared as a result of gene isolation and identification of the chemically regulatable sequences and chimeric genes containing those sequences.

### EXAMPLE 11 Preparation of tobchrPR1013

Nuclei are isolated from leaves of Nicotiana tabaccum by first freezing 35 grams of the tissue in liquid nitrogen and grinding to a fine powder with a mortar and pestle. The powder is added to 250 ml of grinding buffer (0.3 M sucrose, 50 mM Tris, pH 8, 5 mM magnesium chloride, 5 mM sodium bisulfite, 0.5% NP40) and stirred on ice for 10 minutes. This mixture is filtered through six layers of cheesecloth, and the liquid is transferred to centrifuge bottles and subjected to centrifugation at 700 x g for 10 minutes. The pellets are resuspended in grinding buffer and recentrifuged. The pellets are again resuspended in grinding buffer and recentrifuged. The pellets are again resuspended in grinding buffer and this suspension is layered over a 20 ml cold sucrose cushion containing 10 mM Tris, pH 8, 1.5 mM magnesium chloride, 140 mM sodium chloride, 24% sucrose, 1% NP40. These tubes are centrifuged at 17,000 x g for 10 minutes. The pellets at this stage contain mostly nuclei and starch granules. High molecular weight DNA is isolated from the nuclei essentially according to Maniatis, T. et al., supra. The DNA is partially digested with MboI and ligated into the BamHI site of the EMBL3 cloning vector. Approximately 300,000 plaques are screened with a labeled PR-1b cDNA probe. Fifty positive clones are isolated and characterized. Positive plaques are purified and characterized by restriction analysis. These clones are classified into eight distinct groups by restriction mapping. One of the clones is identified as tobchrPR1013. A partial restriction map of tobchrPR1013 is shown in Figure 1. Isolation of DNA and DNA manipulations are essentially as described by Maniatis, T. et al., supra.

### EXAMPLE 12 Preparation of pBS-PR1013Cla

A ClaI fragment from the clone tobchrPR1013 is subcloned into the Bluescript plasmid (Stratagene Cloning Systems), resulting in pBS-PR1013Cla (see Figure 2). The 2 kb XhoI-BglII fragment from pBS-PR1013Cla is sequenced and positions 913 to 1711 (Sequence 1) are found to match perfectly the sequence of a cDNA clone clone for PR-1a isolated by Pfitzner, U.M. et al., supra. Possible rearrangements of the tobacco DNA during the cloning procedure are ruled out by analyzing predicted restriction fragments from genomic DNA. Based on sequence information and a restriction map of pBS-PR1013Cla, digestion of genomic tobacco DNA with either EcoRI, HindIII, XhoI + BglII, or HindIII + PstI should generate fragments of 3.2 kb, 6.7 kb, 2.0 kb or 6.4 kb, respectively which contain the PR-1a gene. To test this prediction, 3 µg of tobacco chromosomal DNA is digested with the appropriate enzymes and electrophoresed on a 0.7% agarose gel. The DNA is transferred to a nylon membrane and probed with a labeled XhoI-BStEII restriction fragment from the PR-1a gene. As a control, pBS-PR1013Cla DNA is digested and electrophoresed on the same gel. As predicted, the EcoRI, HindIII, XhoI + BglII, and HindIII + PstI digests produce bands of the expected molecular weight which comigrate with the control bands. Therefore, the DNA contained in the tobchrPR1013 clone represents a contiguous piece of DNA in the tobacco genome.

### EXAMPLE 13 Preparation of pBS-PR1013Eco

A. The plasmid pBS-PR1013Eco is constructed by subcloning the 3.6 kb EcoRI fragment containing the PR-1a gene from tobchrPR1013 (Example 9) into the unique EcoRI site of the bluescript plasmid. Bluescript plasmid (catalog no. 21203) is obtained from Stratagene Cloning Systems, La Jolla, California. The structure of pBS-PR1013Eco is confirmed by both restriction mapping and DNA sequencing. This construction of the plasmid is shown in Figure 3.

B. Alternatively, the plasmid pBS-PR1013Cla is digested with EcoRI and the 3.6 kb fragment containing the PR-1a gene is isolated. The pBluescript is digested with EcoRI, mixed with and ligated to the previously isolated 3.6 kb EcoRI fragment. This construction of the plasmid is shown in Figure 4.

EXAMPLE 14 Preparation of pBS-PR1013EcoΔPst

The plasmid pBS-PR1013Eco is digested with PstI and the DNA fragments separated on a 2.5% low-gelling temperature agarose gel. The large fragment containing the bluescript vector and a 3.0 kb fragment of the tobacco DNA are precisely excised and heated to 65°C to melt the agarose. 2 µl are added to 15 µl water and the DNA is ligated in agarose as described in Example 1. After overnight incubation, the agarose containing the DNA is then melted by incubating at 65°C for 10 minutes and then transformed from the agarose essentially as described in Example 2. A portion of the bacterial culture from each of six putative positive constructions is inoculated into 5 ml of LB medium (Miller, supra) containing 100 µg/ml ampicillin, and this culture is incubated at 37°C overnight. Cells are harvested by centrifugation in a clinical centrifuge at full speed for 10 minutes. The supernatant is removed and the cells are resuspended in 100 µl of 50 mM glucose, 25 mM Tris, pH 7.5, 10 mM EDTA, and the suspension is transferred to a 1.5 ml Eppendorf centrifuge tube. 25 µl of a freshly prepared 10 mg/ml lysozyme solution is added and the suspension is left on ice for 10 minutes. 200 µl of 0.2 N sodium hydroxide, 1% sodium dodecyl sulfate is then added and the suspension is mixed and allowed to stand on ice for two minutes. 200 µl of 2 M sodium acetate (pH 4.8) is added and the solution is allowed to sit on ice for 15 minutes. The resulting solution is subjected to centrifugation at full speed for 10 minutes in an Eppendorf centrifuge. The supernatant is removed to a fresh centrifuge tube and extracted with 400 µl phenol/chloroform (1:1). The aqueous phase is removed to a fresh tube and extracted with 400 µl chloroform. The aqueous phase from this extraction is removed to a fresh tube and DNA is precipitated by the addition of two volumes ethanol and storage at -20°C for 30 minutes. The precipitated DNA is collected by centrifugation in an Eppendorf microfuge for 15 minutes at full speed. The ethanol is removed and the pellets are washed with 80% ethanol. The ethanol is again removed and the pellets are allowed to air-dry. The DNA is resuspended in 100 µl of TE buffer. Constructions are verified by digestion with PstI. The parental plasmid pBS-PR1013Eco yields a 6 kb and ~650 bp band, the smaller of which is missing in the new constructions. One of these plasmids is selected as the plasmid pBS-PR1013EcoΔPst. The structure of this subclone is verified by sequence analysis. The construction of this plasmid is shown in Figure 5.

EXAMPLE 15 Preparation of pBS-PR1013EcoΔPstΔXho

A preliminary restriction map of pBS-PR1013Eco is established. An XhoI restriction site is located ~1200 bp upstream from the PstI site. The plasmid pBS-PR1013EcoΔPst is digested with XhoI and the fragments are separated on 0.9% low-gelling temperature agarose gel. Two fragments are visualized with sizes of 3.9 kb and 1.7 kb. The band containing the 3.9 kb fragment is carefully excised from the gel and ligated in agarose essentially as described above. The agarose is melted and the DNA transformed into competent E. coli strain HB101 as described above. Cells are plated onto LB-amp plates and incubated as described. One putative positive colony is inoculated into LB-amp media and DNA is isolated essentially as described. The structure of this new subclone pBS-PR1013EcoΔPstΔXho is verified by restriction analysis and DNA sequencing. Figure 6 shows the construction of this plasmid.

EXAMPLE 16 Preparation of pCIB270

Plasmid pBI101.3 (Catalog no. 6024.1) is obtained from Clonetech Labs, Palo Alto, California. This plasmid is first digested with BamHI and then with SalI. The plasmid pBS-PR1013EcoΔPstΔXho is digested with PstI. A PstI/BamHI adaptor having the sequence
5′-GGGATCCCTGCA-3′

is prepared as described in Example 4 and ligated to the PstI-digested pBS-PR1013EcoΔPstΔXho. The resulting material is first digested with BamHI and then with XhoI. The BamHI/XhoI fragment is isolated, mixed with and ligated to the digested pBI101.3, and transformed. A putative positive clone of pCIB270 is isolated and verified by restriction and DNA sequence analysis. The preparation of this plasmid is shown in Figure 7.

EXAMPLE 17 Preparation of M13mp18- or mp19-PR1013EcoΔPstΔXho

The plasmid pBS-PR1013EcoΔPstΔXho is digested with PstI and Asp718. The 1.1 kb Asp718/PstI fragment is isolated after electrophoresis on a 1% TAE low-gelling agarose gel. The replicative form (RF) of the coli phage M13mp18 or M13mp19 is prepared according to Messing, J., Meth. Enzymol. 101, 20 (1983). Alternatively, these vectors can be obtained from Bethesda Research Labs., Gaithersburg, Maryland. Either vector is digested first with Asp718 and then with PstI. After removal of the polylinker piece by electrophoresis on 0.7% TAE low-gelling agarose, the resulting vector RF is mixed and ligated in agarose with the 1.1 kb fragment prepared above. The DNA is transformed, putative positive plaques are isolated and their structure is verified by analysis of the RF DNA. The constructions of M13mp18- and mp19-PR1013EcoΔPstΔXho are shown in Figure 8.

EXAMPLE 18 Preparation of M13mp18- or mp19-PR1013EcoΔPstΔXho.Nco and pCIB 268

Single stranded M13mp18-PR1013EcoΔPstΔXho phage DNA is isolated according to Messing, supra. An 18 bp oligonucleotide primer of the sequence
5′-AATCCCATGGCTATAGGA-3′
is synthesized as described above. The primer is phosphorylated with T4 polynucleotide kinase (Maniatis, T. et al., supra at p. 125) using unlabeled ATP. After incubation at 37° C for 60 minutes the reaction is heated to 68° C for 15 minutes and then placed on ice. This primer and the M13 -40 forward sequencing primer (New England Biolabs #1212) are annealed to single stranded M13mp18-PR1013EcoΔPstΔXho DNA after mixing in a 1:1:1 molar ratio, by slow cooling after heating for 10 minutes at 68° C. The annealed mixture is placed on ice and 1/10 volume of 10 X elongation buffer (20 mM Tris buffer pH 7.5, 6 mM $MgCl_2$, 1 mM DTT, 1 mM dATP, 1 mM dCTP, 1 mM dGTP, and 1 mM dTTP) is added. 10 units of DNA Polymerase I large fragment (Klenow fragment, New England Biolabs #210) and 0.1 unit of T4 DNA Ligase (NEB # 202) are added and the reaction is allowed to proceed 14 hours at 15° C. At that time an additional aliquot of each enzyme is added and the reaction is carried out for an additional 2 hours at 25° C before the mixture is used to transform competent JM101 E. coli. A general flow diagram of this procedure is presented in Figure 9.

To identify the mutated phage, the plaques are lifted to Gene Screen plus (DuPont) and processed according to the manufacturer's recommendations. The filters are pre-hybridized for four hours, and hybridized overnight at 42° C, in the manufacturer's hybridization solution containing 0.9 M NaCl. The filters are then sequentially washed at a NaCl concentration of 0.9 M and monitored by autoradiography increasing the wash temperature by 5° C at each step. Phages identified by hybridization to have been mutated are sequenced to confirm the base change.

The replicative form of one such candidate (M13mp18-PR1013EcoΔPstΔXho.Nco) is isolated and digested with PstI and BstEII to produce a 394 bp fragment which is used to replace the corresponding non-mutated 394 bp fragment in pBS-PR1013EcoΔPstΔXho as illustrated in Figure 10. This results in the preparation of pCIB268 which has an NcoI site at position 930 followed by 217 bp of the coding sequence for the PR-1a gene. The structure of this plasmid is confirmed by sequence analysis.

EXAMPLE 19 Preparation of Chimeric Genes containing GUS and Variable Lengths of the PR Promoter Sequence

A. Construction of pCIB269

A fragment of 930 bp is isolated after electrophoresis of XhoI and NcoI digested pCIB268 on a 1.0 % low gelling temperature agarose TAE gel. This fragment is then ligated into pBS-GUS1.2 (see Example 19 B) which had been digested with XhoI and NcoI. Transformants are isolated and analyzed by restriction analysis and DNA sequencing. One positive plasmid is chosen and named pCIB269. The construction of this plasmid is shown in Figure 11.

B. Construction of pBS-GUS1.2

pBS-GUS1.2 is created by three part ligation of a 391 bp SalI/SnabI fragment from pRAJ265 (Jefferson, R.A. et al., EMBO J. 6, 3091-3907 (1987) and GUS User Manual, Clonetech Labs., Palo Alto, Ca.) with a 1707 bp SnabI/EcoRI fragment from pBI221 (Jefferson, R.A. et al., EMBO J. supra) and pBS digested with SalI and EcoRI, see Figure 12. Transformants are isolated and analyzed by restriction digestion and DNA sequencing. One verified clone is named pBS-GUS1.2.

C. Construction of pCIB200

TJS75Kan is first created by digestion of pTJS75 (Schmidhauser and Helinski, J. Bacteriol. 164, 446-455 (1985)) with NarI to excise the tetracycline gene, followed by insertion of an AccI fragment from pUC4K (Messing, J. and Vierra, J., Gene 19, 259-268 (1982)) carrying a NptI gene. pCIB 200 is then made by ligating XhoI linkers to the EcoRV fragment of pCIB7 (containing the left and right T-DNA borders, a plant selectable nos/nptII chimeric gene and the pUC polylinker, Rothstein, S.J. et al., Gene 53, 153-161 (1987)) and cloning XhoI digested fragment into SalI digested TJS75Kan.

D. Construction of pCIB271

The plasmid pCIB269 (Example 19 A) is digested with XhoI and EcoRI and the 3023 bp fragment carrying the PR-1a promoter linked to the GUS gene with a nos 3′ end is isolated following electrophoresis on a 1.0% low gelling agarose TAE gel. This fragment is then ligated into a broad host range vector pCIB200 which has been digested with SalI and EcoRI. Transformants are isolated and analyzed by restriction analysis. One verified clone is named pCIB271. The construction of this clone is shown in Figure 13.

E. Construction of pCIB272

pCIB268 (Example 18) is digested with AluI plus NcoI and an 833 bp fragment is isolated after electrophoresis on a 1 X TAE 0.7% agarose gel. This fragment is ligated to the $\beta$-glucuronidase gene with the nos 3′ end in pBS-GUS1.2 (Example 19 B). The promoter and GUS are then excised from this plasmid named pCIB282 as an Asp718I/BamHI fragment and ligated into pCIB200 which has been digested with Asp718I and BamHI before transformation into E. coli strain DH5$\alpha$. Transformants are isolated and analyzed by restriction analysis. One verified clone is named pCIB272.

F. Construction of pCIB273

pCIB268 is digested with HaeIII plus NcoI and a 603 bp fragment is isolated after electrophoresis on a 1 X TAE 0.7% agarose gel. This fragment is placed in front of the $\beta$-glucuronidase gene with the nos 3′ end in pBS-GUS1.2. The promoter and GUS are then excised from this plasmid named pCIB283 as an Asp718I/BamHI fragment and ligated into pCIB200 which has been digested with Asp718I and BamHI before transformation into E. coli strain DH5$\alpha$. Transformants are isolated and analyzed by restriction analysis. One verified clone is named pCIB273.

EXAMPLE 20 Preparation of a Chimeric Gene in a Hygromycin Vector

pCIB269 is digested with XhoI and EcoRI and the 3023 bp fragment carrying the PR-1a promoter linked to the GUS gene with a nos 3′ end is isolated following electrophoresis on a 1.0% low gelling agarose TAE gel. This fragment is then converted to a XhoI/SalI fragment by ligation with a EcoRI/SalI adapter having the sequence
5′-AATTCGTCGACG-3′.
The XhoI/SalI fragment is ligated into SalI digested pCIB712 (Rothstein, S.J. et al., supra) to create pCIB219, see Figure 14.

EXAMPLE 21 Preparation of pCIB200/PR1-BT

A. Ligation with pCIB200

The plasmid pCIB1004 (see below) is digested with SphI, phenol extracted, ethanol precipitated and resuspended. An oligonucleotide with the sequence
5′-CCGGTACCGGCATG-3′
is synthesized, purified, kinased, and ligated to the SphI digested plasmid pCIB1004. After ligation, the ligase is inactivated and the DNA digested with KpnI. This DNA is run on a 0.8% low gelling temperature agarose gel. The plasmid pCIB200 (Example 19 C) is digested with KpnI and treated with calf-intestinal alkaline phosphatase and run on a 0.8% low gelling temperature agarose gel. The band containing the pCIB200 vector and the band containing the PR-1 5′ flanking sequence/BT fusion are mixed and the DNA ligated to form the plasmid pCIB200/PR1-BT.

B. Preparation of pCIB1004

The plasmid pCIB10/35Bt(607) is digested with NcoI and BamHI and run on a 0.8% low gelling temperature agarose gel. The plasmid pCIB269 (Example 19 A) is digested with NcoI and XhoI and run on a 0.8% low gelling temperature agarose gel. The plasmid pCIB710 (Rothstein, S.J. et al., supra) is digested with SalI and BamHI and run on a 0.8% low gelling temperature agarose gel. The band with the SalI and BamHI digested vector containing a CaMV 35S promoter 3′ end cloned into pUC19, the band containing the BT gene and the band containing the PR-1 5′ flanking region are excised, mixed and the DNA ligated to form the plasmid pCIB1004.

C. Preparation of pCIB10/35Bt(607)

pCIB10/35Bt(607), a deleted protoxin gene containing a sequence coding for approximately 607 amino acids, is prepared from plasmid pCIB10/35Sbt, a plasmid containing the protoxin gene from Bacillus thuringiensis endotoxin. E. coli MC1061 containing pCIB10/35Sbt was deposited at the American Type Culture Collection, ATCC No. 67329, February 27, 1987. A deleted protoxin gene is made by introducing a BamHI cleavage site (GGATCC) following nucleotide 1976 in the BT gene sequence. This is done by cloning the BamHI fragment containing the protoxin sequence from pCIB10/35Sbt into mp18, and using standard oligonucleotide mutagenesis procedures. After mutagenesis, double-standard replicative form DNA is prepared from the M13 clone, which is then digested with BamHI. The approximately 1.9 kb fragment containing the deleted protoxin gene is inserted into BamHI-cleaved pCIB10/710. The resulting plasmid is selected for on kanamycin. A detailed description for the preparation is given in U.S. Patent Applications Serial No. 122,109, filed November 18, 1987, which is incorporated by reference.

EXAMPLE 22 Preparation of pCIB1233 (pCIB200/PR1-AHAS)

A. Ligation with pCIB200

The plasmid pCIB200 is digested with KpnI and XbaI and run on a 0.8% low gelling temperature agarose gel. The plasmid pCIB1216 is digested with KpnI and XbaI and run on a 0.8% low gelling temperature agarose gel. The ~4.3 kb band containing the PR-1 5′ flanking sequence fused to the AHAS coding sequence and the band containing the pCIB200 vector are excised, mixed and the DNA ligated to form pCIB1233.

B. Preparation of pCIB1216

The plasmid pCIB269 (Example 19 A) is digested with XbaI and NcoI and run on a 0.8% low gelling temperature agarose gel. The plasmid pCIB1207 (see below) is digested with XbaI and NcoI and run on a low gelling temperature agarose gel. The band containing the bluescript vector with the PR-1 5′ flanking sequence and the 3.3 kb band containing the AHAS coding sequence are excised, mixed and the DNA ligated to form the plasmid pCIB1216.

C. Preparation of plasmid pCIB1207 containing the Arabidopsis AHAS gene

Total plant DNA is isolated from 5 weeks old Arabidopsis thaliana ecotype Columbia. 250 µg of this DNA is digested with 4000 units of the restriction enzyme XbaI for 4 hours. The digest is fractionated by electrophoresis on a 0.8% agarose gel. The DNA fragments between 6.5 kb and 4.36 kb (based upon HindIII-digested lambda DNA size markers) are isolated from the gel using NA-45 DEAE membrane (Schleicher and Schuell, Keene, NH, USA) following the manufacturer's recommended procedures. XbaI digested and phosphatase-treated lambda ongC (longC) arm preparation is obtained from Stratagene Cloning Systems (La Jolla, CA, USA). 0.1 µg of the Arabidopsis XbaI insert DNA is ligated to 1.0 µg of the longC arms according to procedures recommended by Stratagene. 2.5 µl of the ligation reaction is packaged in lambda phage heads using the Gigapack Plus kit (Stratagene Cloning Systems) following procedures recommended by the manufacturer. The activity of the library is titered on E. coli VCS257 (Stratagene Cloning Systems).

50 µg of PE16/8-c4 plasmid DNA (J. Polaina, Carlsberg Res. Comm. 49, 577-584) are digested with EcoRI, and the digest products are resolved on a 0.8% agarose gel. The 2 kb EcoRI fragment containing the coding sequence for the yeast AHAS gene is isolated from the gel using NA45 DEAE membrane following procedures recommended by the manufacturer. Radiolabeled probes to the 2 kb fragment are synthesized on the day of the hybridization reaction with the random priming reaction using Prime Time kit (International Biotechnologies Inc., New Haven, CT, USA), following procedures recommended by the manufacturer.

250,000 recombinant phages are plated on VCS257. Duplicate membrane lifts of the phage plaques are made using Colony/Plaque Screen membranes (NEN Research products, Du Pont, Boston, MA, USA) following procedures recommended by the manufacturer. The membranes are prehybridzed in LS hybridization buffer, then transferred to 150 ml of fresh LS hybridization buffer containing 0.25 µg of $^{32}$P-labelled yeast AHAS gene probe prepared as described above. The membranes are incubated for 16 hours at 42°C with gentle shaking, washed twice at room temperature with 2XSSC for 15 minutes per wash, washed three times at 42°C in LS wash buffer (25% formamide, 5XSSC, 1% SDS) for 2 hours per wash, and washed twice at in rinse buffer (0.1XSSC, 0.1% SDS) for 30 minutes per wash. The membranes are mounted and fluorographed with Cronex Lightening Plus Sceens (Du Pont, Wilmington, DE, USA) and XAR-5 film (Kodak). The plaques from regions of the plates that gave positive film signals on both membrane lifts are picked and replated at a low density of 300 plaques per 150 mm plate, and the screening process is repeated until single hybridizing plaques are isolated.

Miniprep of phage DNA from the plaque-purified phage is carried out following procedures accompanying the LambdaSorb Phage Adsorbent kit (Promega, Madison, WI, USA). The phage DNA is cut with restriction enzyme XbaI and the 5.8 kb insert fragment is cloned into the XbaI site of pBS(-) plasmid (Stratagene Cloning Systems). The identity of the cloned fragment with the XbaI fragment containing the Arabidopsis AHAS gene is verified by comparing its restriction map and DNA sequence with those published for the gene by Haughn et al., Mol. Gen. Genet. 211, 266 (1988), and Mazur et al., Plant Physiol. 85, 1110 (1987). This plasmid is designated pCIB1207.

EXAMPLE 23 Preparation of pCIB1232 (pCIB200/PR1-AHAS-SuR)

A. Ligation with pCIB200

The plasmid pCIB200 (Example 19 C) is digested with KpnI and XbaI and run on a 0.8% low gelling temperature agarose gel. The plasmid pCIB1230 is digested with KpnI and XbaI and run on a 0.8% low gelling temperature agarose gel. The ~4.3 kb band containing the PR-1 5′ flanking seqeunce fused to the AHAS-SuR coding sequence and the band containing the pCIB200 vector are excised, mixed and the DNA ligated to form pCIB1232.

B. Preparation of pCIB1230

Plasmid pCIB1208 (see below) is digested with XbaI and NcoI and the fragments are separated by electrophoresis on a 1 X TAE 0.8% low gelling temperature agarose gel. The plasmid pCIB269 (Example 19 A) is digested with NcoI and XbaI and run on a 1 X TAE 0.8% low gelling temperature agarose gel. The pCIB269 fragment containing the bluescript vector and the PR-1 promoter and the 3.3 kb fragment containing the mutated AHAS gene (identified by cross homology and by restriction fragment analysis with the gene decribed Mazur et al., Plant Physiol. 85, 1110-1117 (1987)) are excised, melted and ligated together to create a clone designated as pCIB1230.

C. Preparation of pCIB1208

A genomic DNA library is constructed as described in Example 22 C using DNA isolated from Arabidopsis plants selected for resistance to sulfonylurea herbicide as described by Haughn and Somerville, Mol. Gen. Genet. 204, 430-434 (1986). Plaques containing genes encoding acetohydroxy acid synthase (AHAS) are identified by cross hybridization with a yeast acetohydroxy acid synthase gene probe (see Example 22 C). The recombinant DNA is subcloned into Bluescript (Stratagene) creating a clone designated as pCIB1208. This plasmid contains a gene encoding a mutated acetohydroxyacid synthase that is resistant to inhibition by sulfonylurea herbicides.

EXAMPLE 24 Isolation of a Genomic Clone Encoding the Basic Form of β-1,3-Glucanase (Glucan Endo-1,3-β-glucosidase) from Nicotiana Tabacum

High molecular weight DNA is prepared from leaves of Nicotiana tabacum cv. Havana 425 by the CETAB procedure (Murray and Thompson, Nucleic Acid Res. 8, 4321 (1980)). 100 μg of DNA is digested to completion with SacI. This DNA is separated by electrophoresis on a 0.8% agarose gel. The region of the gel corresponding to a molecular weight range of 3 to 7 kb is cut into 6 equally sized strips and the DNA is electroeluted from these strips and precipitated as separate fractions. The DNA is resuspended and an aliquot from each fraction is run on an agarose gel and analyzed by Southern blotting. The fraction that contains DNA which hybridizes to a β-1,3-glucanase cDNA probe (Shinshi, H. et al., Proc. Natl. Acad. Sci. USA 85, 5541-5545 (1988)) is pooled and used in constructing libraries.

The cloning vector lambdaOngC purchased from Stratagene Corp. is digested with SacI. 1 μg of this DNA is mixed with 0.1 μg of the SacI digested tobacco DNA, and the DNA is ligated with T4 DNA ligase according to the manufacturer's suggestions. The ligated DNA is then packaged into phage particles using an in vitro packaging procedure according to Stratagene. The phages are plated with bacteria as suggested in the lambda manual. About 75,000 plaques are screened using a 32-P labeled β-1,3-glucanase cDNA probe. 11 positive plaques are identified. These plaques are purified by successive rounds of plating and screening.

DNA is isolated from the purified clones and the clones are analyzed by restriction digestion using HindIII, EcoRI, and SacI. The clones are of two types, one represented by the clone 39.1 and the other represented by the clone 39.3. The 4.5 and 4.7 kb inserts in clone 39.1 and 39.3, respectively, are subcloned into the bluescript plasmid digested with SacI, and the subclones pBSGluc39.1 (SacI fragment derived from the lambda clone 39.1) and pBSGluc39.3 (SacI fragment derived from the lambda clone 39.3) are isolated. The sequence of the DNA in the SacI fragments contained in the subclones pBSGluc39.1 and pBSGluc39.3 is determined by DNA sequencing and shown in Sequence 5 and Sequence 6, respectively. The coding sequence is found and a large intervening sequence is located near the 5′ end of the coding sequence.

EXAMPLE 25 Identification of the Transcriptional Start Site for the β-1,3-Glucanase Gene

A. Primer Extension Mapping

A synthetic DNA primer is synthesized which is complementary to bases 2399 to 2416 and used in primer extension experiments as described in Example 5. The RNA for these experiments is isolated from Phytophthora parasitica var. nicotiana infected tobacco. The primer extension products are run against a molecular weight standard in which the labeled primer is used in dideoxy DNA sequencing reactions with the subclone pBSGluc39.1 used as a template. Extension products are identified, after gel electrophoresis and autoradiography as described in Example 5, that correspond to positions 1432, 1446 and 1447 of the β-1,3-glucanase 39.1 sequence, Sequence 5. Since a large intron exists between positions 1554 and 2345 of the pBSGluc39.1 sequence, the molecular weight ladder might not reflect the correct molecular weight of the extension products. Therefore, a second primer extension mapping experiment is conducted using a primer that is complementrary to positions 1530 to 1547. Using this primer, three 5′ ends of the glucanase mRNA are mapped to A residues at positions 1434, 1448 and 1449.

B. S1 nuclease mapping

A synthetic oligonucleotide complementary to positions 1415 to 1504 is synthesized for use as a probe in S1 nuclease mapping. The oligonucleotide is kinased at the 5′ end using 32P-ATP and T4 polynucleotide kinase according to the supplier's recommendation. Following phenol extraction and ethanol precipitation the labeled oligonucleotide is resuspended in formamide hybridization buffer (see Example 6) at a concentration of about 2 nM. The RNA used in these experiments is isolated from Phytophthora parasitica infected tobacco as in the previous example. S1 mapping is conducted on this RNA using the labeled oligonucleotide as described in Example 6. After gel electrophoresis three bands are detected that correspond to positions 1434, 1448 and 1449 on the pBSGluc39.1 DNA sequence.

C. Determination of the transcriptional start site

Primer extension and S1 nuclease mapping procedures both place the 5′ ends of the mRNA at positions 1434, 1448 and 1449. Therefore these sites, which are all adenine residues, correspond to the transcriptional start site of the β-1,3-glucanase gene.

EXAMPLE 26 Preparation of pBSGluc39.1/GUS

Oligonucleotides with the sequences

A) 5′-GTTTATGTGAGGTAGCCATGGTGGGAAGACTTGTTGGG-3′

B) 5′-GATCGCGGTACCGAGCTCCTCTAGGGGGCCAAGG-3′

are synthesized, purified and used in a polymerase catalyzed reaction (PCR) according to the supplier's directions (Perkin Elmer Cetus, DNA thermal cycler; and Perkin Elmer Cetus, GeneAmp Reagent Kit) to amplify a 1494 bp fragment of DNA from pBSGluc39.1. The oligos are designed to add a KpnI site immediately 5′ to the SacI site at base-pair 1 of the 39.1 glucanase sequence and to generate a new NcoI site at base-pair 1462. The DNA derived from the PCR amplification procedure is phenol/chloroform extracted and ethanol precipitated. The DNA is resuspended and digested with both NcoI and KpnI and run on a 0.8% low-gelling temperature agarose gel. The 1462 bp band is excised and used in the following ligation. The plasmid pBS-GUS1.2 (Example 19 B) is digested with NcoI and KpnI and run on a 0.8% agarose gel and the band containing the vector is excised. The band containing the pBS-GUS1.2 vector and the 1462 bp band from the PCR reaction are ligated and used to transform E. coli. Positive colonies are picked and screened for those that contain the 1462 bp insert. The plasmids containing inserts are putative clones of pBSGluc39.1/GUS, however, since the mutation rate in this procedure is relatively high (~1/1000 bases), several clones have to be sequenced throughout the 1462 bp fragment. One clone which has the expected sequence is chosen as the clone pBSGluc39.1/GUS.

EXAMPLE 27 Preparation of pBSGluc39.3/GUS

Oligonucleotides with the sequences

A) 5′-GTTTATCTGAGGTAGCCATGGTGAGAAGACTTGTTGGA-3′

B) 5′-GATCGCGGTACCGAGCTCCCTTGGGGGGCAAG-3′

are synthesized, purified and used in a PCR reaction to amplify a 1677 bp fragment from pBSGluc39.3. The oligonucleotides are designed to introduce a new KpnI site immediately adjacent to the SacI site at position 1 of the glucanase 39.3 sequence and a new NcoI site at position 1646. The DNA from the PCR amplification is phenol/chloroform extracted, ethanol precipitated, resuspended, digested with both NcoI and KpnI and run on a 0.8% low-gelling temperature agarose gel. The band at 1646 bp is excised and used in the following ligation. pBS-GUS1.2 is digested with NcoI and KpnI and run on a 0.8% low gelling temperature agarose gel. The band containing the vector is excised, mixed with the l646 bp band from the PCR reaction and ligated to form the plasmid pBSGluc39.3/GUS. As before, several putative plasmids are isolated and seqeunced and one with the expected sequence is picked as the plasmid pBSGluc39.3/GUS.

EXAMPLE 28 Preparation pCIB200/Gluc39.1-GUS and pCIB200/Gluc39.3-GUS

A. pCIB200 (Example 19 C) is digested with KpnI and XbaI and run on a 0.8% low gelling temperature agarose gel. The plasmid pBSGluc39.1/GUS (Example 26) is digested with KpnI and XbaI and the fragments are separated on a 0.8% low gelling temperature gel. The bands containing the pCIB200 vector and the KpnI-XbaI band containing the 5′ flanking sequence of the glucanase 39.1 gene fused to the GUS gene are excised and the DNA ligated to form the plasmid pCIB200/Gluc39.1-GUS.

B. Likewise, plasmid pBSGluc39.3/GUS (Example 27) is digested and ligated with digested pCIB200 to form the plasmid pCIB200/Gluc39.3-GUS.

EXAMPLE 29 Preparation of pCIB200/Gluc39.1-BT and pCIB200/Gluc39.3-BT

A. pCIB200/Gluc39.1-BT

The plasmid pBSGluc39.1/GUS is digested with KpnI and NcoI and run on a 0.8% low gelling temperature agarose gel. The plasmid pCIB1004 (Example 21 B) is digested with KpnI and NcoI and run on a 0.8% low gelling temperature agarose gel. The plasmid pCIB200 (Example 19 C) is digested with KpnI, dephosphorylated using calf-intestine alkaline phosphatase and run on a 0.8% low gelling temperature agarose gel. The band containing the pCIB200 vector, the band containing the glucanase 5′ flanking region, and the band containing the BT gene are excised, mixed together and ligated to form the plasmid pCIB200/Gluc39.1-BT.

B. pCIB200/Gluc39.3-BT

Likewise, plasmid pBSGluc39.3/GUS is digested and ligated with digested pCIB1004 and digested pCIB200 to form the plasmid pCIB200/Glu39.3-BT.

EXAMPLE 30 Preparation of pCIB200/Gluc39.1-AHAS and pCIB200/Gluc39.3-AHAS

A. Ligation with pCIB200

The plasmid pBSGluc39.1/AHAS is digested with KpnI and XbaI and run on a 0.8% low gelling temperature agarose gel. The plasmid pCIB200 (Example 19 C) is digested with KpnI and XbaI and run on a 0.8% low gelling temperature agarose gel. The band containing the glucanase/AHAS fusion and the band containing the pCIB200 expression vector are excised, mixed and the DNA ligated to form the plasmid pCIB200/Gluc39.1-AHAS.

Likewise, plasmid pBSGlu39.3/AHAS is digested and ligated with digested pCIB200 to form the plasmid pCIB200/Glu39.3-AHAS.

B. Preparation of plasmids pBSGlu39.1/AHAS and pBSGlu39.3/AHAS

The plasmid pBSGluc39.1/GUS (Example 26) is digested with NcoI and XbaI and run on a 0.8% low gelling temperature agarose gel. The plasmid pCIB1207 (Example 22 C) is digested with NcoI and XbaI and run on a 0.8% low gelling temperature agarose gel. The band containing the glucanase 5′ flanking sequence and vector and the band containing the AHAS coding sequence are excised, mixed and the DNA ligated to produce the plasmid pBSGluc39.1/AHAS.

Likewise, plasmid pBSGlu39.3/GUS (Example 27) is digested and ligated with digested pCIB1207 to form the plasmid pBSGlu39.3/AHAS.

EXAMPLE 31 Preparation of pCIB200/Gluc39.1-AHAS-SuR and pCIB200/Gluc39.3-AHAS-SuR

A. Ligation with pCIB200

The plasmid pBSGluc39.1/AHAS-SuR is digested with KpnI and XbaI and run on a 0.8% low gelling temperature agarose gel. The plasmid pCIB200 (Example 19 C) is digested with KpnI and XbaI and run on a 0.8% low gelling temperature agarose gel. The band containing the glucanase/AHAS-SuR fusion and the band containing the pCIB200 expression vector are excised, mixed and the DNA ligated to form the plasmid pCIB200/Gluc39.1-AHAS-SuR.

Likewise, plasmid pBSGluc39.3/AHAS-SuR is digested and ligated with digested pCIB200 to form the plasmid pCIB200/Gluc39.1-AHAS-SuR.

B. Preparation of plasmids pBSGluc39.1/AHAS-SuR and pBSGluc39.3/AHAS-SuR

The plasmids pBSGluc39.1/GUS (Example 26) and pBSGluc39.3/GUS (Example 27) are digested with NcoI and XbaI and the restriction fragments are separated on a 1 X TAE 0.8% low gelling temperature agarose gel. pCIB1208 (Example 23 C) is digested with XbaI and NcoI and the 3.3 kb fragment containing the mutated AHAS gene (sulfonylurea herbicide resistant, SuR) is isolated by agarose gel electrophoresis. The fragments containing the glucuronidase promoter and pBS vector are excised, melted and ligated with the AHAS fragment to create clones designated pBSGluc39.1/AHAS-SuR and pBSGluc39.3/AHAS-SuR, respectively.

4. IDENTIFICATION OF NOVEL GENES

The following example discloses the isolation and characterization of the remaining novel PR protein genes of this invention.

EXAMPLE 32 Isolation of Other Genomic and cDNA Clones

A. Isolation of a genomic clone encoding PR-1′

A genomic library is constructed and screened with a PR-1 cDNA probe as described in Example 11. A clone is isolated and designated lambdatobchrPR1019. A preliminary restriction map is established. A ClaI fragment is subcloned into bluescript and then a restriction map of plasmid, pBS-PR1019Cla, is established. A large XhoI fragment is deleted from pBS-PR1019Cla resulting in the plasmid pBS-PR1019Cla Xho. This plasmid contains about 2.7 kb of tobacco DNA containing the PR1019 gene. Deletions are made in this plasmid and the set of deletions are used for DNA sequencing.

The DNA sequence for about 2100 bp of this gene is shown in Sequence 2. The protein encoded in the PR1019 is found to be about 65% homologous to PR-1a, PR-1b, or PR-1c; therefore, this new gene is named PR-1′.

B. Isolation of a cDNA clone encoding cucumber chitinase

A pathogen inducible chitinase protein is isolated from infected cucumber leaves as described (Metraux, J.P. et al., Physiological and Molecular Plant Pathology, 33, 1-9 (1988)). Peptides are generated from this homogeneous protein preparation essentially as described in the art and as described in Examples 9 and 10. Two regions of the protein are selected and oligonucleotide probes are synthesized that are complimentary to all possible combinations of messenger RNA capable of encoding the peptides. The sequence of the probes are:

```
                 G  T      A
Probe 1:    5'-CCATTCTGNCCCCAGTA-3'

                 G  G  G  G  C
Probe 2:    5'-GGATTATTATAAAATTGNACCCA-3'.
```

Cucumber leaves are infected with Tobacco Necrosis Virus (TNV) and RNA is isolated 5 days after infection as described in Example 5. Poly A+ RNA is isolated by standard techniques and a cDNA library is constructed in the lambda Zap cloning vector (Stratagene), essentially as described (Gubler, U. and Hoffman, B.J., Gene 25, 263 (1983)). 300,000 plaques are plated and duplicate plaque lifts are probed either with $^{32}P$ labeled oligonucleotide mixture 1 (probe 1) or mixture 2 (probe 2). Plaques are isolated that show positive results when screened with either probe. Isolation of phage and automatic excision are carried out as described in the Stratagene Lambda Zap laboratory manual.

Once the chitinase cDNA clones are isolated in the bluescript plasmid they are sequenced by dideoxy sequencing. The sequence of the chitinase gene is presented in Sequence 3.

C. Isolation of the cDNA clone encoding the major form of PR-R.

Nicotiana tabaccum cv. Xanthi-nc leaves are infected with tobacco mosaic virus and harvested five days post infection. Total RNA is prepared as described in Example 5 and poly A+ RNA is isolated using standard techniques. A cDNA library is constructed using this poly A+ RNA in the Lambda ongC cloning vector (Stratagene). About 300,000 plaques are screened with an oligonucleotide probe of the sequence
5′-GAACTTCCTAAAAGCTTCCCCTTTTATGCC-3′.

Positive plaques are purified by standard techniques and DNA is prepared from the phage. A fragment of the recombinant phage which contains the cDNA is subcloned into the bluescript plasmid. The DNA sequence of the cDNA is determined by DNA sequencing. The sequence of one clone which encodes the major form of PR-R (Pierpoint, W.S. et al., Physiol. Plant Pathol. 31, 291 (1987)) is presented in Sequence 4. This encoded protein has a very strong homology to a known trypsin/alpha-amylase inhibitor isolated from maize (Richardson, M. et al., Nature 327, 432 (1987)). The cloned PR-R may be an inhibitor of either proteases or alpha-amylases and as such will confer insecticidal, viricidal or fungicidal activity to plants when expressed transgenically.

D. Isolation of the cDNA encoding PR-Q

Nicotiana tabacum cv. Xanthi-nc leaves are infected with tobacco mosaic virus and harvested five days post infection. Total RNA is prepared as described in Example 5 and polyA+ RNA is isolated using standard techniques. A cDNA library is constructed using this polyA+ RNA in the LambdaOngC cloning vector (Stratagene). About 300,000 plaques are screened using a labeled cDNA probe encoding the tobacco basic chitinase gene (Shinshi et al., Proc. Natl. Acad. Sci. USA 84, 89-93 (1987)) and washing filters at 50° C in 0.125 mM NaCl, 1% SDS, 40 mM sodium phosphate (pH 7.2), 1 mM EDTA. 24 positive plaques are purified and the DNA sequence of one clone named pBScht15 is determined. This sequence is presented in Sequence 7.

The PR-Q protein is partially purified from the intracellular fluid of TMV infected tobacco leaves as described in Example 8. Fractions from the Ultragel ACA54 chromatography containing PR-O, PR-P, PR-Q and PR-R are pooled and concentrated by lyophilization. The proteins are further purified by polyacrylamide gel electrophoresis. The protein band containing PR-Q is excised and protein eluted according to the Applied Biosystems User Bulletin No. 36, March 21, 1988. The protein is treated with pyroglutamate aminopeptidase and sequenced by automated Edman degradation as decribed in Example 9. The sequence of the amino terminus of the PR-Q protein is determined to be (Xxx = unclear):
GlnGlyIleGlySerIleValThrXxxAspLeuPheAsnGluMetLeu
The protein encoded in the clone in Sequence 7 is determined to be the pathogenesis-related protein Q based on: 1) limited structural homology to the the basic tobacco chitinase and 2) identity to the amino-terminal protein sequence of PR-Q determined by protein sequencing.

E. Isolation of the cDNA encoding the acidic form of β-1,3-glucanase

About 300,000 plaques of the cDNA library described in Example 32 D (above) are screened using a labeled cDNA probe encoding the basic form of β-1,3-glucanase (Shinshi, H. et al., supra) and washing filters at 50° C in 0.125 M NaCl, 1 % SDS, 40 mM sodium phosphate (pH 7.2), 1 mM EDTA. 15 positive plaques are isolated and the insert is subcloned into the bluescript plasmid. Partial DNA sequencing reveals that clone 5 and 6 encode identical cDNA's which have about 55% homology to the known DNA sequence of the basic form of β-1,3 glucanase. The sequence of the cDNA in clone 5 and 6 is determined and shown in Sequence 8. It is concluded that this cDNA encodes the acidic form of β-1,3 glucanase based on the limited amino acid sequence homology and the more acidic isoelectric point of the encoded protein.

F. Isolation of a genomic clone encoding the cucumber chitinase gene

Nuclei are isolated from Cucumis sativus cv. Long Marketer and DNA is isolated as described in Example 11. 3 µg of this DNA is digested with either EcoRV, EcoRI, BamHI or HinDIII and the fragments are separated on a 0.5% agarose gel. A Southern blot analysis, using a labeled probe of the cucumber chitinase cDNA (Sequence 3), reveals a single band in the EcoRI digest at about 12 kb. Therefore, it is decided to isolate the cucumber chitinase gene using a total EcoRI digest and ligating into a lambda replacement-type cloning vector.

10 µg of DNA isolated above is digested to completion using EcoRI. This DNA is then extracted with phenol and precipitated with ethanol and ligated into the EcoRI site of EMBL 4. About 100,000 plaques are screened with the labeled cDNA probe of cucumber chitinase, Sequence 3. 10 positive plaques are picked and purified and the DNA insert is analyzed by preliminary restriction digest mapping. All the clones give the same result and one is therefore picked for further analysis. The 12 kb insert in this clone is then subcloned into the bluescript plasmid to give plasmid pBScucchi/chitinase. A restriction map is established for this cloned DNA and fragments are subcloned and the DNA sequence determined.

G. Isolation of a genomic clone encoding acidic β-1,3-glucanase

A genomic library is constructed as described in Example 11 and screened with a cDNA clone for the acidic β-1,3-glucanase, Example 32 E. A lambda clone is isolated, and a 1 kb EcoRI fragment of this lambda clone subcloned into Bluescript as described above in Example 32 A. The bluescript clone is named pBSGL6e.

5. STABLE TRANSFORMATION AND REGENERATION OF PLANTS

Plant tissue is transformed with the vectors described above by any technique known in the art. Such methods used for transfer of DNA into plant cells include, for example, the direct infection of or co-cultivation of plants, plant tissue or cells with A. tumefaciens (Horsch, R.B. et al., Science 225, 1229 (1985); Marton, L., Cell Culture and Somatic Cell Genetics of Plants, Vol. 1, 514-521, 1984), treatment of protoplasts with exogenous DNA by methods such as those described in the following publications: Paszkowski, J. et al., EMBO J. 3, 2717 (1984); EP-A 0 164 575; Shillito, R.D. et al., Bio/Technology 3, 1099 (1985); Potrykus, I. et al., supra; Loerz, H. et al., Mol. Gen. Genet. 199, 178 (1985); Fromm, M. et al., supra; GB 2,140,822; and Negrutiu, I. et al., Plant Mol. Biol. 8, 363 (1987); incubation with polyethylene glycol (PEG) (Negrutiu, I. et al., supra); micro-injection (Reich, T.J. et al., Bio/Technology 4, 1001-1004 (1986); Reich, T.J. et al., Can. J. Bot. 64, 1259-1267 (1986)), microprojectile bombardment (Klein, T.M. et al., Nature 327, 70, (1987)).

EXAMPLE 33 Transformation of Agrobacterium

pCIB270 (Example 16), pCIB271, pCIB272 and pCIB273 (Examples 19 D, E and F) plasmid DNA is purified on cesium chloride ethidium bromide gradients and is used to transform A. tumefaciens strain A136 containing the helper plasmid pCIB542 by the procedure of Holsters, M. et al., Mol. Gen. Genet. 163, 181-187 (1978) resulting in the strains CIB270, CIB271, CIB272 and CIB273.

Using the same procedure, plasmids pCIB271, pCIB272, and pCIB273 are transformed into the virulent A. tumefaciens strains 15955, A208, A281, and the A. rhizogenes strain A4, creating strains designated as pCIB271 X 15955, pCIB271 X A208, pCIB271 X A281, pCIB271 X A4, pCIB272 X 15955, pCIB272 X A208, pCIB272 X A281, pCIB272 X A4, pCIB273 X 15955, pCIB273 X A208, pCIB273 X A281, and pCIB273 X A4.

Agrobacterium strain CIB542 is strain EHA101 (Hood et al., J. Bacteriol. 168, 1291-1301 (1986)) in which the kanamycin marker of the plasmid has been replaced by the spectinomycin/streptomycin portion of Tn7.

EXAMPLE 34 Leaf Disk Transformation of Tobacco

Agrobacterium strains CIB270, CIB271, CIB272 and CIB273 are grown 18-24 hours in glutamate salts media adjusted to pH 5.6 and supplemented with 0.15% mannitol, 50 µg/ml kanamycin, 50 µg/ml spectinomycin and 1 mg/ml streptomycin before they are diluted to an $OD_{600}$ of 0.2 in the same media without the antibiotics. The bacteria are then grown for three to five hours before dilution to an $OD_{600}$ of 0.2-0.4 for inoculation of discs of 5-7 mm punched from leaves of Nicotiana tabacum cv. xanthi that have been grown aseptically in GA7 containers, following a modification of the method of Horsch, R. et al., Science 227, 1229-1232 (1985).

The leaf disks are maintained on 0.7% agar containing Murashige and Skoogs major and minor salts (MS), 1 mg/l benzyladenine and 1 mg/ml α-naphthaleneacetic acid for two days before transfer to the same media containing 50 µg/ml kanamycin, 100 µg/ml carbenicillin and 100 µg/ml mefoxin. Shoots which form on the discs are excised and propagated until six plantlets are obtained by subculturing the shoot tips on MS media containing 50 µg/ml kanamycin in GA7 containers.

The plantlets are rooted on medium containing no hormones and 50 µg/ml kanamycin, transferred to soil and hardened in a phytotron before transfer to the greenhouse for induction treatment with chemical regulators. At flowering time flowers are induced to selfpollinate. Seeds are harvested following maturation.

Induction experiments and results are described in Example 63, 66 and Table II.

EXAMPLE 35 Production of Transgenic Tobacco Callus and Plants

Agrobacterium strain pCIB270 or pCIB271 is used to transform callus forming from the leaf disks (Example 34). Callus forming on kanamycin-containing MSBN selection medium is maintained on a callus growth medium comprised of MS major, minor salts and Fe-EDTA (Gibco # 500-1117; 4.3 g/l), MS vitamins, 100 mg/l myo-inositol, 20 g/l sucrose, 2 mg/l naphthaleneacetic acid and 0.3 mg/l kinetin.

The callus can be used to regenerate transgenic plants by transferring callus pieces to MSBN medium and following methods described in Example 34. The callus is also used to measure gene induction following application of inducing chemicals and to screen for gene inducing chemicals.

EXAMPLE 36 Transformation of Carrot

Agrobacterium strains CIB270, CIB271, CIB272, CIB273, pCIB271 X 15955, pCIB271 X A208, pCIB271 X A281, pCIB271 X A4, pCIB272 X 15955, pCIB272 X A208, pCIB272 X A281, pCIB272 X A4, pCIB273 X 15955, pCIB273 X A208, pCIB273 X A281, and pCIB273 X A4 are grown as described in Example 34. The bacteria, diluted to an $OD_{600}$ of 0.2-0.4, are then used for inoculation of discs cut from surface sterilized carrots.

To surface sterilize the carrots they are peeled and then soaked 20 minutes in a 10% solution of chlorox. The carrots are rinsed with sterile water, sliced into 5 mm pieces and placed basal side up onto water agar. 20-50 µl of bacteria are then applied to the upper surface of the discs. After 7 days the discs are transferred to 0.7% agar containing MS salts, 3% sucrose, 0.1 mg/l 2,4-D, 50 µg/ml kanamycin, 100 µg/ml carbenicillin, and 100 µg/ml mefoxin. Callus forming around the cambial ring is excised and placed on 0.7% MS agar supplemented with 3% sucrose, 0.1 mg/l 2,4-D, 50 µg/ml kanamycin, 100 µg/ml carbenicillin, and 100 µg/ml mefoxin. After the callus has been grown it is cut into small pieces and randomized onto four plates of the same media. When this callus has filled the plate three of the plates are sprayed with water, 50 mM sodium salycilate pH 5.6, or 250 µg/l methyl benzo-1,2,3-thiadiazole-7-carboxylate to induce the expression of the chimeric PR-1a/GUS gene.

Induction experiments and results are described in Example 63 and 66.

EXAMPLE 37 Transformation of Sunflower

Agrobacterium strains CIB270, CIB271, CIB272, CIB273, pCIB271 X 15955, pCIB271 X A208, pCIB271 X A281, pCIB271 X A4, pCIB272 X 15955, pCIB272 X A208, pCIB272 X A281, pCIB272 X A4, pCIB273 X 15955, pCIB273 X A208, pCIB273 X A281, and pCIB273 X A4 are grown as described in Example 34. The bacteria, diluted to an $OD_{600}$ of 0.2-0.4, are then used for inoculation of stems of sunflower plants prepared as follows:

Sunflower seeds are soaked 10 min in 10% captan followed by 10 min in 10% chlorox and rinsing with sterile water. The seed coats are removed and the seeds are germinated on 0.7% water agar in the dark for three days, after which they are placed into a labline incubator set at 23° C with a 12 hour day and night. The seedlings are grown for one week before decapitation and inoculation of the bacteria onto the cut stem surface.

After one week the stems inoculated with CIB270, CIB271, CIB272 or CIB273 are cut and placed on 0.7% agar containing MS salts, 3% sucrose 2 mg/ml α-napthaleneacetic acid, 1 mg/ml 6-benzylaminopurine, 100 µg/ml carbenicillin, 100 µg/ml mefoxim and 50 µg/ml kanamycin. Stems inoculated with pCIB271 X 15955, pCIB271 X A208, pCIB271 X A281, pCIB271 X A4, pCIB272 X 15955, pCIB272 X A208, pCIB272 X A281, pCIB272 X A4, pCIB273 X 15955, pCIB273 X A208, pCIB273 X A281, and pCIB273 X A4 are cut and placed on 0.7% agar containing MS salts, 3% sucrose, 100 µg/ml carbenicillin, and 100 µg/ml mefoxim. The callus is transferred to fresh media every two weeks until sufficient quantity is obtained for 4 plates. Half of the callus growing from the virulent Agrobacterium strains is transferred to media without hormones containing 50 µg/ml kanamycin. After sufficient callus grown in the presence or absence of kanamycin is obtained, it is treated for induction as described above for transformed carrot callus.

EXAMPLE 38 Transformation of Tomato

Agrobacterium strains CIB270, CIB271, CIB272, CIB273, pCIB271 X 15955, pCIB271 X A208, pCIB271 X A281, pCIB271 X A4, pCIB272 X 15955, pCIB272 X A208, pCIB272 X A281, pCIB272 X A4, pCIB273 X 15955, pCIB273 X A208, pCIB273 X A281, and pCIB273 X A4 are grown as described in Example 34. The bacteria, diluted to an $OD_{600}$ of 0.2-0.4, are then used for inoculation of stems of tomato seedlings prepared as follows:

Tomato seeds are soaked 20 min in 10% chlorox and rinsed with sterile water. The seeds are germinated on 0.7% water agar in the dark for three days, after which they are placed into a labline incubator set at 23° C with a 12 hour day and night. The seedlings are grown for one week before decapitation and inoculation of the bacteria onto the cut stem surface.

After one week the stems inoculated with CIB270, CIB271, CIB272 or CIB273 are cut and placed on 0.7% agar containing MS salts, 3% sucrose, 2 mg/ml α-napthaleneacetic acid, 1 mg/ml 6-benzylaminopurine, 100 µg/ml carbenicillin, 100 µg/ml mefoxim, and 50 µg/ml kanamycin. Stems inoculated with pCIB271 X 15955, pCIB271 X A208, pCIB271 X A281, pCIB271 X A4, pCIB272 X 15955, pCIB272 X A208, pCIB272 X A281, pCIB272 X A4, pCIB273 X 15955, pCIB273 X A208, pCIB273 X A281, and pCIB273 X A4 are cut and placed on 0.7% agar containing MS salts, 3% sucrose, 100 µg/ml carbenicillin, and 100 µg/ml mefoxim. The callus is transferred to fresh media every two weeks until sufficient quantity is obtained for 4 plates. Half of the callus growing from the virulent Agrobacterium strains is transferred to media without hormones containing 50 µg/ml kanamycin. After sufficient callus grown in the presence or absence of kanamycin is obtained, it is treated for induction as described above for transformed carrot callus.

EXAMPLE 39 Transformation of Cotton

Agrobacterium strains pCIB271 X 15955, pCIB271 X A208, pCIB271 X A281, pCIB271 X A4, pCIB272 X 15955, pCIB272 X A208, pCIB272 X A281, pCIB272 X A4, pCIB273 X 15955, pCIB273 X A208, pCIB273 X A281, and pCIB273 X A4 are grown as described in Example 34. The bacteria, diluted to an $OD_{600}$ of 0.2-0.4, are then used for inoculation of cotton cotyledons prepared as follows:

The cotton seeds are soaked 20 min in 10% chlorox and rinsed with sterile water. The seeds are germinated on 0.7% water agar in the dark. The seedlings are grown for one week before inoculation of the bacteria onto the cotyledon surface.

The inoculated cotyledons are allowed to form callus before they are cut and placed on 0.7% agar containing MS salts, 3% sucrose, 100 µg/ml carbenicillin, and 100 µg/ml mefoxim. The callus is transferred to fresh media every three weeks until sufficient quantity is obtained for 4 plates. Half of the callus growing from the virulent Agrobacterium strains is transferred to media without hormones containing 50 µg/ml kanamycin. After sufficient callus grown in the presence or absence of kanamycin is obtained, it is treated for induction as described above for transformed carrot callus.

EXAMPLE 40 Preparation of a Special Type of Callus of Zea Mays, Elite Inbred line Funk 2717

Zea mays plants of the inbred line Funk 2717 are grown to flowering in the greenhouse, and self pollinated. Immature ears containing embryos approx. 2-2.5 mm in length are removed from the plants and sterilized in 10% Clorox solution for 20 minutes. Embryos are aseptically removed from the kernels and plated with the embryo-axis downwards on OMS medium containing 0.1 mg/l 2,4-D, 6% (w/v) sucrose and 25 mM L-proline solidified with 0.24% (w/v) Gelrite® (initiation medium). After two weeks' culture in the dark at 27°C, the callus developing on the scutellum is removed from the embryo and plated on B5 medium, (Gamborg, O.L. et al., Experimental Cell Research 50, 151-158 (1968)), containing 0.5 mg/l 2,4-D and solidified with 0.24% (w/v) Gelrite®. The callus is subcultured every two weeks to fresh medium. After a total of eight weeks after placing the embryos on the initiation medium, the special type of callus is identified by its characteristic morphology. This callus is subcultured further on the same medium. After a further period of two months, the callus is transferred to, and serially subcultured on, N6 medium containing 2 mg/l 2,4-D and solidified with Gelrite®.

EXAMPLES 41 Preparation of a Suspension Culture of Zea Mays, Elite Inbred Funk 2717

The callus described in Example 40 is subcultured for a total of at least six months. The type of callus chosen for subculture is relatively non-mucilaginous, granular and very friable, such that it separated into small individual cell aggregates upon placing into liquid medium. Cultures containing aggregates with large, expanded cells are not retained. Approximately 500 mg aliquots of the special callus of Zea mays elite inbred funk 2717 are placed into 30 ml of N6 medium containing 2 mg/l 2,4-D in 125 ml Delong flasks. After one week of culture at 26°C in the dark on a gyratory shaker (130 rpm, 2.5 cm throw), the medium is replaced with fresh medium. The suspensions are again subcultured in this way after another week. At that time, the cultures are inspected, and those which did not show large numbers of expanded cells are retained. Suspension cultures containing aggregates with large, expanded cells are discarded. The preferred tissue consisted of densely cytoplasmic dividing cell aggregates which had a characteristically smoother surface than the usual type of cell aggregates. The cultures retained had at least 50% of the cells represented in these small aggregates. This is the desired morphology. These suspensions also had a rapid growth rate, with a doubling time of less than one week. The suspension cultures are subcultured weekly by transferring 0.5 ml PCV (packed cell volume: settled cell volume in a pipette) into 25 ml of fresh medium. After four to six weeks of subculture in this fashion, the cultures increased two- to three-fold per weekly subculture. Cultures in which more than 75% of the cells are of the desired morphology are retained for further subculture. The lines are maintained by always choosing for subculture the flask whose contents exhibited the best morphology. Periodic filtration through 630 µm pore size stainless steel sieves every two weeks is used in some cases to increase the dispersion of the cultures, but is not necessary.

EXAMPLE 42 Preparation of Protoplasts from Suspension Cultures of Zea Mays

1-1.5 ml PCV of the suspension culture cells prepared as in Example 41 are incubated in 10-15 ml of a filter-sterilized mixture consisting of 4% (w/v) cellulase RS with 1% (w/v) Rhozyme in KMC (8.65 g/l KCl, 16.47 g/l $MgCl_2.6H_2O$ and 12.5 g/l $CaCl_2.2H_2O$, 5 g/l MES, pH 5.6) salt solution. Digestion is carried out at 30°C on a slow rocking table for a period of 3-4 hours. The preparation is monitored under an inverted microscope for protoplast release. The protoplasts which are released are collected as follows: The preparation is filtered through a 100 µm mesh sieve, followed by a 50 µm mesh sieve. The protoplasts are washed through the sieves with a volume of KMC salt solution equal to the original volume of enzyme solution. 10 ml of the protoplast preparation is placed in each of several disposable plastic centrifuge tubes, and 1.5-2 ml of 0.6 M sucrose solution (buffered to pH 5.6 with 0.1% (w/v) morpholinoethane sulfonic acid (MES and KOH)) layered underneath. The tube is centrifuged at 60-100 x g for 10 minutes, and the protoplasts banding at the interface collected using a pipette and placed in a fresh tube. The protoplast preparation is resuspended in 10 ml of fresh KMC salt solution, and centrifuged for five minutes at 60-100 x g. The supernatant is removed and discarded, and the protoplasts resuspended gently in the drop remaining, and then 10 ml of a 13/14 strength KMC solution gradually added. After centrifuging again for five minutes, the supernatant is again removed and the protoplasts resuspended in a 6/7 strength KMC solution. An aliquot is taken for counting, and the protoplasts again sedimented by centrifugation. The protoplasts are resuspended at $10^7$ (ten million) per ml in KM-8p medium (Table I) or in 0.5 M mannitol containing 6 mM $MgCl_2$ or other suitable medium for use in transformation as described in the following examples. This protoplast suspension is used for transformation and is cultured as described in Examples 43 and 44.

EXAMPLE 43 Transformation of Zea Mays Protoplasts by Electroporation

A. All steps except the heat shock are carried out at room temperature (22-28°C). The protoplasts are resuspended in the last step of Example 42 in 0.5 M mannitol containing 0.1% (w/v) MES and 6 mM $MgCl_2$. The resistance of this suspension is measured in the chamber of a Dialog Electroporator (DIA-LOG G.m.b.H., D-4000 Duesseldorf 13, Federal Republic of Germany) and adjusted to 1-1.2 kOhm using a 300 mM $MgCl_2$ solution. The protoplasts are heat-shocked by immersing the tube containing the sample in a water bath at 45°C for five minutes, followed by cooling to room temperature on ice. 4 µg of linearized plasmid containing a plant-selectable hygromycin resistance gene such as described by Rothstein, S.J. et al., supra, or chimeric gene constructs as described in Examples 19 to 31, and 20 µg of calf thymus carrier DNA are added to aliquots of 0.25 ml of this suspension. 0.125 ml of a 24% (w/v) polyethylene glycol (PEG) solution (MW 8000) in 0.5 M mannitol containing 30 mM $MgCl_2$ are added to the protoplasts. The mixture is mixed well but gently, and incubated for 10 minutes. The sample is transferred to the chamber of the electroporator and samples pulsed three times at 10 second intervals, at initial voltages of 1500, 1800, 2300 or 2800 Vcm-1, and an exponential decay time of 10 µsec.

The protoplasts are cultured as follows. The samples are plated in 6 cm petri dishes at room temperature. After a further 5-15 minutes, 3 ml of KM-8p medium (Table I, supra) containing 1.2% (w/v) SeaPlaque agarose and 1 mg/l 2,4-D are added. The agarose and protoplasts are mixed well and the medium allowed to gel.

B. Example 43 A is repeated with one or more of the following modifications:

(1) The resistance of the protoplast preparation is adjusted to 0.5-0.7 kOhm.

(2) The PEG used is PEG with a molecular weight of 4000.

(3) No PEG is added, or one-half volume of 12% (w/v) PEG is added.

(4) The pulses are applied at intervals of three seconds.

(5) The protoplasts are plated after the electroporation in dishes placed on a plate cooled to a temperature of 16°C.

(6) The protoplasts are placed in tubes after the electroporation step, washed with 10 ml of 6/7 strength KMC solution or with W5 solution (comprised of 380 mg/l KCl, 18.375 g/l $CaCl_2.2H_2O$, 9 g/l NaCl; 9 g/l glucose, pH 6.0), then collected by centrifugation at 60 g for 10 minutes, resuspended in 0.3 ml of KM medium, and plated as in A.

(7) The calf thymus carrier DNA is not added.

EXAMPLE 44 Transformation of *Zea Mays* Protoplasts by Treatment with Polyethylene Glycol (PEG)

A. The protoplasts are resuspended at the last step of Example 42 in a 0.5 M mannitol solution containing 12-30 mM $MgCl_2$. A heat shock of 45 °C for five minutes is given as described in Example 43. The protoplasts are distributed in aliquots for transformation in centrifuge tubes, 0.3 ml of suspended protoplasts per tube. During the next 10 minutes the following are added: DNA (as for Example 43) and polyethylene glycol (PEG) solution (MW 6000, 40% (w/v); containing 0.1 M $Ca(NO_3)_2$ and 0.4 M mannitol; pH 8-9 with KOH) to give a final concentration of 20% PEG. The aliquots are incubated for 30 minutes with occasional gentle shaking, and then the protoplasts are placed in petri dishes (0.3 ml original protoplast suspension per 6 cm diameter dish) and cultured as described in Example 43.

B. Example 44 A is repeated and the protoplasts are washed after 30 minutes of incubation in the PEG solution of Example 44 A, by adding 0.3 ml of W5 solution five times at two- to three-minute intervals. The protoplast suspension is centrifuged, the supernatant removed, and the protoplasts are cultured as for Example 43 A.

C. Examples 44 A and 44 B are repeated with the modification that the final concentration of PEG is between 13 and 25% (w/v).

EXAMPLE 45 Regeneration of Callus From Protoplasts

The plates containing the protoplasts in agarose are placed in the dark at 26 °C. After 14 days, colonies arise from the protoplasts. The agarose containing the colonies is transferred to the surface of a 9 cm diameter petri dish containing 30 ml of N6 medium (Table I, *supra*) containing 2 mg/l 2,4-D, solidified with 0.24% w/v Gelrite®. This medium is referred to as 2N6. The callus is cultured further in the dark at 26 °C and callus pieces subcultured every two weeks onto fresh solid 2N6 medium.

EXAMPLE 46 Selection of Transformed Callus of *Zea Mays*

Example 45 is repeated with the modification that 100 mg/l or 200 mg/l hygromycin B is added to the 2N6 medium in order to select for transformed cells.

EXAMPLE 47 Regeneration of Corn Plants

A. Callus is placed on 2N6 medium for maintenance and on ON6 (comprising N6 medium lacking 2,4-D) and N61 medium (comprising N6 medium containing 0.25 mg/l 2,4-D and 10 mg/l kinetin) to initiate regeneration. Callus growing on ON6 and N61 media is grown in the light (16 hours/day light of 10-100 $\mu$Einsteins/$m^2$sec from white fluorescent lamps). Callus growing on N61 medium is transferred to ON6 medium after two weeks, as prolonged time on N61 medium is detrimental. The callus is subcultured every two weeks even if the callus is to be transferred again on the same medium formulation. Plantlets appear in about four to eight weeks. Once the plantlets are at least 2 cm tall, they are transferred to ON6 medium in GA7 containers. Roots form in two to four weeks, and when the roots look well-formed enough to support growth, the plantlets are transferred to soil in peat pots, under a light shading for the first four to seven days. It is often helpful to invert a clear plastic cup over the transplants for two to three days to assist hardening off. Once the plants are established, they are treated as normal corn plants and grown to maturity in the greenhouse. In order to obtain progeny plants are self pollinated or crossed with wild type.

B. Example 47 A is repeated with the modification that 100 mg/l or 200 mg/l hygromycin B is added to the medium used to maintain the callus.

EXAMPLE 48 Preparation of Embryogenic Suspensions from Tissue of Dactylis Glomerata L. (Orchardgrass)

A. Embryogenic callus is initiated from basal sections of the youngest leaves of greenhouse-grown orchardgrass plants (Dactylis glomerata L.) as described by Hanning, G.E. et al., Theor. Appl. Genet., 63, 155-159 (1982). The leaves are surface sterilized by immersion in a 1:10 dilution of Clorox solution (5.25% (w/v) sodium hypochlorite; The Clorox Company, Oakland, Ca.) for about 10 minutes and then cut aseptically into small segments of 1 to 5mm in length or in diameter. These segments are plated on sterile SH-30 medium containing 0.8% (w/v) agarose as a gelling agent. Callus and/or embryogenic structures appear within 2 to 6 weeks after plating, upon culture at about 25° C. Embryogenic callus is maintained by subculturing onto fresh SH-30 medium every 2 to 4 weeks and culturing in the dark at 25° C.

B. Embryogenic suspension cultures are initiated by placing approximately 0.5 g fresh weight of embryogenic callus into 50 ml of liquid medium described by Gray, D.J. et al., Plant Cell Tissue Organ Cult., 4, 123-133 (1985) containing 45 µM dicamba and 4 g/liter casein hydrolysate. The suspension cultures are grown at 27° C under a 16 hours light (40 $\mu E/m^2 sec$), 8 hours dark photoperiod on a gyratory shaker at about 130 rpm in 125 ml Delong flasks sealed with a metal cap and parafilm®. After approximately four weeks the large clumps are allowed to settle for about 30 seconds and 10 ml aliquots of the supernatant medium containing small cell clusters are removed and transferred to 50 ml of fresh medium. This process is repeated every 3 to 4 weeks using the most successful cultures as judged by smaller clump size and better quality based on the presence of small, cytoplasmic cells. After 5 to 8 transfers the suspensions are essentially free of non embryogenic cells and the majority of the embryogenic cell clusters are quite small (150 to 2000 µm).

EXAMPLE 49 Isolation and Purification of Dactylis Glomerata L. Protoplasts

Protoplasts are prepared from embryogenic suspension cultures of Example 48 by aseptically filtering the cells on a Nalgene® 0.2 µm filter unit and then adding 0.5 g fresh weight cells to each 12.5 ml of protoplast enzyme mixture in a petri dish. The enzyme mixture consists of 2% (w/v) Cellulase RS, 7 mM $CaCl_2$ x $H_2O$, 0.7 mM $NaH_2PO_4$ x $H_2O$, 3 mM MES (pH 5.6), glucose (550 mOs/kg $H_2O$ of pH 5.6), and is filter sterilized. The mixture is swirled on an orbital shaker at about 50 rpm in dim (< 5 $\mu E/m^2 sec$) light for about 4 to 5 hours. The digest is then sieved through a stainless steel sieve (100 µm mesh size) and distributed into 12 ml centrifuge tubes which are centrifuged at about 60 to 100 g for about 5 minutes. The protoplast-containing sediment is then washed three times with protoplast culture medium KM-8p adjusted to 550 mOs/kg $H_2O$ with glucose. At this point a flotation step may be included for further purification of the protoplasts. In this case, the washed protoplasts are layered atop 10 ml of KM-8p culture medium adjusted 700 mOs/kg $H_2O$ with sucrose. After centrifugation at 60-100 x g for about 10 minutes, protoplasts banding at the interface are collected using a fine pipette. Finally, the protoplasts are resuspended in 1 to 2 ml KM-8p culture medium and sieved through a stainless mesh screen (20 µm mesh size). The protoplasts released are collected and washed and resuspended in KM-8p medium for culture or in osmotically adjusted medium suitable for transformation according to Examples 51 to 53.

EXAMPLE 50 Dactylis Glomerata L. Protoplast Culture and Growth of Callus

A. The purified protoplasts are plated at a density of about 5 x $10^5$ protoplasts per ml in KM-8p culture medium containing 1.3% (w/v) SeaPlaque® agarose (FMC Corp., Marine Colloids Division, Rockland, Maine, USA) and 30 to 40% (w/v) of conditioned medium (obtained from 3 to 4 week-old Dactylis glomerata L. embryogenic suspension cultures by filtering the medium through a sterile Nalgene® 0.2 µm filter, making the medium 550 mOsm/kg $H_2O$ by addition of glucose, and again filter sterilizing). The plates are then placed in the dark at a constant temperature of 28° C. After 10 to 14 days the agarose is cut into wedges and placed into 'bead culture' as described by Shillito, R.D. et al., Plant Cell Reports, 2, 244-247 (1983) using 20 ml SH-45 suspension culture medium with 3% (w/v) sucrose per 3 ml original agarose embedded culture. The plates are put on a platform shaker and agitated at about 50 rpm in light at 8

μE/m²sec. New suspension cultures are formed as the colonies grow out of the agarose and release cells into the liquid medium. The resultant suspension cultured cells are plated onto agar-solidified SH-30 medium and placed in the dark at 25° C until callus is formed.

B. Protoplasts are cultured as described in Example 50 A above except that the culture media contains no conditioned medium.

EXAMPLE 51 Transformation of Dactylis Glomerata L. Protoplasts by Means of Electroporation

A. Immediately after purification of the protoplasts, electroporation is performed according to Shillito, R.D. et al., Bio/Technology 3, 1099-1103 (1985) using linearized plasmid as described in Example 20. The protoplasts are resuspended after the last wash at a density of about 7 x $10^6$ protoplasts per ml in the electroporation buffer (0.4 M mannitol, 6 mM $MgCl_2$). The protoplasts are placed in 0.7 ml aliquots in 10 ml plastic centrifuge tubes. Plasmid DNA of Example 20 and sonicated calf thymus DNA (Sigma) to give final concentrations of 10 μg/ml and 50 μg/ml respectively is added to the tubes. Then 0.38 ml polyethylene glycol (PEG) solution [24% (w/v) PEG 6000 in 0.4 M mannitol 30 mM $MgCl_2$, 0.1% (w/v) MES (pH 5.6)] is added and the solution gently mixed. The protoplast suspension is transferred into the chamber of a Dialog® Electroporator and 10 pulses of 3250 V/cm initial voltage and exponential decay constant of 10 μsec applied at 30 sec intervals. The sample is removed from the chamber, and placed in a 10 cm diameter petri dish. 10 ml of KM-8p medium containing 1.2% (w/v) SeaPlaque® agarose is added, the protoplasts distributed evently throughout the medium, and the agarose allowed to gel.

B. Example 51 A is repeated except that the initial voltage used is 3500 V/cm, 4000 V/cm, 5000 V/cm, 3000 V/cm, or 2500 V/cm.

C. Examples 51 A and B are repeated except that PEG of MW 4000 or PEG of MW 8000 is used.

D. Examples 51 A to C are repeated except that the final PEG concentration is between 10% and 30% (w/v).

EXAMPLE 52 Transformation of Dactylis Glomerata L. Protoplasts by Treatment with Polyethylene Glycol (PEG)

A. PEG mediated direct gene transfer is performed according to Negrutiu, I. et al., supra. The DNA used is linearized plasmid described in Example 20.

The protoplasts are suspended following the last wash in 0.5 M mannitol containing 15 mM $MgCl_2$ at a density of about 2 x $10^6$ per ml. The protoplast suspension is distributed as 1 ml aliquots into 10 ml plastic centrifuge tubes. The DNA is added as described in Example 51 above, and then 0.5 ml of the PEG solution added (40% (w/v) PEG 4000 in 0.4 M mannitol, 0.1 M $Ca(NO_3)_2$, pH 7.0). The solutions are mixed gently and incubated for 30 minutes at room temperature (about 24° C) for 30 minutes with occasional shaking. 1.4 ml of the wash solution is then added, and the contents of the tube gently mixed. The wash solution consists of 87 mM mannitol, 115 mM $CaCl_2$, 27 mM $MgCl_2$, 39 mM KCl, 7 mM Tris-HCl and 1.7 g/l myo-inositol, pH 9.0. Four further 1.4 ml aliquots of wash solution are added at 4 minute intervals, with mixing after each addition. The tube is then centrifuged at about 60 x g for about 10 minutes, and the supernatant discarded. The sedimented protoplasts are taken up in 1 ml KM-8p culture medium, and placed in a 10 cm petri dish. 10 ml of KM-8p medium containing 1.2% (w/v) SeaPlaque® agarose is added. The protoplasts are evenly distributed throughout the medium, and the agarose allowed to gel.

B. Example 52 A is repeated with one or more of the following modifications:

(1) The pH of the wash solution is adjusted to 5.6 or 7.0.

(2) The PEG used is PEG of MW 6000, PEG of MW 2000 or PEG of MW 8000.

(3) The wash medium consists of 154 mM NaCl, 125 mM $CaCl_2$, 5 mM KCl, 5 mM glucose, pH to 6.0 with KOH, of 0.2 M $CaCl_2$, 0.1% (w/v) MES, pH 6.0 with KOH, or of 0.2 M $CaCl_2$, 7 mM Tris/HCl, pH 9.0 with KOH.

EXAMPLE 53 Transformation of Dactylis Glomerata L. Protoplasts by Electroporation or PEG Treatment

Transformation is carried out as described in Examples 51 or 52, except that the protoplasts are treated at 45 °C for about 5 minutes prior to distribution of the aliquots into tubes for transformation or after distribution of the aliquots, and before addition of the PEG.

EXAMPLE 54 Selection of Transformed Colonies

A. The culture plates (petri dishes) containing the protoplasts from Examples 51 to 53 are incubated for 10 days in the dark at about 25 °C and then cut into 5 equal slices for 'bead cultures' (Shillito, R.D. et al., Plant Cell Reports 2, 244-247 (1983)). Four of the slices are placed each into 20 ml SH-45 culture medium with 4 g/l casein hydrolysate and 20 µg/ml hygromycin B. The fifth slice is put into 20 ml of the same medium but without hygromycin B as a non-selected control. After 4 to 5 weeks the putative transformed protoplast-derived cell colonies growing in hygromycin B are cut out of the agarose and placed into a 19 mm petri dish with 2 ml of liquid SH-45 medium containing 20 µg/ml hygromycin B, which is agitated at about 50 rpm on an orbital shaker. After another 4 to 5 weeks all colonies which grow to make new suspensions are transferred into 125 ml Erlenmeyer flasks and grown in a manner similar to the parent suspension culture, except that 20 µg/ml hygromycin B is included in the medium.
The new suspensions are subcultured every 1 to 3 weeks using SH-45 medium containing 4 g/l casein hydrolysate and 20 µg/ml hygromycin B. Cells from these suspensions are also plated on solidified SH-30 medium containing 20 µg/ml hygromycin B and incubated at about 25 °C in the dark. Calli grown from the plated cells are subcultured every two weeks onto fresh medium. The cells which grow in the presence of hygromycin B are presumed to be transformants.

B. Selection is carried out as described in Example 54 A except that the protoplast-derived cell colonies growing in hygromycin B containing medium are placed on agar plates of SH-30 medium containing 20 µg/ml hygromycin B and incubated at about 25 °C in the dark.

EXAMPLE 55 Regeneration of Transformed Dactylis Glomerata L. Plants

A. Dactylis Glomerata L. callus (obtained as described in Example 54) derived from protoplasts is grown on solidified SH-30 medium, and subcultured every two weeks. Any embryos which form are removed and plated on germination medium (SH-0) and placed in the light (45 to 55 µE/m²sec). Germination of these embryos occurs in 1 to 4 weeks and the resultant plantlets are placed on SH-0 medium in the light to form root systems. They are moved into the greenhouse at the six to twelve leaf stage, and hardened off gradually.

B. Callus (obtained as described in Example 54) derived from protoplasts is grown on SH-0 medium solidified with 0.24% (w/v) Gelrite® in the light (45 to 55 µE/m²sec), and subcultured every two weeks. The resultant plantlets are placed on a 1:1 mixture of SH-0 and OMS media solidified with a combination of 0.12% (w/v) Gelrite® and 0.4% (w/v) agar in the light to form root systems. They are moved to the greenhouse at the six to twelve leaf stage, and hardened off gradually.

C. Small plantlets are obtained as described in 44A and 44B above, and are placed on OMS medium solidified with 0.8% (w/v) agar in the light to form root systems. They are moved to the greenhouse at the six to twelve leaf stage, and hardened off gradually.

D. Small plantlets are obtained as described in 44A above and are placed on a 1:1 mixture of SH-0 and OMS media solidified with a combination of 0.12% (w/v) GelRite® and 0.4% (w/v) agar in the light to form root systems. They are moved to the greenhouse at the six to twelve leaf stage, and hardened off gradually.

6. TRANSIENT GENE EXPRESSION

The following examples describe how the chimeric genes can be introduced and utilized without the gene necessarily being stably incorporated into the genome of the plant.

EXAMPLE 56 Introduction of DNA into Protoplasts of N. Tabacum by Treatment with PEG.

A. Preparation of protoplasts of N. tabacum can be carried out in accordance with the methods described in the following publications: Paszkowski, J. et al., EMBO J. 3, 2717 (1984); GB patent application 2 159 173, European Patent Application EP 0 129 668; Shillito, R.D. and Potrykus, I. In: Methods in Enzymology, eds. Wu, R. and Grossman, L., Academic Press, Orlando, Florida, Vol. 153, 313-306, (1987) or by other methods known in the art. These publications are incorporated by reference.

B. DNA is introduced into protoplasts by a modification of the method of Negrutiu, I. et al., Plant Mol. Biol. 8, 363 (1987). The protoplasts prepared as described in Example 56 A are resuspended following the last washing step in a solution consisting of 0.4 M mannitol, 15-30 mM $CaCl_2$, 0.1% w/v MES at a density of 1.6-2 x $10^6$ per ml. The protoplast suspension is distributed as 0.5 ml aliquots into 10 ml plastic centrifuge tubes. The DNA of Examples 19 to 31 is added in 10 µl sterile distilled water, sterilized as described by Paszkowski, J. et al., EMBO J. 3, 2717 (1984), and then 0.5 ml of the PEG solution (40% (w/v) PEG MW 8000 in 0.4 M mannitol, 0.1 M $Ca(NO_3)_2$, pH 7.0) is added. The solutions are mixed gently and incubated for 30 minutes at room temperature (about 24° C) with occasional shaking. 1 ml of the wash solution is then added, and the contents of the tube gently mixed. The wash solution consists of 154 mM NaCl, 125 mM CaCl2, 5 mM KCl, 5 mM glucose, pH to 6.0 with KOH. Further aliquots of 2 ml, 3 ml and 4 ml of wash solution are added sequentially at 5 minute intervals, with mixing after each addition. The tube is then centrifugated at about l0-l00 x g for about 10 minutes, and the supernatant discarded. The pelleted protoplasts are taken up in sufficient K3 culture medium with 0.3 M glucose as the osmoticum, and no sucrose, to achieve a final density of 100,000 per ml and cultured in a 10 cm petri dish.

C. Example 56 B is repeated with one or more of the following modifications:

(1) The pH of the wash solution is adjusted to 5.6 or 7.0.

(2) The PEG used is PEG with a molecular weight of 4000.

(3) The wash medium consists of 0.2 M $CaCl_2$, 0.1% w/v MES, pH 6.0 with KOH, or of 0.2 M $CaCl_2$, 7 mM Tris/HCl, pH 9.0 with KOH.

(4) 50 µg of sheared calf thymus DNA in 25µl sterile water is added together with the plasmid DNA.

(5) The plasmid DNA is linearized before use by treatment with an appropriate restriction enzyme (e.g. BamHI) .

EXAMPLE 57 Introduction of DNA into Protoplasts of N. Tabacum by Electroporation.

A. Introduction of DNA into protoplasts of N. tabacum is effected by treatment of the protoplasts with an electric pulse in the presence of the appropriate DNA, in a modification of the methods of Fromm, M.E. In: Methods in Enzymology, eds. Wu, R. and Grossman, L., Academic Press, Orlando, Florida, Vol. 153, 307, (1987); and Shillito R.D and Potrykus I., ibid., p. 283-306.

Protoplasts are isolated as described in Example 56 A. The protoplasts are resuspended following the last wash in the following solution: 0.2 M mannitol, 0.1% w/v MES / 72 mM NaCl, 70 mM $CaCl_2$, 2.5 mM KCl, 2.5 mM glucose, pH to 5.8 with KOH, at a density of 1.6-2 x $10^6$ per ml. The protoplast suspension is distributed as 1 ml aliquots into plastic disposable cuvettes and 10 µg of DNA added as described in Examples 56 B and C. The resistance of the solution at this point when measured between the electrodes of the 471 electrode set of the electroporation apparatus described below is in the range of 6 Ohms.

The DNA of Examples 19 to 31 is added in 10 µl sterile distilled water, sterilized as described by Paszkowski, J. et al., EMBO J. 3, 2717 (1984). The solution is mixed gently and then subjected at room temperature (24-28° C) to a pulse of 400 V/cm with an exponential decay constant of 10 ms from a BTX-Transfector 300 electroporation apparatus using the 471 electrode assembly. The protoplasts are left undisturbed for 5 minutes, and then placed in a petri dish and K3 medium as described in Example 56 A added to bring the density of protplasts to 100,000 per ml.

B. Example 57 A is repeated with one or more of the following modifications:

(1) The voltage used is 200 V/cm, or between l00 V/cm and 800 V/cm.

(2) The exponential decay constant is 5 ms, 15 ms or 20 ms.

(3) 50 µg of sheared calf thymus DNA in 25µl sterile water is added together with the plasmid DNA.

(4) The plasmid DNA is linearized before use by treatment with an appropriate restriction enzyme (e.g. BamHI).

EXAMPLE 58 Introduction of DNA into Protoplasts of Zea Mays Line 2717.

Protoplasts of maize inbred line Funk 2717 are prepared as described in Examples 40 to 47 above, and resuspended in in either of the solutions described for resuspension of the N. tabacum protoplasts in Examples 56 and 57 above at a density of $10^7$ per ml. Transformation is carried out essentially as described in Examples 56 and 57. The protoplasts are cultured following transformation at a density of 2 x $10^6$ per ml in KM-8p medium with no solidifying agent added and containing 1 mg/l 2,4-D.

EXAMPLE 59 Introduction of DNA into Protoplasts of Sorghum Bicolor.

Protoplasts of sorghum suspension FS 562 are prepared essentially as described for Zea mays in Example 40 above, and resuspended in in either of the solutions described for resuspension of the N. tabacum protoplasts in Examples 56 and 57 above at a density of $10^7$ per ml. Transformation is carried out essentially as described in Example 58. The protoplasts are cultured following transformation at a density of 2 x $10^6$ per ml in KM-8p medium, with no solidifying agent added.

EXAMPLE 60 Introduction of DNA into Protoplasts of Nicotiana Plumbaginifolia, Petunia Hybrida andLolium Multiflorum.

Protoplasts of N. plumbaginifolia, P. hybrida or L. multiflorum are prepared as described in Shillito and Potrykus, supra, and treated as described in Examples 56 and 57. They are cultured in the medium described by Shillito and Potrykus, supra, without addition of agarose or any other gelling agent.

EXAMPLE 61 Introduction of DNA into Protoplasts of Glycine Max

Protoplasts of Glycine max are prepared by the methods as described by Tricoli, D.M. et al., Plant Cell Reports, 5, 334-337 (1986), or Chowhury, V.K. and Widholm, J.M., Plant Cell Reports 4, 289-292 (1985), or Klein, A.S. et al., Planta 152, 105-114 (1981). DNA is introduced into these protoplasts essentially as described in Examples 56 and 57. The protoplasts are cultured as described in Klein, A.S. et al., supra, Chowhury V.K. and Widholm, J.M.. supra, or Tricoli, D.M. et al., supra, without the of addition of alginate to solidify the medium.

## 7. CHEMICAL REGULATION OF TRANSGENIC PLANT TISSUE

The following examples describe procedures for the chemical regulation of chemically regulatable genes in representative transgenic plant tissue. While the following examples are directed to induction, similar procedures would be applicable for systems operating by repression.

A variety of techniques are available for the treatment of transgenic cells with a chemical regulator. For example, cells may be suspended in liquid medium containing the regulator. Callus may be grown on, or transferred to medium containing the regulator, or the regulator may be applied to callus with a sterile paint brush or plant mister. Likewise plants or parts of plants are treated with a solution or a suspension of the regulator by the way of a paint brush or, preferably, a plant mister.

Expression of the phenotypic trait in transgenic plants containing a chemically regulatable chimeric gene is proportional to the amount of the regulator applied to the plant or plant tissue.

EXAMPLE 62 Chemical Induction in Transgenic Callus Cultures

A. Callus obtained according to previously described procedures is assayed for chemical induction by applying with a sterile paint brush or a plant mister a filter sterilized solution of 0.1 to 50 mM salicylic acid which has been adjusted to a pH between 6.0 and 8.0 by the addition of NaOH.
After induction the callus is allowed to incubate for two days and then harvested, frozen in liquid nitrogen and stored at -80°C until they are assayed for gene induction as described in Example 65 and/or Example 66.

B. Alternatively, the following solutions are used for induction:

(1) 0.1 to 50 mM benzoic acid which has been adjusted to a pH between 6.0 and 8.0 by the addition of NaOH.

(2) 0.1 to 50 mM polyacrylic acid which has been adjusted to a pH between 6.0 and 8.0 by the addition of NaOH.

(3) Methyl benzo-1,2,3-thiadiazole-7-carboxylate. The solution is made by resuspending a wettable powder containing the chemical in sterile water for several minutes at a concentration of 1 mg/ml. Insoluble solid is removed from the solution by filter sterilizing.

(4) 0.1 to 50 mM benzo-1,2,3-thiadiazole-7-carboxylic acid, pH between 6.0 and 8.0. The compound is dissolved in a minimum of dimethyl sulfoxide (DMSO), then diluted with sterile water to the desired concentration. The obtained suspension is applied without filtering.

(5) 0.1 to 50 mM n-propyl benzo-1,2,3-thiadiazole-7-carboxylate, prepared as in (4).

(6) 0.1 to 50 mM benzyl benzo-1,2,3-thiadiazole-7-carboxylate, prepared as in (4).

(7) 0.1 to 50 mM benzo-1,2,3-thiadiazole-7-carboxylic acid N-sec-butylhydrazide, prepared as in (4).

(8) 0.1 to 50 mM 2,6-dichloroisonicotinic acid, pH between 6.0 and 8.0, prepared as in (4).

(9) 0.1 to 50 mM methyl 2,6-dichloroisonicotinate, prepared as in (4).

EXAMPLE 63 Chemical Induction in Transgenic Plants

A. Gene expression is induced in plants obtained according to previously described procedures by applying a fine spray of a solution of 0.1 to 50 mM salicylic acid which has been adjusted to a pH between 6.0 and 8.0 with NaOH, to the leaves using a plant mister.

The plants are allowed to continue to grow for two days and are then harvested, frozen in liquid nitrogen and stored at -80 °C until they are assayed as described in Example 65 and/or Example 66.

B. Alternatively, the following solutions are used for induction:

(1) 0.1 to 50 mM benzoic acid which has been adjusted to a pH between 6.0 and 8.0 by the addition of NaOH.

(2) 0.1 to 50 mM polyacrylic acid which has been adjusted to a pH between 6.0 and 8.0 by the addition of NaOH.

(3) Methyl benzo-1,2,3-thiadiazole-7-carboxylate. The solution is made by resuspending a wettable powder containing the chemical in sterile water for several minutes at a concentration of 1 mg/ml. Insoluble solid is removed from the solution by filter sterilizing.

(4) 0.1 to 50 mM benzo-1,2,3-thiadiazole-7-carboxylic acid, pH between 6.0 and 8.0. The compound is dissolved in a minimum of dimethyl sulfoxide (DMSO), then diluted with sterile water to the desired concentration. The obtained suspension is applied without filtering.

(5) 0.1 to 50 mM n-propyl benzo-1,2,3-thiadiazole-7-carboxylate, prepared as in (4).

(6) 0.1 to 50 mM benzyl benzo-1,2,3-thiadiazole-7-carboxylate, prepared as in (4).

(7) 0.1 to 50 mM benzo-1,2,3-thiadiazole-7-carboxylic acid N-sec-butylhydrazide, prepared as in (4).

(8) 0.1 to 50 mM 2,6-dichloroisonicotinic acid, pH between 6.0 and 8.0, prepared as in (4).

(9) 0.1 to 50 mM methyl 2,6-dichloroisonicotinate, prepared as in (4).

EXAMPLE 64 Induction of Expression of the Introduced DNA in Protoplasts of N. Tabacum, Zea Mays, N. Plumbaginifolia, P. Hybrida, L. Multiflorum and Sorghum Bicolor

The protoplasts treated as described in Examples 56 to 61 above are cultured at 26 °C in the dark for 2 days. After this time salicylic acid is added to a concentration of between 0.1 to 50 mM as a neutralized solution, pH between 6.0 and 8.0. The protoplasts are cultured for a further 2 days, harvested by centrifugation, quick frozen and assayed as described in the following Examples.

B. Alternatively, neutralized solutions or suspensions of the following compounds are added to the protoplasts to give a final concentration of between 0.1 and 50 mM:

(1) benzoic acid

(2) polyacrylic acid

(3) methyl benzo-1,2,3-thiadiazole-7-carboxylate
(4) benzo-1,2,3-thiadiazole-7-carboxylic acid
(5) n-propyl benzo-1,2,3-thiadiazole-7-carboxylate
(6) benzyl benzo-1,2,3-thiadiazole-7-carboxylate
(7) benzo-1,2,3-thiadiazole-7-carboxylic acid N-sec-butylhydrazide
(8) 2,6-dichloroisonicotinic acid
(9) methyl 2,6-dichloroisonicotinate

C. Alternatively, induction is carried out as described in Example 64 A and B except that the protoplasts are cultured for 1 day, 3 days or 5 days before induction.

D. Induction is carried out as described in Examples 64 A, B or C except that the protoplasts are cultured for 1 day, 3 days or 5 days after induction and before being harvested.

EXAMPLE 65 Assay for Chemically Inducible DNA Sequence: mRNA Production

Plant materials induced and harvested as in Examples 62 to 64 are assayed for the induction of mRNA species by the isolation of RNA and the primer extension assay as described in Example 5.

Callus or regenerated plant material derived from transgenic plants containing chemically regulatable chimeric genes coding for GUS, AHAS or BT and induced by salicylic acid, methyl benzo-1,2,3-benzothiadiazole-7-carboxylate, benzo-1,2,3-thiadiazole-7-carboxylic acid, n-propyl benzo-1,2,3-thiadiazole-7-carboxylate, benzyl benzo-1,2,3-thiadiazole-7-carboxylate, benzo-1,2,3-thiadiazole-7-carboxylic acid N-sec-butylhydrazide, 2,6-dichloroisonicotinic acid, methyl 2,6-dichloroisonicotinate, or TMV demonstrates elevated levels of mRNA upon assay with the GUS, AHAS or BT primer, respectively, in the primer extension assay. The level of mRNA is proportional to the amount of regulator applied.

EXAMPLE 66 Assay for Chemically Inducible DNA Sequences: β-Glucuronidase Enzymatic Activity

A. ELISA assay

Frozen leaf tissue is ground in a mortar with a pestle in the presence of liquid nitrogen to produce a fine powder. Leaf extracts are prepared by mixing a given weight (g) with an equal volume (ml) of GUS extraction buffer (50 mM sodium phosphate buffer pH 7.0, 0.1% Triton-X 100, 0.1% sarkosyl, 10 mM β-mercaptoethanol) as described by Jefferson, R.A. et al., Proc. Natl. Acad. Sci. USA 83, 8447-8451 (1986).

The reactions are carried out in the wells of ELISA plates by mixing 5-25 µl extract with 120-100 µl GUS assay buffer (50 mM sodium phosphate buffer pH 7.0, 0.1% Triton X-100, 10 mM β-mercaptoethanol) containing 4-methyl umbelliferyl glucuronide (MU) at a final concentration of 2 mM in a total volume of 125 µl. The plate is incubated at 37°C for 1-5 hours and the reaction is stopped by the addition of 150 µl 3 M $Na_2CO_3$. The concentration of fluorescent indicator released is determined by reading the plate on a Flow Labs Fluoroskan II ELISA plate reader.

B. Results

TABLE II

Specific Activity nKat MU/mg protein

| Plant [a] | A | B | C | D | E [b] |
|---|---|---|---|---|---|
| H 1 | 5.39 ±1.9 | 13.9 ±1.2 | 13.5 ±0.8 | 20.1 ±5.1 | 33.6 ±2.6 |
| H 3 | 0.21 ±0.02 | 6.92 ±0.88 | 11.95 ±0.85 | 1.66 ±0.02 | 6.54 ±1.43 |
| H 4 | 8.39 ±1.31 | 27.5 ±0.7 | 18.52 ±1.07 | 2.28 ±0.78 | 25.1 ±0.9 |
| H 6 | 0.10 ±0.04 | 4.08 ±0.58 | 2.58 ±.01 | 0.09 ±0.06 | 2.8 ±1.2 |
| H 9 | 0.23 | 10.8 | 9.1 | 0.5 | 7.5 |
| Alu 1 | 5.6 | 20.0 | 206.9 | 20.5 | 70.5 |
| Alu 2 | 2.0 | 6.6 | 83.2 | 8.9 | 155.3 |
| Alu 3 | 0.1±.07 | 2.8±.9 | 2.0±.9 | 2.0±.5 | 5.8±3.8 |
| Alu 4 | 2.1±1 | 43.0±19 | 7.1±2.6 | 6.1±3.2 | 7.5±3.5 |
| Alu 5 | 0.6±.2 | 4.4±2.3 | 1.2±.6 | 0.5±.3 | 8.3±3.7 |
| Alu 6 | 0.8 | 28.8 | 67.0 | 3.1 | 36.4 |
| Alu 7 | 1.7 | 38.7 | 29.1 | 21.7 | 63.0 |
| Alu 8 | 0.6 | 0.4 | 0.4 | 0.6 | 0.2 |
| Alu 9 | 0.9 | 0.6 | 0.4 | 0.3 | nd |

| | | | | | |
|---|---|---|---|---|---|
| Alu 10 | 4.0 | 39.2 | 52.0 | 8.1 | 37.9 |
| Alu 11 | 1.6±.8 | 1.9±.9 | 2.8±1.3 | 2.2±2.6 | 2.8±2.9 |
| Alu 12 | 0.7±.4 | 57.1±22 | 73.4 | 2.3±1.2 | 78.0±41.4 |
| Alu 13 | 0.1 | 19.1 | 13.2 | 1.9 | 10.6 |
| Hae 1 | 0.6 | 1.6 | 2.6 | 1.7 | 3.9 |
| Hae 2 | 2.3 | 8.8 | 11.8 | 0.4 | 0.8 |
| Hae 3 | 1.7 | 11.1 | 11.2 | 3.0 | 1.6 |
| Hae 4 | 2.0 | 8.1 | 10.8 | 1.7 | 0.82 |
| Hae 5 | 18.1 | 59.4 | 132.5 | 36.6 | 14.0 |
| Hae 8 | 0.4 | 30.6 | 28.9 | 0.6 | 29.0 |
| Hae 9 | 2.6 | 63.9 | 129.7 | 12.5 | 55.2 |
| Hae 10 | 1.6 | 70.5 | 98.2 | 13.5 | 94.5 |
| Hae 11 | 2.6 | 81.8 | 58.5 | 3.9 | 46.0 |
| Hae 12 | 0.2 | 2.5 | 5.8 | 0.6 | 14.5 |
| Hae 13 | 0.01 | 0.7 | 0.1 | nd | nd |
| Hae 14 | 0.1 | 1.8 | 8.6 | 0.1 | 3.3 |
| Hae 15 | 0.1 | 6.4 | 7.2 | 3.4 | 7.4 |
| Hae 16 | 0.1 | 2.3 | 4.3 | 0.8 | 2.7 |

Footnotes

a H 1-9 are tobacco plants obtained by transformation of leaf disks by strain CIB271

Alu 1-13 are tobacco plants obtained by transformation of leaf disks by strain CIB272

Hae 1-16 are tobacco plants obtained by transformation of leaf disks by strain CIB273

b A Water

B Salicylic acid

C Methyl benzo-1,2,3-thiadiazole-7-carboxylate

D Buffer

E TMV

Transformed tobacco callus (Example 23):

The following GUS activities are obtained from samples of transformed callus which has been harvested 21 hours after treatment. Transformed with CIB271: $H_2O$ spray: 1.8 nKat MU/mg protein; 50 mM sodium salicylate spray: 3.8 nKat MU/mg protein. Transformed with CIB271 (different experiment): $H_2O$ spray; 2.8 nKat MU/mg protein; 250 µg/l methyl benzo-1,2,3-thiadiazole-7-carboxylate spray: 13.7 nKat MU/mg protein. Transformed with CIB273: $H_2O$ spray: 0.46 nKat MU/mg protein; 250 µg/l methyl benzo-1,2,3-thiadiazole-7-carboxylate spray: 31.2 nKat MU/mg protein.

Transformed carrot (Example 36):

The following GUS activities are obtained from samples of callus transformed by pCIB271 which has been harvested 21 hours after treatment. $H_2O$ spray: 5.2 pKat MU/mg protein. 50 mM sodium salicylate spray: 11.6 pKat MU/mg protein. 250 µg/l methyl benzo-1,2,3-thiadiazole-7-carboxylate spray: 22.1 pKat MU/mg protein.

Transformed tomato (Example 38):

The following GUS activities are obtained from samples of transformed callus which has been harvested 21 hours after treatment. Transformed with CIB271: $H_2O$ spray: 17.2 pKat MU/mg protein; 250 µg/l methyl benzo-1,2,3-thiadiazole-7-carboxylate spray: 56.1 pKat MU/mg protein. Transformed with CIB273 X A4: $H_2O$ spray: 4.6 pKat MU/mg protein; 250 µg/l methyl benzo-1,2,3-thiadiazole-7-carboxylate spray: 22.1 pKat MU/mg protein.

Transient expression in tobacco protoplasts (Example 56)

treated with pCIB269: $H_2O$: 30.6 ± 13 pKat MU/mg protein; methyl benzo-1,2,3-thiadiazole-7-carboxylate: 66.6 ± 20 pKat MU/mg protein.

EXAMPLE 67 Assay for Chemical Induction of the Endogenous Gene

As a control procedure, tobacco plant materials induced as described in Examples 62 to 64 by salicylic acid, methyl benzo-1,2,3-thiadiazole-7-carboxylate and TMV are assayed for transcription of mRNA from the endogenous PR-1a gene by the methods described in Example 5. Elevated levels of mRNA are detected after induction by all three inducers.

EXAMPLE 68 Assay for Chemically Inducible DNA Sequences: Acetohydroxyacid Synthase (AHAS) Enzymatic Activity

A. Sample preparation for AHAS assay

The weight of the leaf and/or root tissue material to be assayed is determined, the tissue is placed into liquid nitrogen and macerated to a fine powder. One volume (in ml) of cold homogenization buffer (50 mM $NaH_2PO_4$ buffer pH 7.0, 0.5 mM EDTA pH 7.0, 0.5 mM $MgCl_2$, 1 mM sodium pyruvate pH 7.0, 10 µM flavine adenine dinucleotide, 1 mM phenylmethylsulfonyl fluoride, 10% glycerol) equal to two times the weight (in g) of tissue is added and the mixture is transferred to a French pressure cell. All subsequent steps are carried out keeping the material cold (about 4° C). The cells are disrupted at 16,000 psi, collecting the cell exudate into a container on ice. Alternatively the tissue is ground with homogenization buffer in a mortar and pestle in place of the French pressure cell treatment. The exudate is centrifuged 5 min at 10,000 rpm to clear the cell debris. The volume of the supernate is measured and enzyme grade ammonium sulfate added to 25% saturation (144 mg $(NH_4)_2SO_4$/ ml supernate). The mixture is stirred on ice for 15 min, then centrifuged for 5 min at 10,000 rpm. The pellet is discarded and the supernate adjusted to 50% saturation with ammonium sulfate (158 mg $(NH_4)_2SO_4$/ ml supernate). After stirring on ice for 15 min, the pellet is collected by centrifugation at 10,000 rpm for 5 min. The pellet obtained is dissolved in 1 ml of column buffer for each 2 g of leaf or 5 g of root material. The extract is desalted by application to a Sephadex G50 column equilibrated with column buffer (50 mM $NaH_2PO_4$ buffer pH 7.0, 0.1 mM EDTA, 0.5 mM $MgCl_2$, 10 µM flavine adenine dinucleotide, 1 mM phenylmethylsullfonyl fluoride, 10% glycerol). The sample is eluted with column buffer, collecting 2 ml of sample for each ml of sample applied to the column.

B. Tube assay

5-50 µl of an extract prepared according to Example 68 A are dissolved in a total volume of 0.5 ml of reaction mixture (20 mM $NaH_2PO_4$ buffer pH 7.0, 20 mM sodium pyruvate pH 7.0, 0.5 mM thiamine

pyrophosphate, 5 mM $MgCl_2$, 10 µM flavine adenine dinucleotide). The mixture is incubated for 30 min at 37° C. The reaction is stopped by the addition of 50 µl of 6N $H_2SO_4$. The mixture is incubated for 15 min at 60° C, then treated with 625 µl of α-napthol reagent (500 µl 5% α-napthol in 2.5 M NaOH and 125 µl 25 mg/ml creatine) and incubated another 15 min at 60° C. If a precipitate has formed, this is spun out, and the absorbance measured at 520 mµ. The pMoles acetoin formed are calculated from a standard curve developed using 0.4 µg to 10 µg acetoin. The specific activity is expressed as pKat/mg protein.

C. Elisa plate assay

1-20 µl of a sample are placed in an Elisa plate well with a total volume of 0.15 ml of reaction mixture. The mixture is reacted for 30 min at 37° C. The reaction is stopped by the addition of 50 µl of 2.4 M $H_2SO_4$ containing 1% creatine. The mixture is incubated for 30 min at 60° C. Any precipitate present is separated by centrifugation. 150 µl of the assay mixture are transferred to a new Elisa plate, treated with 100 µl 10% α-napthol in 5M NaOH and incubated another 20 min at 60° C. The absorbance is measured at 520 mµ, and the specific activity expressed as under Example 68 B.

EXAMPLE 69 Assay for Chemically Inducible DNA Sequences: Bacillus Thuringiensis Endotoxin

A. plant extraction procedure

About 100 mg plant tissue is homogenized in 0.1 ml extraction buffer (50 mM $Na_2CO_3$ pH 9.5, 10 mM EDTA, 0.05 % Triton X-100, 0.05% Tween, 100 mM NaCl, 1 mM phenylmethylsulfonyl fluoride (added just prior to use), and 1 mM leupeptine (added just prior to use)). After extraction, 2 M Tris pH 7.0 is added to adjust the pH to 8.0-8.5. The extract is then centrifuged 10 minutes in a Beckman microfuge and the supernatant used for ELISA analysis.

B. ELISA

An ELISA plate is pretreated with ethanol. Affinity-purified rabbit anti-Bt antiserum (50 µl) at a concentration of 3 µg/ml in borate-buffered saline (100 mM boric acid, 25 mM sodium borate, 75 mM NaCl, pH 8.4-8.5) is added to the plate and this allowed to incubate overnight at 4° C. Antiserum is produced by immunizing rabbits with gradient-purified Bt (Bacillus thuringiensis endotoxin) crystals [Ang, B.J. & Nickerson, K.W., Appl. Environ. Microbiol. 36, 625-626 (1978)] solubilized with sodium dodecyl sulfate. The plate is washed with wash buffer (10 mM Tris-HCl pH 8.0, 0.05% Tween 20, 0.02% sodium azide), then treated 1 hour at room temperature with blocking buffer (10 mM sodium phosphate buffer pH 7.4, 140 mM NaCl, 1% bovine serum albumin, 0.02% sodium azide), and washed again. The plant extract is added in an amount to give 50 µg of protein (typically ca. 5 µl of extract; the protein is determined by the method of Bradford, M., Anal. Biochem. 72, 248 (1976) using a commercially available kit from Bio-Rad), and incubated overnight at 4° C. After washing, 50 µl affinity-purified goat anti-Bt antiserum are added at a concentration of 3 µg/ml protein in ELISA diluent (10 mM sodium phosphate buffer pH 7.4, 140 mM NaCl, 0.05% Tween 20, 1% bovine serum albumin, 0.02% sodium azide), and this allowed to incubate for 1 hour at 37° C. After washing, 50 µl rabbit anti-goat antibody bound to alkaline phosphatase (Sigma Chemicals, St. Louis, Mo., USA) diluted 1:500 in ELISA diluent is added and allowed to incubate for 1 hour at 37° C. After washing, 50 µl substrate (0.6 mg/ml p-nitrophenyl phosphate, 0.05 mg/ml $MgCl_2$, 10% diethanolamine, pH 9.8 adjusted with HCl) is added and allowed to incubate for 30 minutes at room temperature. The reaction is terminated by adding 50 µl 3 M NaOH. The absorbance is read at 405 nm in a modified ELISA reader (Hewlett Packard, Stanford, Ca., USA).

## Claims

1. A non-coding DNA sequence capable of regulating the transcription of an associated DNA sequence in a plant or plant tissue wherein this regulation is dependent upon a chemical regulator.

2. A substantially pure non-coding DNA sequence according to claim 1.

3. A DNA sequence according to claim 2 wherein said associated DNA sequence is a coding sequence.

4. A DNA sequence according to claim 2 wherein said regulation is dependent upon an inducing chemical regulator.

5. A substantially pure DNA sequence according to claim 1 which is, or has substantial sequence homology to, a non-coding chemically regulatable DNA sequence which is part of a naturally occurring chemically regulatable gene.

6. A DNA sequence according to claim 5 capable of regulating the transcription of an associated DNA sequence in a plant or plant tissue wherein said associated DNA sequence is a coding sequence.

7. A DNA sequence according to claim 6 wherein said transcription is regulated by an inducing chemical regulator.

8. A DNA sequence according to claim 5 wherein said chemically regulatable gene is a PR protein gene.

9. A DNA sequence according to claim 8 wherein said PR protein gene is a tobacco PR-1a, PR-1b, PR-1c, PR-1′, PR-Q or PR-R gene, a cucumber chitinase gene, or a tobacco basic or acidic β-1,3-glucanase gene.

10. A DNA sequence according to claim 8 wherein said PR protein gene is a tobacco PR-1a, PR-1b, PR-1c, PR-1′ or PR-R gene or a cucumber chitinase gene.

11. A DNA sequence according to claim 8 wherein said PR protein gene is a tobacco PR-1a gene.

12. A DNA sequence according to claim 8 wherein said PR protein gene is a tobacco PR-1′ gene.

13. A DNA sequence according to claim 8 wherein said PR protein gene is a basic tobacco β-1,3-glucanase gene.

14. A DNA sequence according to claim 8 which is located in the 5′-flanking region of said PR protein gene.

15. A DNA sequence according to claim 6 wherein said transcription is regulated by a regulator selected from the group consisting of benzoic acid, salicylic acid, acetylsalicylic acid, polyacrylic acid and substituted derivatives thereof.

16. A DNA sequence according to claim 6 wherein said transcription is regulated by a regulator selected from the group consisting of benzo-1,2,3-thiadiazolecarboxylic acid, benzo-1,2,3-thiadiazolethiocarboxylic acid, cyanobenzo-1,2,3-thiadiazole, benzo-1,2,3-thiadiazolecarboxylic acid amide, benzo-1,2,3-thiadiazolecarboxylic acid hydrazide, and derivatives thereof.

17. A DNA sequence according to claim 16 wherein said transcription is regulated by methyl benzo-1,2,3-thiadiazole-7-carboxylate.

18. A DNA sequence according to claim 6 wherein said transcription is regulated by dichloroisonicotinic acid or a derivative thereof.

19. A DNA sequence which comprises a first DNA component sequence which is, or has substantial sequence homology to, a non-coding, chemically regulatable DNA sequence of a naturally occurring chemically regulatable gene, this first component sequence being capable of regulating the transcription of an associated DNA sequence in a plant or plant tissue, wherein this regulation is dependent upon a chemical regulator, and a second DNA component sequence which is, or has substantial sequence homology to, part but not all of a transcribable DNA sequence with which the first component is associated in the naturally occurring chemically regulatable gene.

20. A substantially pure DNA sequence according to claim 19.

21. A DNA sequence according to claim 20 wherein said naturally occurring chemically regulatable gene occurs in a plant and said second DNA component sequence is a coding sequence.

22. A DNA sequence according to claim 21 wherein said gene is a PR protein gene.

23. A DNA sequence according to claim 21 wherein the coding sequence codes for a signal peptide.

24. A chimeric DNA sequence comprising a first non-coding chemically regulatable DNA component sequence which is capable of regulating the transcription of an associated DNA sequence in a plant or plant tissue, wherein this regulation is dependent upon a chemical regulator, and a second DNA component sequence capable of being transcribed in a plant or plant tissue.

25. A chimeric DNA sequence according to claim 24 wherein said second DNA component sequence is capable of regulating the expression of a phenotypic trait by an anti-sense mechanism.

26. A chimeric DNA sequence according to claim 24 wherein said second DNA component sequence is a coding sequence that is capable of being transcribed and translated to produce a polypeptide resulting in a phenotypic trait.

27. A chimeric DNA sequence according to claim 24 wherein said second DNA component sequence is adjacent to the first component sequence.

28. A chimeric DNA sequence according to claim 24 wherein said first DNA component sequence is regulated by a repressing chemical regulator.

29. A chimeric DNA sequence according to claim 24 wherein said first DNA component sequence is regulated by an inducing chemical regulator.

30. A chimeric DNA sequence according to claim 24 wherein said first DNA component sequence is, or has substantial sequence homology to, a chemically regulatable DNA sequence in a naturally occurring chemically regulatable gene from a plant.

31. A chimeric DNA sequence according to claim 24 wherein said first DNA component sequence is, or has substantial sequence homology to, a chemically regulatable DNA sequence in a naturally occurring chemically regulatable gene from a plant and said second DNA component sequence is a coding sequence that is capable of being transcribed and translated to produce a polypeptide resulting in a phenotypic trait.

32. A chimeric DNA sequence according to claim 31 wherein said second DNA component sequence is adjacent to said first DNA coding sequence.

33. A chimeric DNA sequence according to claim 31 wherein said first DNA component sequence is regulated by an inducing chemical regulator.

34. A chimeric DNA sequence according to claim 31 wherein said first DNA component sequence is, or has substantial sequence homology to, the chemically inducible DNA sequence in a PR protein gene.

35. A chimeric DNA sequence according to claim 34 wherein said PR protein gene is a tobacco PR-1a, PR-1b, PR-1c, PR-1′, PR-Q or PR-R gene, a cucumber chitinase gene or a tobacco basic or acidic β-1,3-glucanase gene.

36. A chimeric DNA sequence according to claim 34 wherein said PR protein gene is a tobacco PR-1a, PR-1b, PR-1c, PR-1′ or PR-R gene or a cucumber chitinase gene.

37. A chimeric DNA sequence according to claim 34 wherein said PR protein gene is a tobacco PR-1a or PR-1′ gene.

38. A chimeric DNA sequence according to claim 34 wherein said PR protein gene is a basic tobacco β-1,3-glucanase gene.

39. A chimeric DNA sequence according to claim 31 wherein said first DNA component sequence is the 5′ flanking region of the tobacco PR-1a gene and contains about 300 or more base pairs adjacent to the transcriptional start site.

40. A chimeric DNA sequence according to claim 39 wherein said first DNA component sequence contains between about 600 and 1000 base pairs adjacent to the transcriptional start site.

41. A chimeric DNA sequence according to claim 31 wherein the second DNA component sequence codes for a phenotypic trait.

42. A chimeric DNA sequence according to claim 41 wherein said phenotypic trait is selected from the group consisting of tolerance or resistance to a herbicide, fungus, virus, bacterium, insect, nematode or arachnid; production of secondary metabolites; male or female sterility; and production of an enzyme or other reporter compound.

43. A chimeric DNA sequence according to claim 41 wherein said phenotypic trait is tolerance or resistance to herbicides or resistance to insects.

44. A chimeric DNA sequence according to claim 43 wherein said tolerance or resistance to herbicides is caused by a DNA sequence coding for wild-type or herbicide resistant acetohydroxyacid synthase (AHAS).

45. A chimeric DNA sequence according to claim 43 wherein said resistance to insects is caused by a DNA sequence coding for Bacillus thuringiensis endotoxin (BT).

46. A chimeric DNA sequence according to claim 41 wherein said phenotypic trait is a reporter compound selected from the group consisting of luciferase (LUX), chloramphenicol acetyltransferase (CAT), neomycin phosphotransferase (NPT), nopaline synthase (NOS), octopine synthase (OCS), beta-l,3-glucuronidase (GUS), acetohydroxyacid synthase (AHAS), and Bacillus thuringiensis endotoxin (BT).

47. A chimeric DNA sequence according to claim 41 wherein said phenotypic trait is a reporter compound selected from the group consisting of beta-l,3-glucuronidase (GUS), acetohydroxyacid synthase (AHAS), and Bacillus thuringiensis endotoxin (BT).

48. A chimeric DNA sequence according to claim 24 wherein said first DNA component sequence is regulated by a chemical regulator inducing PR protein genes in plants.

49. A chimeric DNA sequence according to claim 48 wherein the chemical regulator is selected from the group consisting of benzoic acid, salicylic acid, acetylsalicylic acid, polyacrylic acid and substituted derivatives thereof.

50. A chimeric DNA sequence according to claim 48 wherein the chemical regulator is selected from the group consisting of benzo-1,2,3-thiadiazolecarboxylic acid, benzo-1,2,3-thiadiazolethiocarboxylic acid, cyanobenzo-1,2,3-thiadiazole, benzo-1,2,3-thiadiazolecarboxylic acid amide, benzo-1,2,3-thiadiazolecarboxylic acid hydrazide, and derivatives thereof.

51. A chimeric DNA sequence according to claim 48 wherein the chemical regulator is selected from the group consisting of benzo-1,2,3-thiadiazole-7-carboxylic acid, benzo-1,2,3-thiadiazole-7-thiocarboxylic acid, 7-cyanobenzo-1,2,3-thiadiazole, benzo-1,2,3-thiadiazole-7-carboxylic acid amide, benzo-1,2,3-thiadiazole-7-carboxylic acid hydrazide, and derivatives thereof.

52. A chimeric DNA sequence according to claim 48 wherein the chemical regulator is selected from the group consisting of benzo-1,2,3-thiadiazolecarboxylic acid, alkyl benzo-1,2,3-thiadiazolecarboxylate in which the alkyl group contains one to six carbon atoms, and substituted derivatives of these compounds.

53. A chimeric DNA sequence according to claim 48 wherein the chemical regulator is dichloroisonicotinic acid or a derivative thereof.

54. A chimeric DNA sequence according to claim 48 wherein the chemical regulator is selected from the group consisting of benzo-1,2,3-thiadiazole-7-carboxylic acid, methyl benzo-1,2,3-thiadiazole-7-carboxylate, n-propyl benzo-1,2,3-thiadiazole-7-carboxylate, benzyl benzo-1,2,3-thiadiazole-7-carboxylate, benzo-1,2,3-thiadiazole-7-carboxylic acid sec-butylhydrazide, 2,6-dichloroisonicotinic acid, and methyl 2,6-dichloroisonicotinate.

55. A chimeric DNA sequence according to claim 48 wherein the chemical regulator is methyl benzo-1,2,3-thiadiazole-7-carboxylate.

56. A chimeric DNA sequence according to claim 24 comprising a first DNA component sequence which is, or has substantial sequence homology to, a non-coding, chemically regulatable DNA sequence of a naturally occurring chemically regulatable gene, this first DNA component sequence being capable of regulating the transcription of an associated DNA sequence in a plant or plant tissue, wherein this regulation is dependent upon a chemical regulator, a second DNA component sequence which is, or has substantial sequence homology to, part but not all of a transcribable DNA sequence with which the first component is associated in the naturally occurring chemically regulatable gene, and a third DNA component sequence capable of being transcribed in a plant or plant tissue.

57. A chimeric DNA sequence according to claim 56 wherein the first DNA component sequence is, or has substantial sequence homology to, the chemically inducible DNA sequence in a PR protein gene.

58. A chimeric DNA sequence according to claim 56 wherein the second DNA component sequence codes for a signal peptide.

59. A chimeric DNA sequence according to claim 58 wherein the second DNA component sequence codes for a peptide which is, or has substantial sequence homology to, the signal peptide from a PR protein gene.

60. A chimeric DNA sequence according to claim 59 wherein the PR protein gene is the PR-1a gene.

61. A chimeric DNA sequence according to claim 56 wherein the third DNA component sequence codes for a phenotypic trait.

62. A vector comprising the non-coding DNA sequence of claim 1.

63. A vector according to claim 62 comprising the chimeric DNA sequence of claim 24.

64. A vector according to claim 62 which is functional in plant cells or in Agrobacterium.

65. E. coli HB101/pTJS75-Km, ATCC number 67628, deposited on February 12, 1988, according to claim 62.

66. Plasmid pBS-PR1013Cla, ATCC number 40426, deposited on February 12, 1988, according to claim 62.

67. Plasmid pBS-pR1019, ATCC number 40427, deposited on February 12, 1988, according to claim 62.

68. Plasmid pBS-Gluc39.1, ATCC number 40526, deposited on December 29, 1988, according to claim 62.

69. Plasmid pBS-Gluc39.3, ATCC number 40527, deposited on December 29, 1988, according to claim 62.

70. Plasmid pBScucchi/chitinase, ATCC number 40528, deposited on December 29, 1988, according to claim 62.

71. Plasmid pBSGL6e, ATCC number 40535 , deposited on January 18, 1989, according to claim 62.

72. Plant tissue, plant or seed containing the chimeric DNA sequence of claim 24.

73. A substantially pure signal peptide having the amino acid sequence
MetGlyPheValLeuPheSerGlnLeuProSerPheLeuLeuValSerThrLeuLeuPheLeuValIleSerHisSerCysArgAla
or a peptide having substantial sequence homology to this peptide.

74. A DNA sequence which codes for the signal peptide of claim 73 or for a peptide having substantial sequence homology to that signal peptide.

75. A substantially pure DNA sequence according to claim 19 as shown in "Sequence 1" or having substantial sequence homology to the sequence shown in "Sequence 1".

76. A substantially pure DNA sequence according to claim 19 as shown in "Sequence 2" or having substantial sequence homology to the sequence shown in "Sequence 2".

77. A substantially pure DNA sequence as shown in "Sequence 3" or having substantial sequence homology to the sequence shown in "Sequence 3".

78. A substantially pure DNA sequence as shown in "Sequence 4" or having substantial sequence homology to the sequence shown in "Sequence 4".

79. A substantially pure DNA sequence according to claim 19 as shown in "Sequence 5" or having substantial sequence homology to the sequence shown in "Sequence 5".

80. A substantially pure DNA sequence according to claim 19 as shown in "Sequence 6" or having substantial sequence homology to the sequence shown in "Sequence 6".

81. A substantially pure DNA sequence as shown in "Sequence 7" or having substantial sequence homology to the sequence shown in "Sequence 7".

82. A substantially pure DNA sequence as shown in "Sequence 8" or having substantial sequence homology to the sequence shown in "Sequence 8".

83. A process for the preparation of a substantially pure chemically regulatable DNA sequence from a chemically regulatable gene in a naturally occurring system containing that gene, which process comprises the steps of

(a) activating expression in said system of RNA from the chemically regulatable gene;

(b) isolating said RNA;

(c) differentially screening a genomic library for RNA generated from RNA isolated from said activated system as well as RNA generated from RNA isolated from a control system that is not activated;

(d) isolating a genomic clone;

(e) subcloning the chemically regulatable gene from said genomic clone; and

(f) isolating the desired chemically regulatable DNA sequence.

84. A substantially pure DNA sequence isolated by the process of claim 83.

85. A process according to claim 83 wherein said system is a plant, which process comprises the steps of

(a) activating expression in said plant of polyA+ RNA from the chemically regulatable gene;

(b) isolating said polyA+ RNA;

(c) constructing a cDNA library from said polyA+ RNA;

(d) differentially screening said cDNA library with cDNA generated from RNA in a control plant that is not activated;

(e) isolating cDNA clones that are chemically regulatable;

(f) isolating a genomic clone from a genomic library of said plant using as a probe the cDNA clone of step (e);

(g) subcloning the chemically regulatable gene from said genomic clone; and

(h) isolating the desired chemically regulatable DNA sequence.

86. A process according to claim 85 wherein said chemically regulatable gene is a PR protein gene.

87. A process according to claim 86 wherein said PR protein gene is activated in step (a) by a chemical inducer or a pathogen.

88. A process according to claim 87 wherein said chemical inducer is selected from the group consisting of benzoic acid, salicylic acid, acetylsalicylic acid, polyacrylic acid, benzo-1,2,3-thiadiazolecarboxylic acid, benzo-1,2,3-thiadiazolethiocarboxylic acid, cyanobenzo-1,2,3-thiadiazole, benzo-1,2,3-thiadiazolecarboxylic acid amide, benzo-1,2,3-thiadiazolecarboxylic acid hydrazide, dichloroisonicotinic acid, and derivatives thereof.

89. A process for regulating transcription of a chemically regulatable gene, which process comprises applying a chemical regulator to plant tissue, plant or seed containing the chimeric DNA sequence of claim 24.

90. A process according to claim 89 wherein said regulator is selected from the group consisting of benzoic acid, salicylic acid, acetylsalicylic acid, polyacrylic acid, benzo-1,2,3-thiadiazolecarboxylic acid, benzo-1,2,3-thiadiazolethiocarboxylic acid, cyanobenzo-1,2,3-thiadiazole, benzo-1,2,3-thiadiazolecarboxylic acid amide, benzo-1,2,3-thiadiazolecarboxylic acid hydrazide, dichloroisonicotinic acid, and derivatives thereof.

91. A process according to claim 89 wherein the first DNA component sequence of said chimeric DNA is a chemically inducible sequence which is, or has substantial sequence homology to, a chemically inducible sequence of a PR gene.

92. A process according to claim 89 wherein the chemically regulatable gene codes for a phenotypic trait selected from the group consisting of a developmental state, sterility, disease resistance, pest resistance, herbicide tolerance or resistance, production of a secondary metabolite and production of an assayable enzyme marker.

93. A process according to claim 92 wherein said enzyme marker is luciferase (LUX), chloramphenicol acetyltransferase (CAT), neomycin phosphotransferase (NPT), nopaline synthase (NOS), octopine synthase (OCS), beta-l,3-glucuronidase (GUS), acetohydroxyacid synthase (AHAS), or Bacillus thuringiensis endotoxin (BT).

94. A process according to claim 93 wherein said enzyme marker is beta-l,3-glucuronidase (GUS), acetohydroxyacid synthase (AHAS), or Bacillus thuringiensis endotoxin (BT).

95. A process for identifying a chemical regulator which comprises transforming a host with the chimeric DNA sequence of claim 31 or a vector containing said chimeric DNA sequence, applying a putative chemical regulator to the transformed host, and measuring the expression of the phenotypic trait.

96. A process according to claim 95 wherein the transformed host is plant tissue or plant cells.

97. A process according to claim 96 wherein the first DNA component sequence of said chimeric DNA is a chemically inducible sequence which is, or has substantial sequence homology to, a chemically inducible sequence of a PR protein gene.

98. A process according to claim 95 wherein the phenotypic trait coded for by the chimeric DNA is an assayable enzyme marker.

99. A process according to claim 98 wherein said enzyme marker is luciferase (LUX), chloramphenicol acetyltransferase (CAT), neomycin phosphotransferase (NPT), nopaline synthase (NOS), octopine synthase (OCS), beta-l,3-glucuronidase (GUS), acetohydroxyacid synthase (AHAS), or Bacillus thuringiensis endotoxin (BT).

100. A process for identifying a chemically regulatable DNA sequence which comprises the steps of

(a) transforming a host with a putative chemically regulatable DNA sequence or a vector containing said sequence, a second DNA sequence which is a host-selectable gene marker and a third DNA sequence which codes for a phenotypic trait;

(b) applying a chemical regulator to said transformed host; and

(c) measuring the expression or selecting for change in expression of the phenotypic trait coded for by the third DNA sequence.

101. A process according to claim 100 wherein said third DNA sequence is a host-selectable or an assayable gene marker.

102. A process according to claim 100 wherein the transformed host is plant tissue or plant cells.

103. A process according to claim 100 wherein the second or third DNA sequence is a gene marker for herbicide resistance or antibiotic resistance.

104. A process according to claim 100 wherein said gene marker is an antibiotic resistance gene selected from the group consisting of hygromycin, kanamycin, chloramphenicol, bleomycin, puromycin, lincomycin and methotrexate resistance genes.

105. A process according to claim 100 wherein the gene marker is an aminoglycoside phosphotransferase IV hygromycin resistance gene.

106. A process according to claim 100 wherein the third DNA sequence codes for an assayable enzyme marker selected from the group consisting of luciferase (LUX), chloramphenicol acetyltransferase (CAT), neomycin phosphotransferase (NPT), nopaline synthase (NOS), octopine synthase (OCS), beta-1,3-glucuronidase (GUS), acetohydroxyacid synthase (AHAS), and Bacillus thuringiensis endotoxin (BT).

107. A process according to claim 100 wherein the third DNA sequence codes for an assayable enzyme marker selected from the group consisting of beta-1,3-glucuronidase (GUS), acetohydroxyacid synthase (AHAS), and Bacillus thuringiensis endotoxin (BT).

108. A substantially pure chemically regulatable DNA sequence identified by the process of claim 100.

109. A method for selecting transformed plant cells or tissue which have been subjected to transforming DNA comprising a first DNA component sequence that is chemically regulatable by a known chemical regulator and an associated second DNA sequence coding for a phenotypic marker, which process comprises the steps of (a) treating putative transformants with said known chemical regulator and (b) selecting transformants according to expression of the phenotypic trait coded for by the second DNA sequence.

110. A method according to claim 109 wherein said phenotypic trait is antibiotic resistance.

111. A method according to claim 110 wherein said antibiotic resistance is resistance to hygromycin, kanomycin, chloroamphenicol, bleomycin, puromycin, lincoymcin, G418 and methotrexate.

112. A method according to claim 109 wherein said phenotypic trait is an assayable enzyme marker.

113. A method according to claim 112 wherein said enzyme marker is selected from the group consisting of luciferase (LUX), chloramphenicol acetyltransferase (CAT), neomycin phosphotransferase (NPT), nopaline synthase (NOS), octopine synthase (OCS), beta-1,3-glucuronidase (GUS), acetohydroxyacid synthase (AHAS), and Bacillus thuringiensis endotoxin (BT).

114. A method according to claim 113 wherein said enzyme marker is selected from the group consisting of beta-l,3-glucuronidase (GUS), acetohydroxyacid synthase (AHAS), and Bacillus thuringiensis endotoxin (BT).

115. An assay for beta-1,3-glucuronidase (GUS) activity which comprises reacting plant tissue extracts in samples of less than 0.5 ml containing 1.5 to 3 mM 4-methyl umbelliferyl glucuronide at around 37° C for 1 to 5 hours and determining the concentration of the fluorescent indicator released.

116. A method for the determination of the amount of a specific RNA in a solution of RNA comprising

(a) labeling a RNA specific primer oligonucleotide of a length of between 12 and 18 nucleotides to high specific radioactivity,

(b) hybridizing the RNA with the labeled oligonucleotide at a concentration of between 0.1 and 20 nM for between 2 minutes and 24 hours at a temperature around 37° C,

(c) elongating the primer oligonucleotide with reverse transcriptase in the presence of nucleotide triphosphates at a concentration of between 0.003 and 1 mM for between 1 and 120 minutes, and

(d) separating and visualizing the elongation products with autoradiography.

117. A process for the preparation of a chemically regulatable chimeric DNA sequence comprising a first non-coding, chemically regulatable DNA component sequence which is capable of regulating the transcription of an associated DNA sequence in a plant or plant tissue, wherein this regulation is dependent upon a chemical regulator, and a second DNA component sequence capable of being transcribed in a plant or plant tissue, characterized in that the DNA component sequences are ligated.

118. A process for the prepartion of a chimeric DNA sequence comprising a first DNA component sequence which is, or has substantial sequence homology to, a non-coding, chemically regulatable DNA sequence of a naturally occurring chemically regulatable gene, this first DNA component sequence being capable of regulating the transcription of an associated DNA sequence in a plant or plant tissue, wherein this regulation is dependent upon a chemical regulator, a second DNA component sequence which is, or has substantial sequence homology to, part but not all of a transcribable DNA sequence with which the first component is associated in the naturally occurring chemically regulatable gene, and a third DNA component sequence capable of being transcribed in a plant or plant tissue, characterized in that the DNA component sequences are ligated concurrently or consecutively.

```
10 30 50 70 90 110
CTCGAGGATTTCAAACTCTAGCTTCACTAAAACTTGAGCTTTCTTTTCCACTAATGTCGAAAAACGAAATAAACATAAGCTATTTACAAAAAATAAAAAAATACTCCATTTGAATCTAAA
130 150 170 190 210 230
GTCAAGTCGTGATTGGGATAAGAAAATAGAAATTTATTTATACTCCAGATCAAGCCGTGATTGGAATGAGATAATAGAAAAGTATGATAGTACATGAGTAACATCAAGTTGGAAATTAAG
250 270 290 310 330 350
GGAAGGAAATTAGAGAAAGAACTGAAGAATATCCAAATATTCTTTACGTCCAAATTTGATAGTTATTTAACGTCATCGAGATGACGGCCATGTTCAAGTTTTCCACAAATATTGAGAAAA
370 390 410 430 450 470
GAAAGAAGAAGACACAAACTGTGTTTGGTATTATTATAGTTTTTTCTTTTAGAGAATTGATTGTACATATAAGAAATATAATATAAGATTTAGAAATAAGATTATTAGAAAAATCAAACA
490 510 530 550 570 590
TCAAAGTATTTATTTTAAATTCTTTTTCCAATGGACATTCCCATTCTGAAAAAAAAGAGATATAAATATGGAAGTAAAAATTAATCAGATCGTTAAATGTAGAAAATATTAATTAACACA
610 630 650 670 690 710
TTAACCATAACCAGTCTACTTTATTTAACAAAAAGCACATCTGATAGATCAAAAAAGTGTTTAACTTCATGCATTGACAATTTAAAATTATTTTGCAACATCGGGTAAAACTATTTTACA
730 750 770 790 810 830
ACAATTGGTAACTGCATATATAAGTTTAATATGGTAACCTAGAAAATAGGATAAATTATCTATAACAGGATATATTACATTGATATTACCATGTCAAAAAATTTAGTAAGTACATGAATA
850 870 890 910 930 950
ATCACCGTGAAATCTTCAAGATTTCTCCTATAAATACCCTTGGTAGTAAATCTAGTTTTTCCATTCAAGATACAACATTTCTCCTATAGTCATGGGATTTGTTCTCTTTTCACAATTGCC
                                                                                           MetGlyPheValLeuPheSerGlnLeuPr
970 990 1010 1030 1050 1070
TTCATTTCTTCTTGTCTCTACACTTCTCTTATTCCTAGTAATATCCCACTCTTGCCGTGCCCAAAATTCTCAACAAGACTATTTGGATGCCCATAACACAGCTCGTGCAGATGTAGGTGT
oSerPheLeuLeuValSerThrLeuLeuLeuPheLeuValIleSerHisSerCysArgAlaGlnAsnSerGlnGlnAspTyrLeuAspAlaHisAsnThrAlaArgAlaAspValGlyVa
1090 1110 1130 1150 1170 1190
AGAACCTTTGACCTGGGACGACCAGGTAGCAGCCTATGCGCAAAATTATGCTTCCCAATTGGCTGCAGATTGTAACCTCGTACATTCTCATGGTCAATACGGCGAAAACCTAGCTGAGGG
lGluProLeuThrTrpAspAspGlnValAlaAlaTyrAlaGlnAsnTyrAlaSerGlnLeuAlaAlaAspCysAsnLeuValHisSerHisGlyGlnTyrGlyGluAsnLeuAlaGluGl
1210 1230 1250 1270 1290 1310
AAGTGGCGATTTCATGACGGCTGCTAAGGCTGTTGAGATGTGGGTCGATGAGAAACAGTATTATGACCATGACTCAAATACTTGTGCACAAGGACAGGTGTGTGGACACTATACTCAGGT
ySerGlyAspPheMetThrAlaAlaLysAlaValGluMetTrpValAspGluLysGlnTyrTyrAspHisAspSerAsnThrCysAlaGlnGlyGlnValCysGlyHisTyrThrGlnVa
1330 1350 1370 1390 1410 1430
GGTTTGGCGTAACTCGGTTCGTGTTGGATGTGCTAGGGTTCAGTGTAACAATGGAGGATATGTTGTCTCTTGCAACTATGATCCTCCAGGTAATTATAGAGGCGAAAGTCCATACTAATT
lValTrpArgAsnSerValArgValGlyCysAlaArgValGlnCysAsnAsnGlyGlyTyrValValSerCysAsnTyrAspProProGlyAsnTyrArgGlyGluSerProTyrEnd
1450 1470 1490 1510 1530 1550
GAAACGACCTACGTCCATTTCACGTTAATATGTATGGATTGTTCTGCTTGATATCAAGAACTTAAATAATTGCTCTAAAAAGCAACTTAAAGTCAAGTATATAGTAATAGTACTATATTT
1570 1590 1610 1630 1650 1670 207↓
GTAATCCTCTGAAGTGGATCTATAAAAAGACCAAGTGGTCATAATTAAGGGGAAAAATATGAGTTGATGATCAGCTTGATGTATGATCTGATATTATTATGAACACTTTTGTACTCATAC
1690 1710 313↓ 1730 1750 1770 1790
GAATCATGTGTTGATGGTCTAGCTACTTGCGATATTACGAGCAAAATTCTTAACTACATGCCTTAGGAACAAGCTTACACAGTTCATATAATCTACTAGAGGGCCAAAAACATGAAAATT
1810 1830 1850 1870 1890 1910
ACCAATTTAGATGGTAGGAGGATATTGAAAGTGGAGCAGCTAGTTTTAATAACTGACCGTTAGTCTTAAAATTGACGGTATAAAAATATTTACATAATCAGGTCATTTATAAGGTAATTA
1930 1950 1970 1990 2010 2030
TAGGTAAATATTTATGACGAATTCTCAATAGTAATCTGAAAAAAAATTGTAACTAACCTATTATACTAAAACTACTATAATAGGTTAGATTACATTAATCATGTCATTAGAAGATCTT
```

SEQUENCE 1

```
     10         30         50         70         90        110
TTACTGTTAAAGTATTCGTACTCGTAGATATTTCAAAAATTAGATAGCGATTTTATCATCTAATCAACTATAGATCTAAGTCGAAAATATACCTTGAGTATAGATTTTTA

          130        150        170        190        210
CTTACCAGCCTTTCATCTTCTTCCAGAAATAAGAGTGGAAATCAACGGTAGCAGATAACGTTGAGATATGATCTTGTAGTAATTGACACCTAACGGGACTGCTTTCCTTT

     230        250        270        290        310        330
AAAATATAGACGATAATATCAGTTATAAGGATGCTTTCACTTTCTAATTAAAGCATATTCTGATTCGGTTTTATAGAATTTGATAGAAGTTAGACAGCATCCCTCCAATT

          350        370        390        410        430
GAACAGGTTCGATTACTCAACTTCCCGTCTACTTAAACAAATTCAACTCTTTTCGCGTACTATATTTATGTTAAAATACACTGGTAATAATGTATAAAACTATATGTATG

     450        470        490        510        530        550
TATATAATATAACTTCATGATTTATCAAAGTTGGTCAAATTATGATCATGGCTATAATAGTTGTTCATCAATAGATAGAATTTTTTAATGGCGGCTTTTGTTTCTTTAAC

          570        590        610        630        650
GAATATGATCTAACGTTCTTTGAATTAACGGAGCTATAATTTTATACTATTCTTTTCAAATATGGCTAAAGAGTTTGCTAACAAGTTGCAAACTTTGTAACTCAGCTATA

     670        690        710        730        750        770
TTCTTCCCTATAAAAATCATCCTTACTATGCTTTGTTTCTCACCAAAACACAAAAATGGGATTCTTAACAACAATAGTTGCTTGTTTCATTACCTTTGCAATATTAATTC
                                                         MetGlyPheLeuThrThrIleValAlaCysPheIleThrPheAlaIleLeuIleH

          790        810        830        850        870
ACTCATCCAAAGCTCAAAACTCCCCCCAAGATTATCTTAACCCTCACAATGCAGCTCGTAGACAAGTTGGTGTTGGCCCCATGACATGGGACAATAGGCTAGCAGCCTAT
isSerSerLysAlaGlnAsnSerProGlnAspTyrLeuAsnProHisAsnAlaAlaArgArgGlnValGlyValGlyProMetThrTrpAspAsnArgLeuAlaAlaTyr

     890        910        930        950        970        990
GCCCAAAATTATGCCAATCAAAGAATTGGTGACTGCGGGATGATCCACTCTCATGGCCCTTACGGCGAAAACCTAGCCGCCGCCTTCCCTCAACTTAACGCTGCTGGTGC
AlaGlnAsnTyrAlaAsnGlnArgIleGlyAspCysGlyMetIleHisSerHisGlyProTyrGlyGluAsnLeuAlaAlaAlaPheProGlnLeuAsnAlaAlaGlyAl

          1010       1030       1050       1070       1090
TGTAAAAATGTGGGTCGATGAGAAGCGTTTCTATGATTACAATTCAAATTCTTGTGTAGGAGGAGTATGTGGACACTATACTCAGGTGGTGTGGCGTAACTCAGTACGTC
aValLysMetTrpValAspGluLysArgPheTyrAspTyrAsnSerAsnSerCysValGlyGlyValCysGlyHisTyrThrGlnValValTrpArgAsnSerValArgL

     1110       1130       1150       1170       1190       1210
TCGGTTGTGCTAGGGTTCGAAGCAACAATGGTTGGTTTTTCATAACTTGCAATTATGATCCACCAGGTAATTTTATAGGACAACGTCCCTTTGGCGATCTTGAGGAGCAA
euGlyCysAlaArgValArgSerAsnAsnGlyTrpPhePheIleThrCysAsnTyrAspProProGlyAsnPheIleGlyGlnArgProPheGlyAspLeuGluGluGln

          1230       1250       1270       1290       1310
CCCTTTGATTCCAAATTGGAACTTCCAACTGATGTCTAATGAGTGCACGTACATGAACAAATTATCATGAATAAAGGAAAATAAAATGCAGTGCTATGCTATGTGATTTT
ProPheAspSerLysLeuGluLeuProThrAspValEnd
```

SEQUENCE 2 (1)

1330 1350 1370 1390 1410 1430

AGCTTCCCAGTTGGATAATAATCTGATGGTGTAGTAACTGGCCGAGTGTTTGGACCCTACCTTGCATGTTGGTAGATGGAATCAGTTTATATATGAGTTTTCTGTTGGCT

1450 1470 1490 1510 1530

TATGTTATTTTTATTAAAATCTTATTCTAGTTTTACAGTTTTTTATTCGCACTATATGTGTATGGGTCCAAATTTGATCGACCACGACCTATGACGAGTACAACAAACGA

1550 1570 1590 1610 1630 1650

CCGGGTACCTTCGAATCGGCGTCCCAAGGGAAACGACCCTAGGGCGAAAGAAGAGACTGTATGACTCTTGTAGTAGTGTAAGCCCATTAGGGAACATTAAGAATATTCCG

1670 1690 1710 1730 1750

CCGAATACTAATTGTATTTTGTTTCTTACAATTCGGAAGGGTTTCTCTCTAGTATATAAAGGGGACAAAAAACCTTGTAAGTGGCGGTGGATACTCTGAGAAGCTTACTA

1770 1790 1810 1830 1850 1870

ATCTTGGATGAAAAGAAAAACTCTCTTCTCTTTATCTATTATTATTCTAGAGATTATTATCTTCAGCTACGATTTACCCCTTCATCTTTGATTGATTTGTTCAAAAAGGT

1890 1910 1930 1950 1970

TTTAACATCTTTTGAGTCAAACAATTTGGCNCCGTCTATGGGGATTTCTATAGCTGAAATCATAGTTCTCATCTAGATTTCTGAAGTGATAATTACTTTTCTTCAAACCT

1990 2010 2030 2050 2070 2090

CATAAAAATCAACAATGGCSGGAAAGGAAGTGAAGCTAAAGGCGGTASAGATCGTCTCGAATAACTCCTGAACTCCCTCAACGAAGACGGCAGAGAAGACAATGAGAATA

SEQUENCE 2 (2)

10 30 50 70 90

AAAGAAAGCTCTTTAAGCAATGGCTGCCCACAAAATAACTACAACCCTTTCCATCTTCTTCCTCCTTTCCTCTATTTTCCGCTCTTCCGACGCGGCTGGA

M A A H K I T T T L S I F F L L S S I F R S S D A A G

110 130 150 170 190

ATCGCCATCTATTGGGGTCAAAACGGCAACGAGGGCTCTCTTGCATCCACCTGCGCAACTGGAAACTACGAGTTCGTCAACATAGCATTTCTCTCATCCT

I A I Y W G Q N G N E G S L A S T C A T G N Y E F V N I A F L S S F

210 230 250 270 290

TTGGCAGCGGTCAAGCTCCAGTTCTCAACCTTGCTGGTCACTGCAACCCTGACAACAACGGTTGCGCTTTTTTGAGCGACGAAATAAACTCTTGCAAAAG

G S G Q A P V L N L A G H C N P D N N G C A F L S D E I N S C K S

310 330 350 370 390

TCAAAATGTCAAGGTCCTCCTCTCTATCGGTGGTGGCGCGGGGAGTTATTCACTCTCCTCCGCCGACGATGCGAAACAAGTCGCAAACTTCATTTGGAAC

Q N V K V L L S I G G G A G S Y S L S S A D D A K Q V A N F I W N

410 430 450 470 490

AGCTACCTTGGCGGGCAGTCGGATTCCAGGCCACTTGGCGCTGCGGTTTTGGATGGCGTTGATTTCGATATCGAGTCTGGCTCGGGCCAGTTCTGGGACG

S Y L G G Q S D S R P L G A A V L D G V D F D I E S G S G Q F W D V

510 530 550 570 590

TACTAGCTCAGGAGCTAAAGAATTTTGGACAAGTCATTTTATCTGCCGCGCCGCAGTGTCCAATACCAGACGCTCACCTAGACGCCGCGATCAAAACTGG

L A Q E L K N F G Q V I L S A A P Q C P I P D A H L D A A I K T G

610 630 650 670 690

ACTGTTCGATTCCGTTTGGGTTCAATTCTACAACAACCCGCCATGCATGTTTGCAGATAACGCGGACAATCTCCTGAGTTCATGGAATCAGTGGACGGCG

L F D S V W V Q F Y N N P P C M F A D N A D N L L S S W N Q W T A

710 730 750 770 790

TTTCCGACATCGAAGCTTTACATGGGATTGCCAGCGGCACGGGAGGCAGCGCCGAGCGGGGGATTTATTCCGGCGGATGTGCTTATTTCTCAAGTTCTTC

F P T S K L Y M G L P A A R E A A P S G G F I P A D V L I S Q V L P

810 830 850 870 890

CAACCATTAAAGCTTCTTCCAACTATGGAGGAGTGATGTTATGGAGTAAGGCGTTTGACAATGGCTACAGCGATTCCATTAAAGGCAGCATCGGCTGAAG

T I K A S S N Y G G V M L W S K A F D N G Y S D S I K G S I G *

910 930 950 970 990

GAAGCTCCTAAGTTTAATTTTAATTAAAGCTATGAATAAACTCCAAAGTATTATAATAATTAAAAAGTGAGACTTCATCTTCTCCATTTAGTCTCATATT

1010 1030 1050 1070 1090

AAATTAGTGTGATGCAATAATTAATATCCTTTTTTTCATTACTATACTACCAATGTTTTAGAATTGAAAAGTTGATGTCAATAAAAACATTCCAAGTTTA

TTT

SEQUENCE 3

1 AAAAAGAAAA AAAAAATGAA CTTCCTCAAA AGCTTCCCCT TTTTTGCCTT
51 CCTTTATTTT GGCCAATACT TTGTAGCTGT TACTCATGCT GCCACTTTTG
101 ACATTGTCAA CAAATGCACC TACACAGTCT GGGCCGCGGC CTCTCCAGGT
151 GGAGGCAGGC GGCTCGACTC AGGCCAATCT TGGAGCATTA ATGTGAACCC
201 AGGAACAGTC CAGGCTCGCA TTTGGGGTCG AACCAATTGC AACTTCGATG
251 GCAGTGGCCG AGGTAATTGT GAGACTGGAG ACTGTAACGG GATGTTAGAG
301 TGTCAAGGCT ATGGAAAAGC ACCTAACACT TTAGCTGAAT TTGCACTTAA
351 TCAACCCAAT CAGGACTTTG TCGACATCTC TCTTGTTGAT GGATTTAACA
401 TCCCCATGGA ATTCAGCCCG ACCAATGGAG GATGTCGTAA TCTCAGATGC
451 ACAGCACCTA TTAACGAACA ATGCCCAGCA CAGTTGAAAA CACAAGGTGG
501 ATGTAACAAC CCATGTACTG TGATAAAAAC CAATGAATAT TGTTGTACAA
551 ATGGGCCTGG ATCATGTGGG CCTACTGATT TGTCGAGATT TTTTAAGGAA
601 AGATGCCCTG ATGCTTATAG TTATCCACAG GATGATCCAA CCAGTTTGTT
651 TACGTGTCCT TCTGGTACTA ATTACAGGGT TGTCTTCTGC CCTTGAAATT
701 GAAGCCTGCA AAATTATGAC TATGTAATTT GTAGTTTCAA ATATATAAGC
751 TACACAAGTA GTACTAAGCA CTATTAAATA AAAAAGAGAG TGACAAAGAG
801 GAGAGGCTGT GGGTCAGATT CTCTTGTTCG CTGTTGTCGT TGTTGTAGCA
851 TTCTGGTTTT AAGAAATAAA GAAGATATAT ATCTGCTAAA TTATTAAATG

SEQUENCE 4

```
10 30 50 70 90
AAAAAGAAAAAAAAAAATGAACTTCCTCAAAAGCTTCCCCTTTTTTGCCTTCCTTTATTTTGGCCAATACTTTGTAGCTGTTACTCATGCT
MetAsnPheLeuLysSerPheProPhePheAlaPheLeuTyrPheGlyGlnTyrPheValAlaValThrHisAla

110 130 150 170
GCCACTTTTGACATTGTCAACAAATGCACCTACACAGTCTGGGCCGCGGCCTCTCCAGGTGGAGGCAGGCGGCTCGACTCAGGCCAATCT
AlaThrPheAspIleValAsnLysCysThrTyrThrValTrpAlaAlaAlaSerProGlyGlyGlyArgArgLeuAspSerGlyGlnSer

190 210 230 250 270
TGGAGCATTAATGTGAACCCAGGAACAGTCCAGGCTCGCATTTGGGGTCGAACCAATTGCAACTTCGATGGCAGTGGCCGAGGTAATTGT
TrpSerIleAsnValAsnProGlyThrValGlnAlaArgIleTrpGlyArgThrAsnCysAsnPheAspGlySerGlyArgGlyAsnCys

290 310 330 350
GAGACTGGAGACTGTAACGGGATGTTAGAGTGTCAAGGCTATGGAAAAGCACCTAACACTTTAGCTGAATTTGCACTTAATCAACCCAAT
GluThrGlyAspCysAsnGlyMetLeuGluCysGlnGlyTyrGlyLysAlaProAsnThrLeuAlaGluPheAlaLeuAsnGlnProAsn

370 390 410 430 450
CAGGACTTTGTCGACATCTCTCTTGTTGATGGATTTAACATCCCCATGGAATTCAGCCCGACCAATGGAGGATGTCGTAATCTCAGATGC
GlnAspPheValAspIleSerLeuValAspGlyPheAsnIleProMetGluPheSerProThrAsnGlyGlyCysArgAsnLeuArgCys

470 490 510 530
ACAGCACCTATTAACGAACAATGCCCAGCACAGTTGAAAACACAAGGTGGATGTAACAACCCATGTACTGTGATAAAAACCAATGAATAT
ThrAlaProIleAsnGluGlnCysProAlaGlnLeuLysThrGlnGlyGlyCysAsnAsnProCysThrValIleLysThrAsnGluTyr

550 570 590 610 630
TGTTGTACAAATGGGCCTGGATCATGTGGGCCTACTGATTTGTCGAGATTTTTTAAGGAAAGATGCCCTGATGCTTATAGTTATCCACAG
CysCysThrAsnGlyProGlySerCysGlyProThrAspLeuSerArgPhePheLysGluArgCysProAspAlaTyrSerTyrProGln

650 670 690 710
GATGATCCAACCAGTTTGTTTACGTGTCCTTCTGGTACTAATTACAGGGTTGTCTTCTGCCCTTGAAATTGAAGCCTGCAAAATTATGAC
AspAspProThrSerLeuPheThrCysProSerGlyThrAsnTyrArgValValPheCysProEnd

730 750 770 790 810
TATGTAATTTGTAGTTTCAAATATATAAGCTACACAAGTAGTACTAAGCACTATTAAATAAAAAAGAGAGTGACAAAGAGGAGAGGCTGT

830 850 870 890
GGGTCAGATTCTCTTGTTCGCTGTTGTCGTTGTTGTAGCATTCTGGTTTTAAGAAATAAAGAAGATATATATCTGCTAAATTATTAAATG
```

SEQUENCE 4 (a): Sequence 4 plus amino acid sequence of polypeptide encoded by the coding region of the gene

1 GAGCTCCTCT AGGGGGCCAA GGCAAACCTT TTTGCTAATG GGAAAAAAAT
51 ATTAGACCAA GAGTTTGTAA TAGCTACTCA ATTTCAATTT ACAAAGGGGA
101 AAATTTAACT GTTTTGCCCT TATATCTTTT GGTCCCGAAA CATAAAATAT
151 CCCATCCGAA ATTCCAAATG GTCCATTATC GGCAAGTAGC TTTCTTTTAT
201 TATAGTTAGT TGACAAAACA CTATCGAGAT ATCATTAGTA TAATAATAAC
251 TTCAAAGTCC ATCTTCTTAG CTGCCTCCTC ATTAGAGCCG CCACTAAAAT
301 AAGACCGATC AAATAAAAGC CGCCATTAAA ATAATGAATT TTAGGACTCT
351 CAATTGTCAC GTAAGTGCCA AAACTCTTCC AATACTTTGC TGCAACTTGG
401 GGCTGCTAGC TTCTGAGCTT CCTGGGATAT TTCTATGTTT ATCTCTTAAT
451 TTACATCTCA ACTAATATCA AGAAATTAAA CAGGTGCAGC AAATCATAAA
501 ATTTTCCTCT AAAGAAGAAA ATGACTCCGG TTACTGATTC ATTGGCCTTT
551 TCAGAGTCTG CGTGCCATAT TCACTAATGG GTCGTTTGGT ACAAGAAATA
601 ATGATAATAA TTTCGAAATA GAATTTGGGA TTTCATTTAT TTCATATTTA
651 ATTATAAATA TTAGCTAATT TCGAAATAAA TTTTACATTA AAATAGTGAA
701 ATCAACTATC TCATATATAA GGTGGAATAG CTAATCTCAT AGCCACCTCA
751 GTTAGAATCC AGTTTCCTCT AATAAATGCA GCGAAATATT AGAAGACTTT
801 CATTAAATCA ATTCATATAA TTTAAAAATA CTAGACATGG AAAAAAAAAA
851 CGATTCGAGA CTGTTATGGA AGGCGTTGCC TTCGATGTAG ATTCTCATCC
901 ATTGCTTTCG TGCAATAGCA ATATGACATC TTATTCTTAG AACTACTTTT
951 AAATGAAAGT CATATAGAAC TTTAAAATCT CTCAACTAGT TTTAAGGGAA
1001 TTCAAAATAC GACCAATATT TATTACTTAC TTATGGATAA ATTTAAATAA
1051 TATGTATTTT ATCTTGAAAT TGAATTGAAA ATATTAAATT ACTTGGTTTA
1101 ATATAAACAA TAGATATCGT TAAGTATTTA CCACAAACAT TTTATTAGTT
1151 GTAACGATGA TTAAGCAGGA ATTCCTCTGG TTGTGCAGGA TGAAAGAAAC
1201 TAACTAGCTA TAATTTCTTT TGTAAAGTCA AGATAGTACG GCACCTTATG
1251 GAGAAAATAA ATAACTTTAC ATCATCAAAC TCATCTTCTT TTTTCCACAA
1301 AATGATCAAG TTGACATGTT AATAGCCAGG TCACCGGGGG CGGCTCTTAA
1351 CTTCATTAGC CTACGAATAA CAAATCCAAT ATTATATTTA CACAAGGCTA

SEQUENCE 5 (1)

1401 TATATATATC TCAATATAAA TAGCTCGTTG TTCATCTTAA TTCTCCCAAC
1451 AAGTCTTCCC ATCATGTCTA CCTCACATAA ACATAATACT CCTCAAATGG
1501 CTGCTATCAC ACTCCTAGGA TTACTACTTG TTGCCAGCAG CATTGACATA
1551 GCAGGTTTCT GGTCAAATAT TTGAACTTCC CAGCCAAAAA TATTGTCTTA
1601 TAATTTTGTG TGCGCAAAAT TTTAATTTAG TTGATAGTTA TTTGCTTATT
1651 TTTCTTTTCA AATTGCTTGT GTTTTTTTCT CAAATTAACT TGCACCGTAT
1701 TCATTTAGCG ATAGTTATTT GCTCTATTTT TGTGTAACAC TCACTCACAA
1751 ACTTTTCAAT TTGAGGGGAG GACAGTGAAT CTAAGATTGA AATTTATGAG
1801 TTTAATTAGA CTAATTCCCA TTTGATTTAT TGGCTAGAAG TCAATTATTT
1851 GCATAGTGAG TCTTTTAACA CACAGATTTG AGTTAAAGCT ACTACGTTCG
1901 TATTAACCCA TAACATATAC ACCTTCTGTT CTAATTTCTT TGACACTTTT
1951 TGTTAGTTTG TTCCAAAAAG GACGGACATA TTTGATATTT GAGAATACTT
2001 TACCTTAACC TTAATAGAAT TTTTTATGAC ATCACATATA TTATGGAATA
2051 TATACGACCA TAATTTTCAA ATATCTTATA GTCGTACAAA TATTATAGCA
2101 TGTTTAATAC CACAACTTTC AAATTCTTCT TTTCCTTAAA AACAAAATAT
2151 GTCACATAAA TTAAAATAGA GGAAGTATAC TACATCAATC AGCCCCTAGT
2201 GGAGGGGACC CTACTGTAAG TTTTTAAGTT TTCAAGAATT CAGTAATTGA
2251 TTAGGAGCCC GTCTGGACAT AAAAAAAAAT TCCTTTTTTT CCAAAAAATG
2301 CCCACTAAAT TTCTAACACT ATTTTGTAAT TCTTATTGAG CAGGGGGCTC
2351 AATCGATAGG TGTTTGCTAT GGAATGCTAG GCAACAACTT GCCAAATCAT
2401 TGGGAAGTTA TACAGCTCTA CAAGTCAAGA AACATAGGAA GACTGAGGCT
2451 TTATGATCCA AATCATGGAG CTTTACAAGC ATTAAAAGGC TCAAATATTG
2501 AAGTTATGTT AGGACTTCCC AATTCAGATG TGAAGCACAT TGCTTCCGGA
2551 ATGGAACATG CAAGATGGTG GGTACAGAAA AATGTTAAAG ATTTCTGGCC
2601 AGATGTTAAG ATTAAGTATA TTGCTGTTGG GAATGAAATC AGCCCTGTCA
2651 CTGGCACATC TTACCTAACC TCATTTCTTA CTCCTGCTAT GGTAAATATT
2701 TACAAAGCAA TTGGTGAAGC TGGTTTGGGA AACAACATCA AGGTCTCAAC
2751 TTCTGTAGAC ATGACCTTGA TTGGAAACTC TTATCCACCA TCACAGGGTT
2801 CGTTTAGGAA CGATGCTAGG TGGTTTGTTG ATCCCATTGT TGGCTTCTTA
2851 AGGGACACAC GTGCACCTTT ACTCGTTAAC ATTTACCCCT ATTTCAGTTA

SEQUENCE 5 (2)

2901 TTCTGGTAAT CCAGGCCAGA TTTCTCTCCC CTATTCTCTT TTTACAGCAC
2951 CAAATGTGGT GGTACAAGAT GGTTCCCGCC AATATAGGAA CTTATTTGAT
3001 GCAATGCTGG ATTCTGTGTA TGCTGCCCTC GAGCGATCAG GAGGGGCATC
3051 TGTAGGGATT GTTGTGTCCG AGAGTGGCTG GCCATCTGCT GGTGCATTTG
3101 GAGCCACATA TGACAATGCA GCAACTTACT TGAGGAACTT AATTCAACAC
3151 GCTAAAGAGG GTAGCCCAAG AAAGCCTGGA CCTATTGAGA CCTATATATT
3201 TGCCATGTTT GATGAGAACA ACAAGAACCC TGAACTGGAG AAACATTTTG
3251 GATTGTTTTC CCCCAACAAG CAGCCCAAAT ATAATATCAA CTTTGGGGTC
3301 TCTGGTGGAG TTTGGGACAG TTCAGTTGAA ACTAATGCTA CTGCTTCTCT
3351 CGTAAGTGAG ATGTGAGCTG ATGAGACACT TGAAATCTCT TTACATACGT
3401 ATTCCTTGGA TGGAAAACCT AGTAAAAACA AGAGAAATTT TTTCTTTATG
3451 CAAGATACTA AATAACATTG CATGTCTCTG TAAGTCCTCA TGGATTGTTA
3501 TCCAGTGACG ATGCAACTCT GAGTGGTTTT AAATTCCTTT TCTTTGTGAT
3551 ATTGGTAATT TGGCAAGAAA CTTTCTGTAA GTTTGTGAAT TTCATGCATC
3601 ATTAATTATA CATCAGTTCC ATGTTTGATC AGATTGGGAT TTGGTAACTT
3651 CAATGTTTAG TTATTTATTA ATTAGTGTCT TTATCATTTG ACTATCAATT
3701 AATCTTTATT TGGCAAGGCT TGATATATTT GAGTTACTCT TAGGTATTTG
3751 CAAGCAACTG ATCTTTCTTT TATCCCGTTT CTCCGTTAAA CCTCATTAGA
3801 AATATATTAT AATGTCACCT ACTCTGTGGT TTAAGACATT CCCTTACATT
3851 ATAAGGTATT TCACGTCGTA TCAGGTCGAA AAAAATAATG GTACGCTCTT
3901 TCTTATCACA AATTTCTCTC AACTTCTAGA CCAATTGAAT CTTGTCTCCA
3951 ATAAGCATTG CTTTTACTGT ATGTTTCTCT CTATCAATTC AAGGCTCAAG
4001 CCATCAAATC GCTTGGTATT TCTCGCTCCC ATTTAACCAA TCGAGCTGTT
4051 AGCTCTGCTA AAGTCTCATT CTTCAGCTTC TCAAACCACT CAGAGCTTCT
4101 CCAACCAAAA ATGTAGAAAA AATCTTTGAT CCACATGTAA AACCCCAGAA
4151 TTGTCATTCG AGTGAAGGAA GGTGCTGGAA ACGCGACATC AAACACGGAT
4201 TTGCCAGCTG ATTGTCATAG GAAAAAGAAA AGTTGATCAG ATAGTTCGTG
4251 CGGGGTATTT CCTACCTCGC CTTTGATCAT AGCTCTAGGC TTGCGAAACT
4301 AAGTTATGCA CGAGGCCCCC TGCGAATCCA TAAATCTAAG AAGCATGCCA
4351 CAACAAACGG GATAACTTCC AGCTAAGAGA TCTATTTTGA TCTTACCCAC
4401 AAACTAGCTC CGTGTGAATT GTCCATTTCA TCAACTCCAA AGGCAGGAAA
4451 GAAGACTCTA TTCAGAGAGC AGCAAAAGAG CTC

SEQUENCE 5 (3)

```
   1 GAGCTCCCTT GGGGGGCAAG GGCAAAACTT TTTGCTAAAT GGAAAAATAT
  51 TATACCAAGT GTTTGTAATA GTTACTCAAT TTGAATTAAC AAAGGGGCAA
 101 ATTTGACTAT TTTGCCCTTA TATCTTTTGG TCACAAAAAC ATAAAATATC
 151 CCATCCGAAA TTCCAAATGG TCCATTATCG GCAAGTAGCT TTCTTTAATT
 201 ATAGTTAGTT GACAAAACAC TATCAAGATA TCATTATTAT AATAATAACT
 251 TCAAAGTCCA TCATCTTAGC TGCCTCCTCA GTAGAGCCGC CAGTAAAATA
 301 AGACCGATCA AATAAAAGCC GCCATTAAAA TAATGAATTT TAGGACTCTC
 351 GATTGGCACG TAAGTGCCAA AACTCTTCCA ATACTTTGCT GCAACTTGGG
 401 GCTGCTAGGT TCTGAGCTTC CAGATATGGG ATATTTCTAA GTTTATCTCC
 451 TAATTTACAT CTCAACTAAT ATTAAGAAAT TAAACAGGTA CAGCAAATCA
 501 TAAAATTTTC CTCTAAAGAA GACAATGAAT CCGGTTACTG ATTCATTGGC
 551 CTTTTCAGAG TCTGCATGCC ATATTCACTA AGGGGTCGTT TGGTACAAGA
 601 AATAATAATA ATAATTTCGG GATAGAATTT GAGATTGCAT TTATCTTGTG
 651 TTTAATTATA AGTATTAGCT AATTTCAGAA TAAATTTTAC ACTAAAATAG
 701 TAAAATCAAC TATCACATGT AGAAGGTGGA ATGGAATAGC TAATCCCATA
 751 GCCACTCACA TAGAATATCC TTATTTATCT CACTATTTTA CCAAATGATC
 801 GGTTAGTCTT CATGAGAATC CAGTATCCTC AATAAATGCA GTAAGAAGTT
 851 AGAAAATTTT CATTAAATCA ATTCATATAA TTTAAAAATA TTAGATATGG
 901 AGCACTTAAG ATACAATAAA AGATGTACCG TTAATAATAA AAGATAAGAT
 951 AGAGTTTTAA ATAGGAAAAA AAAAACGGTT CGAGACACTC TTATGGAAGG
1001 CGTTGTCTTC AAAGTAGATT CTCATTCATT GCTCTGGTGC AATAGCAAAA
1051 TGACATCTTA CTCTTAAGAT ACAGCGAGCC ACTCTACAAT CTTCTATTGT
1101 ATACTCAAAT GAAAGTTTTA GAGAACTTTC AAATCTCTCA ACTACTTTTA
1151 AGGGAATTCA AAATACGACC AATATTTATT ACTTACTTAC TTATAGTTAA
1201 ATGATATGAA TTTTATTTTA AATTTGAATT GAAAATATTA AATTACTTGA
1251 TTTAATATAA ACAATAGATA TCGCTAAGTA TTTACCACAA ACATGGAGAT
1301 ACTACAGAAG ATTTTATTAT TTGTAACGAT GATTAAGCAG CTATTCATCT
1351 GGTTGTGCAG GATGAAAGAA AGTAACTAGC TATAATTTCT TTTGTAAAGT
```

SEQUENCE 6 (1)

1401 CAAGATAGTA CGGCACCTTT GGANNAAATA TAAAACTTGT ACATCATCAA
1451 ACTCATTTTC TTTTTTCCAC AAAATGATCA AGTTGACATG TTAATAGCCA
1501 GGTCAGCCGG GGCGGCTCTT AACTTCATTA GCCTACGAAT AACAAATACT
1551 CCAATAATAT TCCACTCGAA TATTTACATT TACACAAGGC TATCTCAATA
1601 TAAATAGCTC ATTGTTCATC TTAATTCTTC CAACAAGTCT TCTCATCATG
1651 TCTACCTCAG ATAAACATAA TACTCCTCAA ATGGCTGCTA TCACACTGCT
1701 AGGATTACTA CTTGTTGCCA GCACCATTGA GATAGCAGGT TTCTGGTCAA
1751 ATATTTGAAC TTCCGAGCCA AAAATATTGT CTTATAATTT TGTTGGTGCA
1801 AAATCTTAAT TTAGTTGATA GTTATTTGCT TATTTTTCTT ATCAAATTGC
1851 TTGTGTTTTT TTCTCAAATT TACTTGCACT ATATTCATTT AGCGATAGTT
1901 ATTTGCTCTT TTTTCGGGTA ACACTCACTC ACAAGCTTTT CAAATTTGAG
1951 GGGAGGGCGG TGAATCTAAA ATTTGAAATT TATGAGTTTA GACTAGTGTC
2001 CATTTGATTT ATTGGCTAGA CGTCTATTAG TTGTATAGTA AATCTTTTAA
2051 CACATACACC GACCTGAGTC AAAGCTATTA GGTTCGTATT AACACATAAC
2101 ACATATTCCC TCTGTTCTAA TTTATGTGAC ACACTTTCTG TTAGTTTCTT
2151 CCAAAGAGAA TGACATATTT GATATTTAAA AATATTTTAA CTTTAAACTT
2201 TTTATTCTCA ACCTTTTATA ACATCACAAA TATTATGGAA CATATTAGAC
2251 TACAAGTTCC AAATATCTTA TAGTCTGTAC AAATATTATA GCATGTTTAA
2301 TAACACAATT TTCACATTCT TCTTTTTCTT AAACTTTGTG CCGAATTAAA
2351 TTATGTCACA TAAATTAAAA TGGTTACATC ATCCCCTAGT GGAGGGACCT
2401 ACCATGTCTA CTGTAAGTTT TTAACTTTTC AAGAATTACA TAATTGATTT
2451 AGTTTCTAAC ACTAATTCTA ATTCTTATTG AGCAGGGGCT CAATCAATAG
2501 GTGTTTGCTA TGGAATGCTA GGCAACAACT TGCCAAATCA TTGGGAAGTT
2551 ATACAGCTCT ACAAGTCAAG AAACATAGGA AGACTGAGGC TTTATGATCC
2601 AAATCATGGA GCTTTACAAG CATTAAAAGG CTCAAACATT GAAGTTATGT
2651 TAGGACTTCC CAATTCAGAT GTCAAGCACA TTGCTTCCGG AATGGAACAT
2701 GCAAGATGGT GGGTACAAAA AAATGTTAAA GATTTCTGGC CAGATGTTAA
2751 GATTAAGTAT ATTGCTGTTG GGAATGAAAT CAGCCCTGTC ACAGGCACAT
2801 CTTACCTTAC CTCATTTCTT ACTCCTGCCA TGGTAAATAT TTACAAAGCA
2851 ATTGGTGAAG CTGGTTTAGG AAACAACATC AAGGTCTCAA CTTCTGTAGA

SEQUENCE 6 (2)

2901 CATGACCTTG ATTGGAAACT CTTATCCACC ATCACAGGGT TCGTTTAGGA
2951 ACGATGCTAG GTGGTTTACT GATCCAATTG TTGGGTTCTT AAGGGACACA
3001 CGTGCACCTT TACTCGTTAA CATTTACCCC TATTTCAGCT ATTCTGGTAA
3051 TCCAGGGCAG ATTTCTCTCC CCTATTCTCT TTTTACAGCA CCAAATGTGG
3101 TAGTACAAGA TGGTTCACGC CAATATAGGA ACTTATTTGA TGCAATGCTG
3151 GATTCTGTGT ATGCTGCCCT CGAGCGATCA GGAGGGGCAT CTGTAGGGAT
3201 TGTTGTGTCC GAGAGTGGCT GGCCATCTGC TGGTGCATTT GGAGCTACAT
3251 ATGACAATGC AGCAACTTAC TTGAGGAACT TAATTCAACA CGCTAAAGAG
3301 GGTAGCCCAA GAAAGCCTGG ACCTATTGAG ACCTATATAT TTGCCATGTT
3351 TGATGAGAAC AACAAGAACC CTGAACTGGA GAAACATTTT GGATTGTTTT
3401 CCCCCAACAA GCAGCCCAAA TATAATCTCA ACTTTGGGGT CTCTGGTGGT
3451 GTTTGGGACA GTTCAGTTGA AACTAATGCT ACTGCTTCTC TCATAAGTGA
3501 GATGTGAGAT GAGACACTTG AAATCTCTTT ACATAAGTAT TGCTTAGATG
3551 GAAAGCTTAG TAAAAACAAG AGAAATTTAT TCTTCATGCA AGACACTAAA
3601 TAACATTGCA CGTCTCTGTA AGTCCTCATG GATTGTTATC CAGAGACGAT
3651 GCAACTCTGA GTGGTTTTAA ATCCCTTTTC TTTGTGATGT TGGTAATTTG
3701 GCAAGAAACT TTCTGTAAGT TTGTGAATTT CATGACTTTC TGTAAGTTTG
3751 TGAATTTCAT GCACCATCAA TTATACATCT TTTCCATGTT TGATCACATT
3801 AGGATTTGGT AATTGCAAAG TTTAGTTATT TATTAATTAG TGTCTTTATC
3851 ATTTGACTCG ATCAATTAAT CTTTAATTGG TAAGGCTTGA TATATCGGAG
3901 CGACTCTTAG GTAGTGGCAT TCAACTGATC TTTCTGTTAT CCCATGTCTC
3951 CGTTAACCCT CATTAGAAAT ATATTATAAT GTCACCTAAA TCAGAGGTTT
4001 TAGAGCTTCA AAATCGATTG ACAACAGTTT TGGAGTTACA CTGTGGTTTA
4051 GGACATTCTG TTACATTATA AGGTATTTCA CGTCGTATCA AGGTCGAATA
4101 AAATAATGGT ACGCTCTTTC TTATCACAAA TTTCTCTCAA CTTCTAGACC
4151 AATTGAATCT TGTCTCCAAT AAGTATTGCT TTTACTCTAT GTTTCTCTCT
4201 ATCAATTCAA GGCTCAAGCC ATCAAATCGC TTGGTATTTC TCGCTCTCAA
4251 TTAACCAATC GAGCTGTTAA CTCTGCTAAA GTCTCATTCT TCAGCTTCTC
4301 AAACCACTCA AAGCTTCTCC AACAAAAAAA GTAGAAAAAA ACTTTGATCC
4351 ACATGTAAAA CCCCATAACC ATGTCATTCG AGTGAAGGAA GGTGCTTGAA

SEQUENCE 6 (3)

4401 ATGCAACGAA CAAACACGGC TTTGCCACTG CTTGTGATAG GTAAAAGAAA

4451 ACTTGATTAG ATAGTTCGTG CGGGGCATTT CCTACCTCGC CTTTGATCTT

4501 AGCTTTAGGC TTGCGAAACT AAGTAGAACC TAAGGCCCCA GCGAATCCGT

4551 AAATCTGAGG AGCATGCCAC AGCAAAAAGG TTAGCTTTCA GATAAGAGAT

4601 CTATTTGATC TTACCCACTA ACTAGCTCTG TGTGAATTGT CCATTTCATC

4651 AACTCCAAAG GCAGGAAAGA AGACTATTGA GAGAGCAGCA AAAGAGCTC

SEQUENCE 6 (4)

| | |
|---|---|
| 1- 50 | AAAAAAAAAA AAAAACATAA GAAAGTACAG AGGAAAATGG AGTTTTCTGG |
| 51-100 | ATCACCAATG GCATTGTTTT GTTGTGTGTT TTTCCTGTTC TTAACAGGGA |
| 101-150 | GCTTGGCACA AGGCATTGGT TCTATTGTAA CGAGTGACTT GTTCAACGAG |
| 151-200 | ATGCTGAAGA ATAGGAACGA CGGTAGATGT CCTGCCAATG GCTTCTACAC |
| 201-250 | TTATGATGCA TTCATAGCTG CTGCCAATTC CTTTCCTGGT TTTGGAACTA |
| 251-300 | CTGGTGATGA TACTGCCCGT AGGAAAGAAA TTGCTGCCTT TTTCGGTCAA |
| 301-350 | ACTTCTCACG AAACTACTGG TGGATCCCTG AGTGCAGAAC CATTTACAGG |
| 351-400 | AGGGTATTGC TTTGTTAGGC AAAATGACCA GAGTGACAGA TATTATGGTA |
| 401-450 | GAGGACCCAT CCAATTGACA AACCGAAATA ACTATGAGAA AGCTGGAACT |
| 451-500 | GCAATTGGAC AAGAGCTAGT TAACAACCCT GATTTAGTGG CCACAGATGC |
| 501-550 | TACTATATCA TTCAAAACAG CTATATGGTT TTGGATGACA CCACAGGACA |
| 551-600 | ACAAGCCATC TTCCCACGAC GTTATCATCG GTCGTTGGAC TCCGTCTGCC |
| 601-650 | GCGGATCAGG CGGCGAATCG AGTACCAGGT TACGGTGTAA TTACCAACAT |
| 651-700 | CATTAACGGT GGAATTGAAT GTGGCATAGG ACGGAATGAC GCAGTGGAAG |
| 701-750 | ATCGAATTGG ATACTACAGG AGGTATTGTG GTATGTTAAA TGTTGCTCCG |
| 751-800 | GGGGAAAACT TGGACTGTTA CAACCAAAGG AACTTCGGCC AGGGCTAGGC |
| 801-850 | TTCGTTACAT AGAATGCAGA TCATGTTATG TATACAAGTT ATATTTGTAT |
| 851-900 | TAATTAATGA ATAAGGGGAT TGTGTATCCA TTAAGAATTA GGTGAAATAT |
| 901-950 | TTCTGTTATT TGTCTTCTTG GGAAGAACCA ATAGCTCCTA TATATGAGGC |
| 951- 0 | GCTTTTAAGT GATGAGGCTA CTGCATTGAT GAAAACGAAA TTTCTATCCA |
| 1001- 20 | GAAATAAAAG TTCCTTGTCT |

SEQUENCE 7

| | |
|---|---|
| 1- 50 | CAGGTGCTCA AGCAGGAGTT TGTTATGGAA GGCAAGGGAA TGGATTACCA |
| 51-100 | TCTCCAGCAG ATGTTGTGTC GCTATGCAAC CGAAACAACA TTCGTAGGAT |
| 101-150 | GAGAATATAT GATCCTGACC AGCCAACTCT CGAAGCGCTT AGAGGCTCCA |
| 151-200 | ACATTGAGCT CATGCTAGGT GTCCCGAATC CGGACCTTGA GAATGTTGCT |
| 201-250 | GCTAGCCAAG CCAATNCAGA TACTTGGGTC CAAAACAATG TTAGGAACTA |
| 251-300 | TGGTAATGTC AAGTTCAGGT ATATAGCAGT TGGAAATGAA GTTAGTCCCT |
| 301-350 | TAAATGAAAA CTCTAAGTAT GTACCTGTCC TTCTCAACGC CATGCGAAAC |
| 351-400 | ATTCAAACTG CCATATCTGG TGCTGGTCTT GGAAACCAGA TCAAAGTCTC |
| 401-450 | CACAGCTATT GAAACTGGAC TTACTACAGA CACTTCTCCT CCATCAAATG |
| 451-500 | GGAGATTCAA AGATGATGTT CGACAGTTTA TAGAGCCTAT CATCAACTTC |
| 501-550 | CTAGTGACCA ATCGCGCCCC TTTGCTTGTC AACCTTTATC CTTACTTTGC |
| 551-600 | AATAGCAAAC AATGCAGATA TTAAGCTTGA GTATGCACTT TTTACATCCT |
| 601-650 | CTGAAGTTGT TGTAAATGAT AACGGAAGAG GATACCGAAA CCTTTTTGAT |
| 651-700 | GCCATCTTAG ATGCCACATA CTCGGCCCTT GAAAAGGCTA GTGGCTCGTC |
| 701-750 | TTTGGAGATT GTTGTATCAG AGAGTGGTTG GCCTTCAGCT GGAGCAGGAC |
| 751-800 | AATTAACATC CATTGACAAT GCCAGGACTT ATAACAACAA CTTGATTAGT |
| 801-850 | CACGTGAAGG GAGGGAGTCC CAAAAGGCTT CCGGTCCAAT AGAGACCTAC |
| 851-900 | GTTTTCGCTC TGTTTGATGA AGATCAGAAA GACCCTGAAA TTGAGAAGCA |
| 901-950 | TTTTGGACTA TTTTCAGCAA ACATGCAACC AAAGTACCAG ATCAGTTTTA |
| 951- 0 | ACTAGTTAAA AGCAAGAGGA GAGCATTAAT AGGAATAAGG ACTTTCCTTT |
| 1001- 50 | GTATGAAGAG AAAGTAGTCC ATTGGCACTA TGTACTGAAA CTATATATCA |
| 1051-100 | TGCTCATAAA GAAAGCAGTT ATTACAATAA TGAAACACTT ACAAGAAAAG |
| 1101-107 | CCATCAA |

SEQUENCE 8

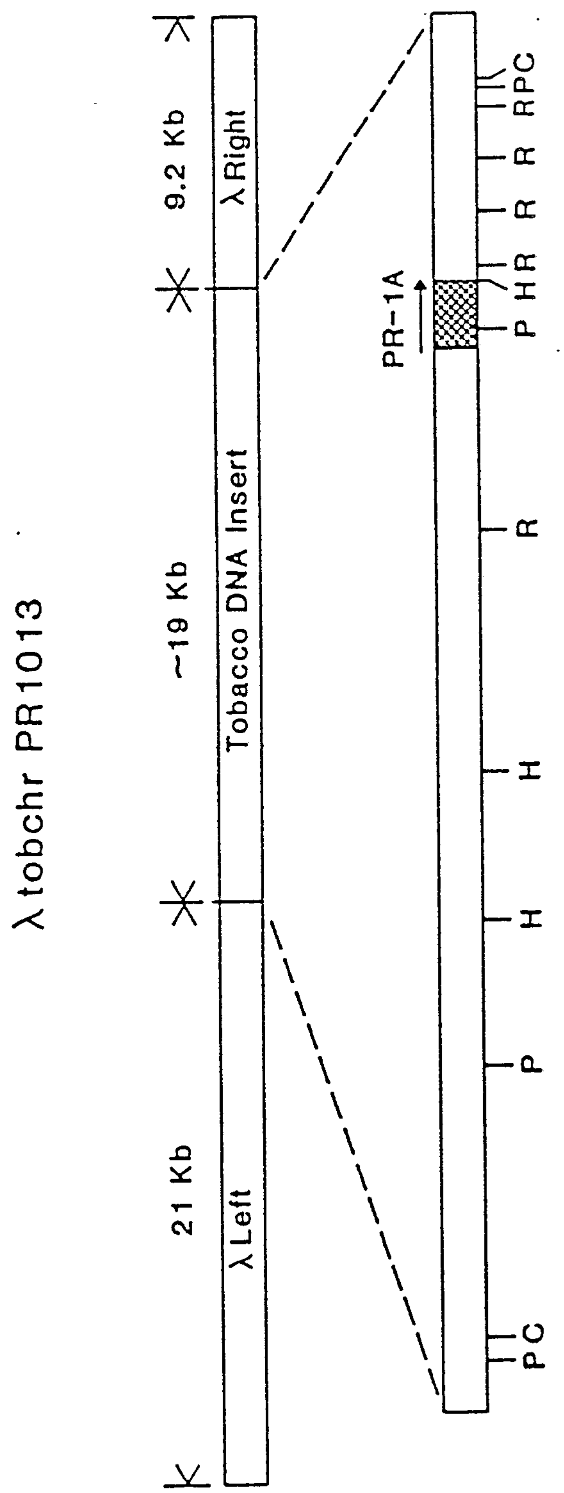
λ tobchr PR1013
9.2 Kb
~19 Kb
21 Kb
λ Right
Tobacco DNA Insert
λ Left
PR-1A
RPC
R
R
PHR
R
H
H
P
PC

FIGURE 1

Construction of pBS-PR1013 Cla From λtobchr PR1013

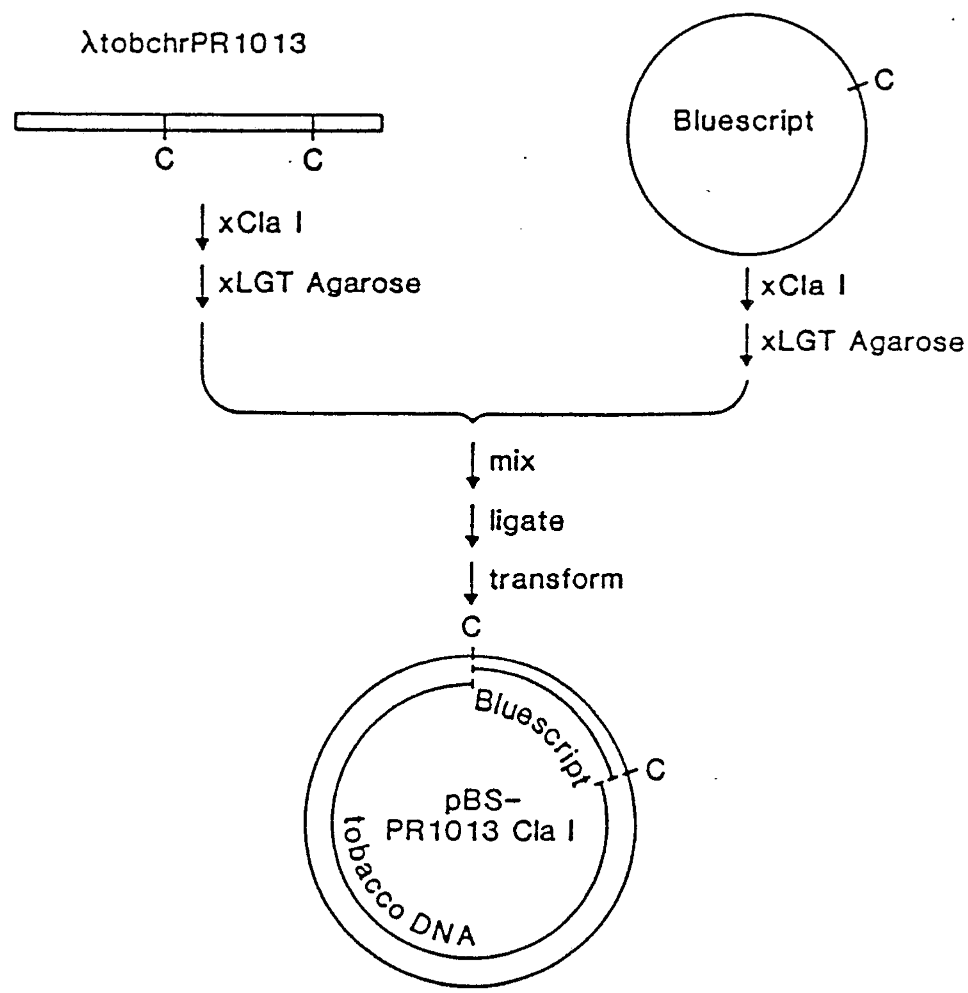


FIGURE 2

Construction of pBS-PR1013 Eco
From λtobchr PR1013

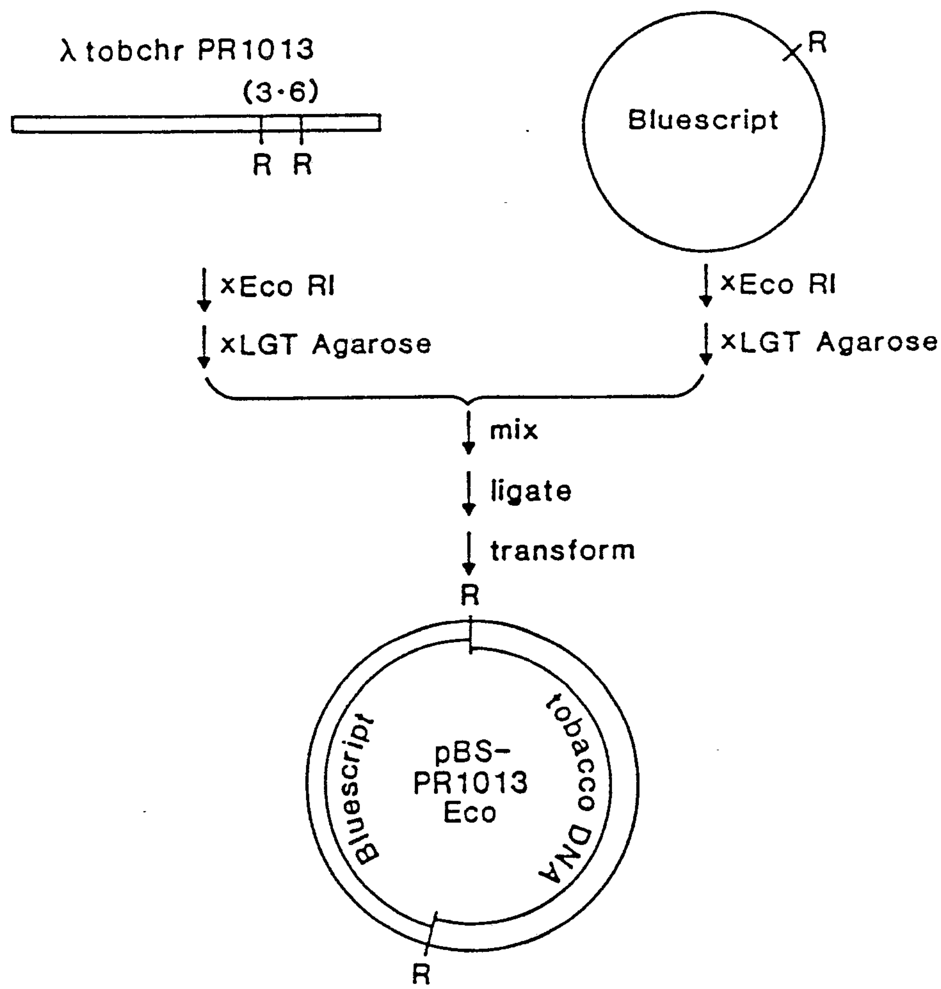


FIGURE 3

Construction of pBS-PR1013 Eco
From pBS-PR1013 Cla

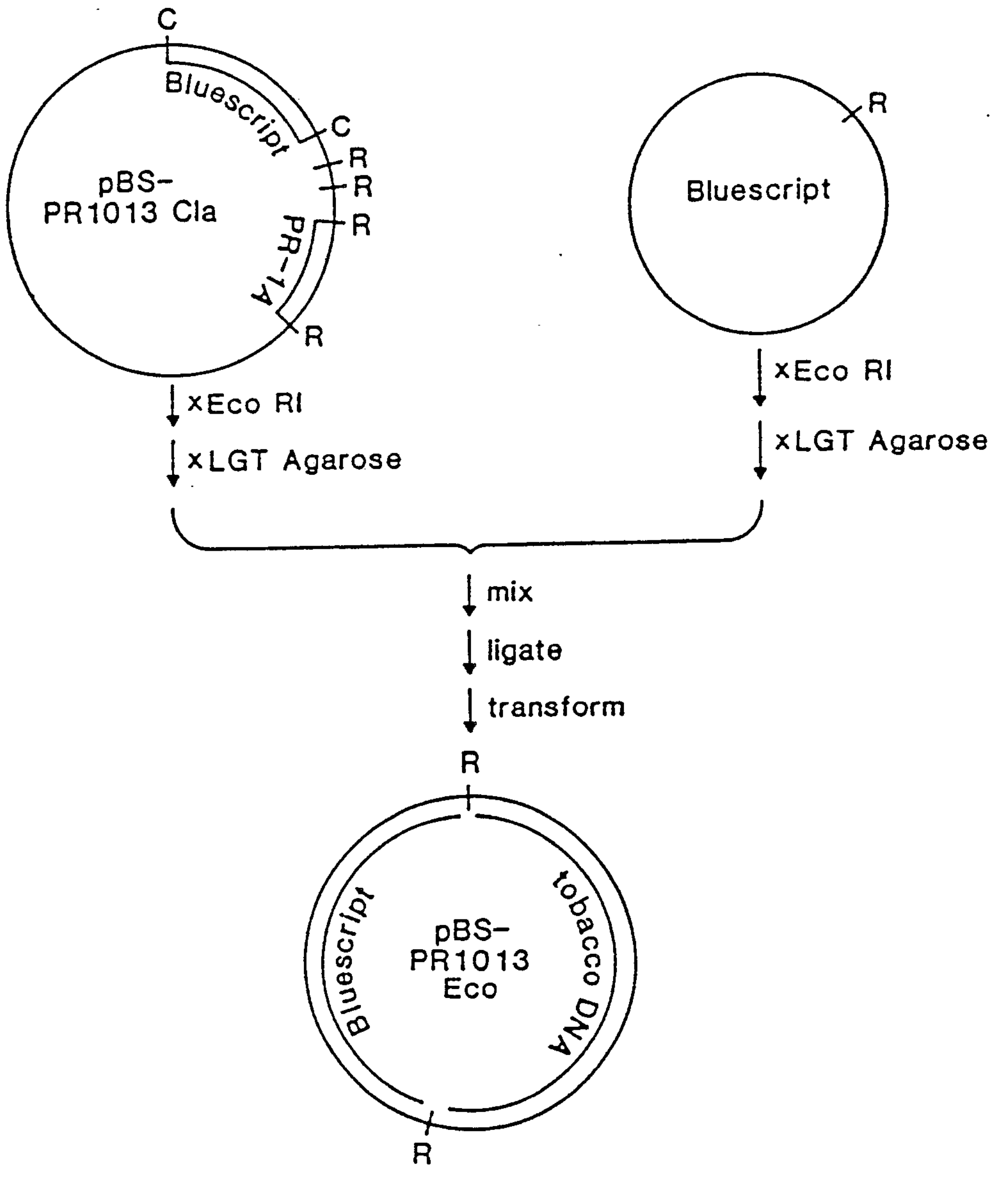


FIGURE 4

Construction of pBS-PR1013 EcoΔPst
From pBS -PR1013 Eco

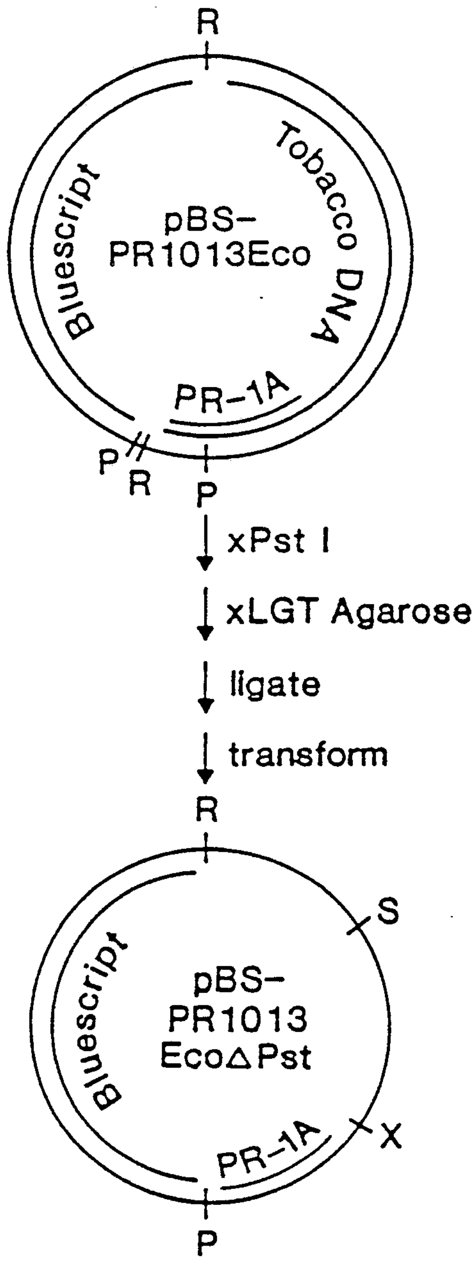


FIGURE 5

Construction of pBS-PR1013 Eco△Pst△Xho
From pBS-PR1013 Eco△Pst

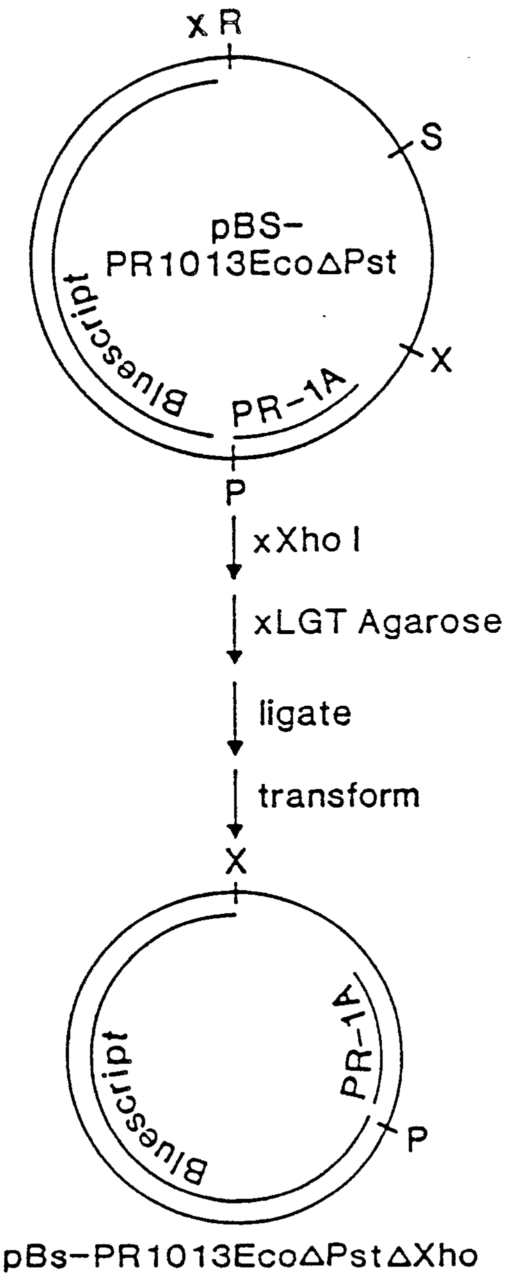


FIGURE 6

Construction of pCIB270

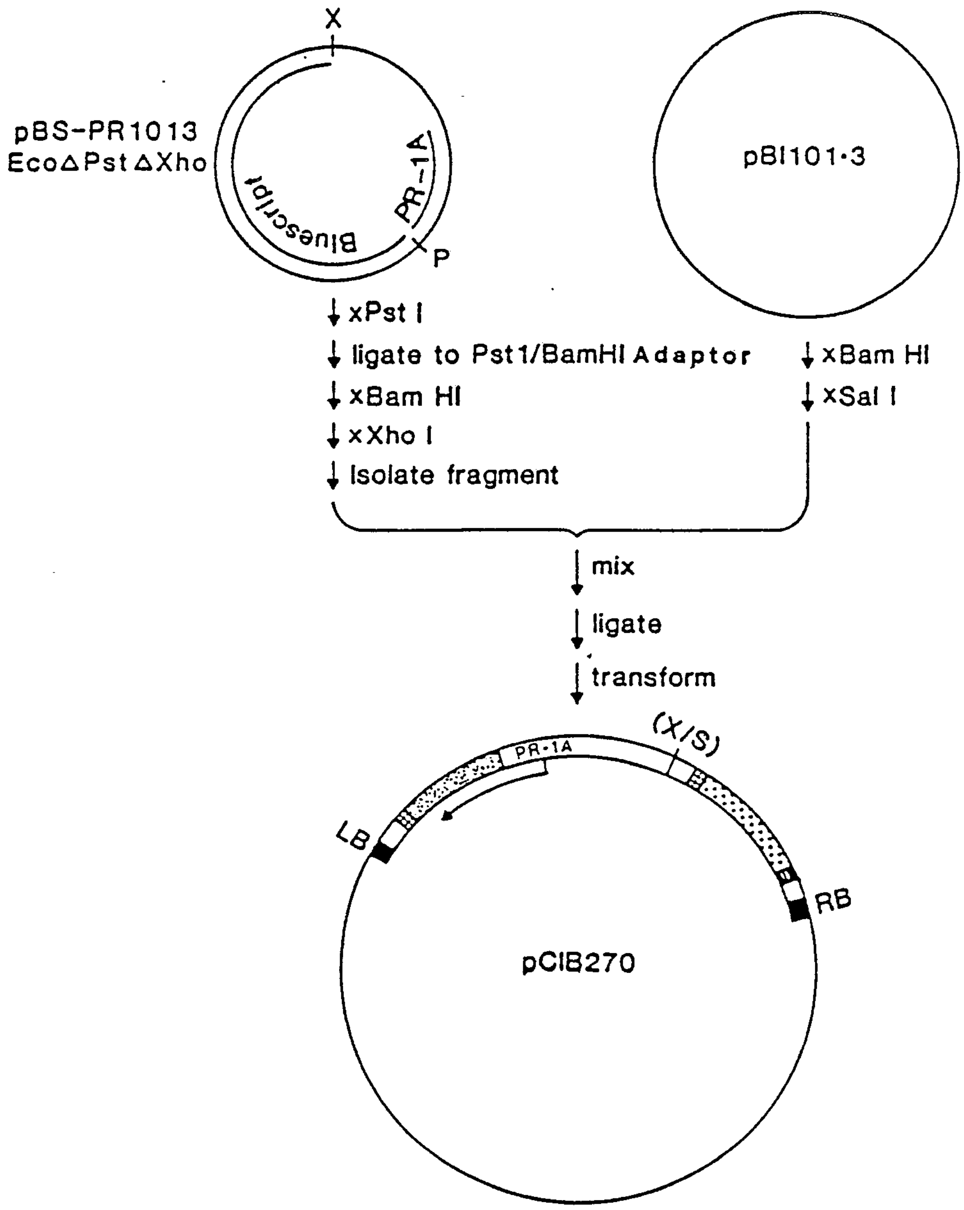


FIGURE 7

Construction of M13 mp18-PR1013 EcoΔ PstΔXho and M13mp19-PR1013 EcoΔ PstΔXho

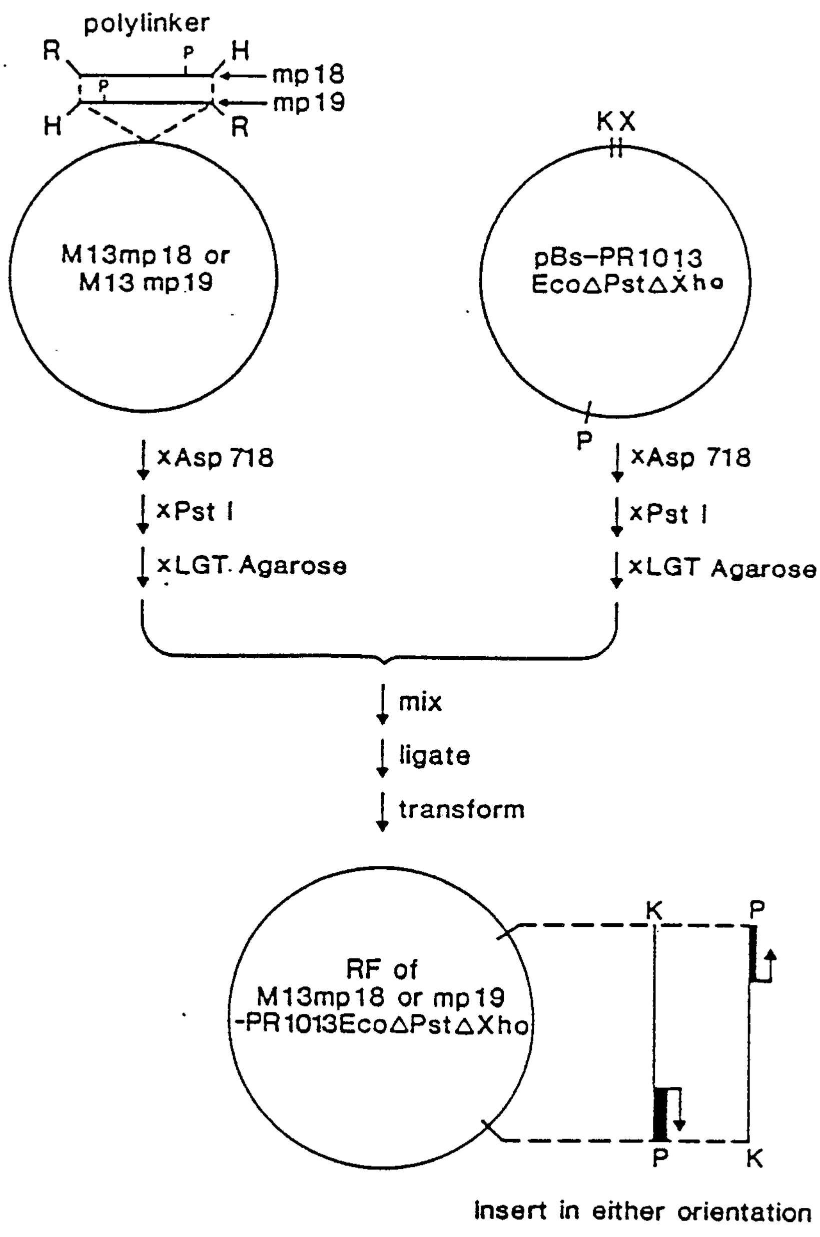


FIGURE 8

Conversion of ATG to Nco I Site

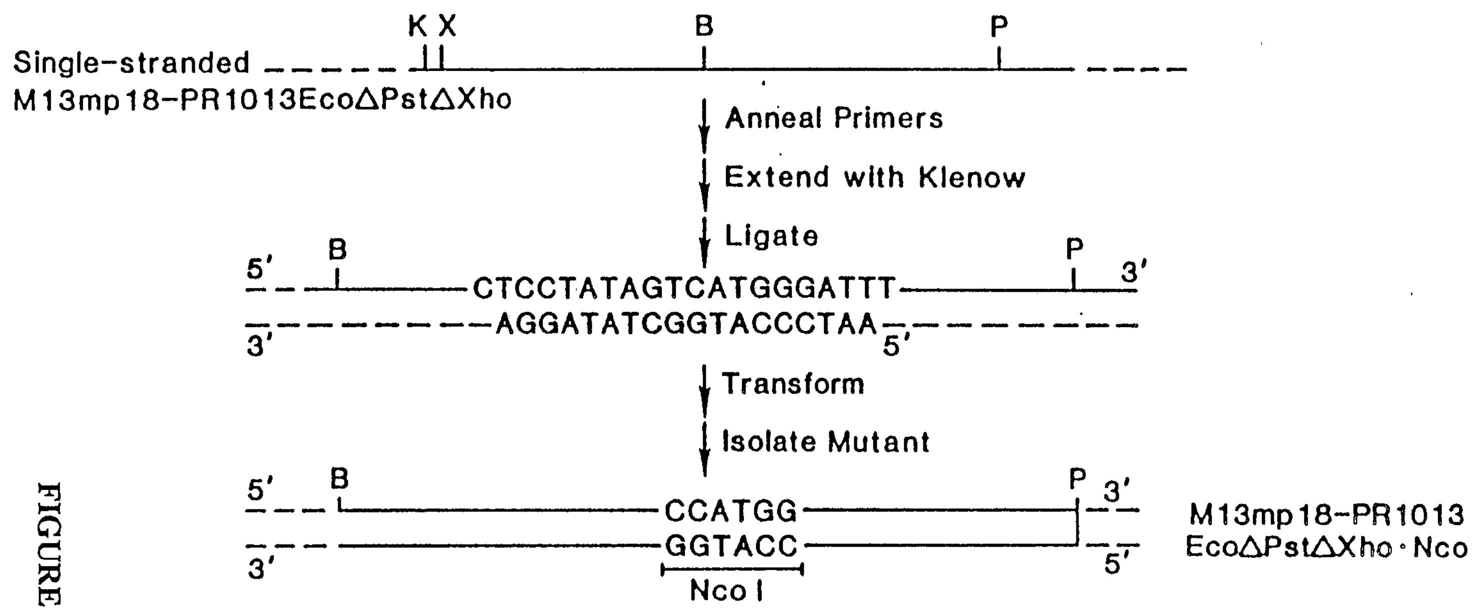


FIGURE 9

Construction of pCIB268

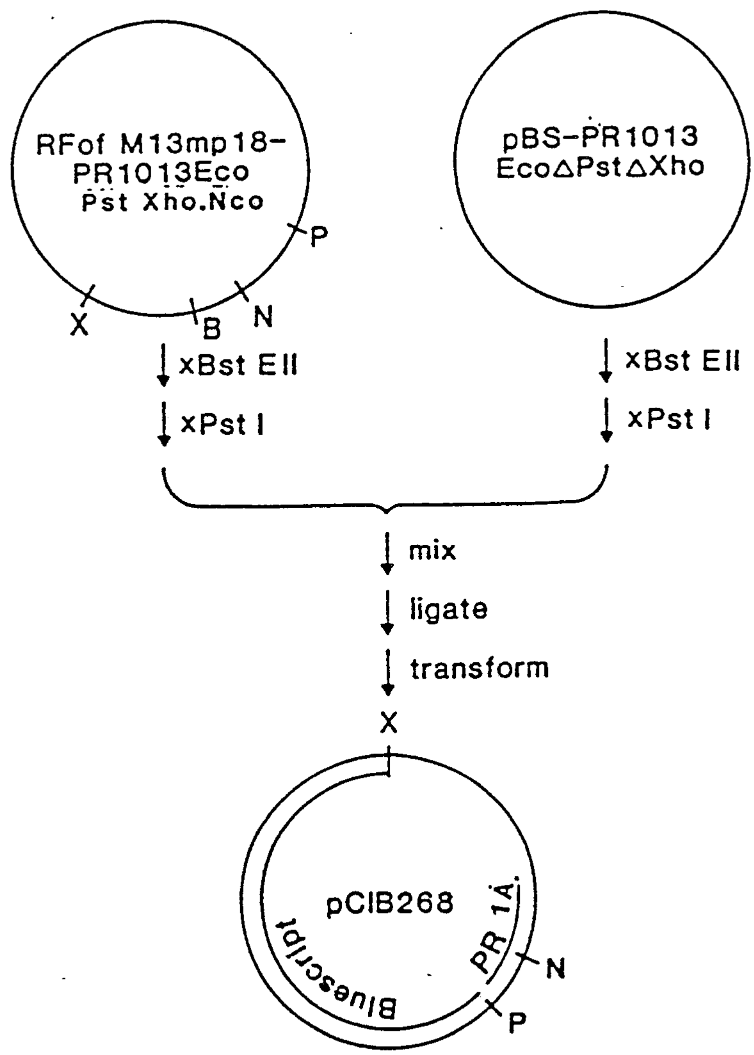


FIGURE 10

Construction of pCIB269

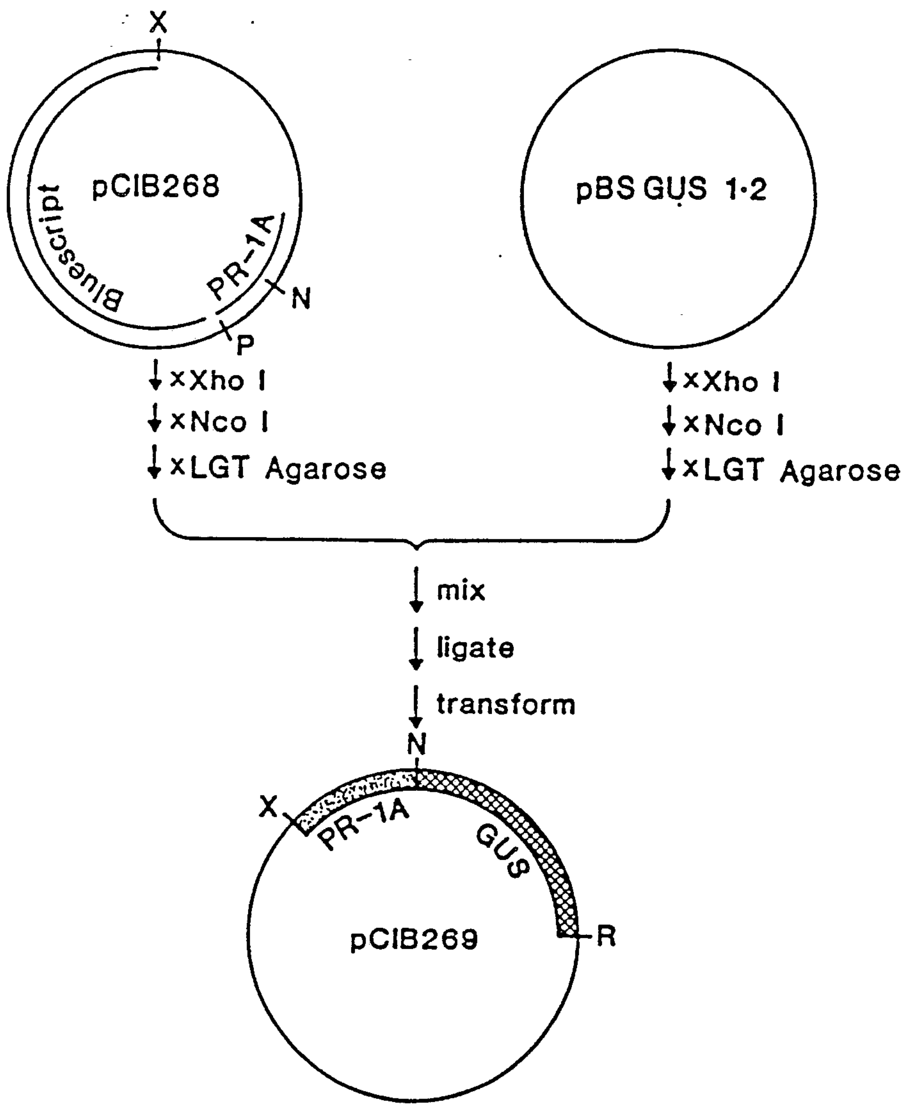


FIGURE 11

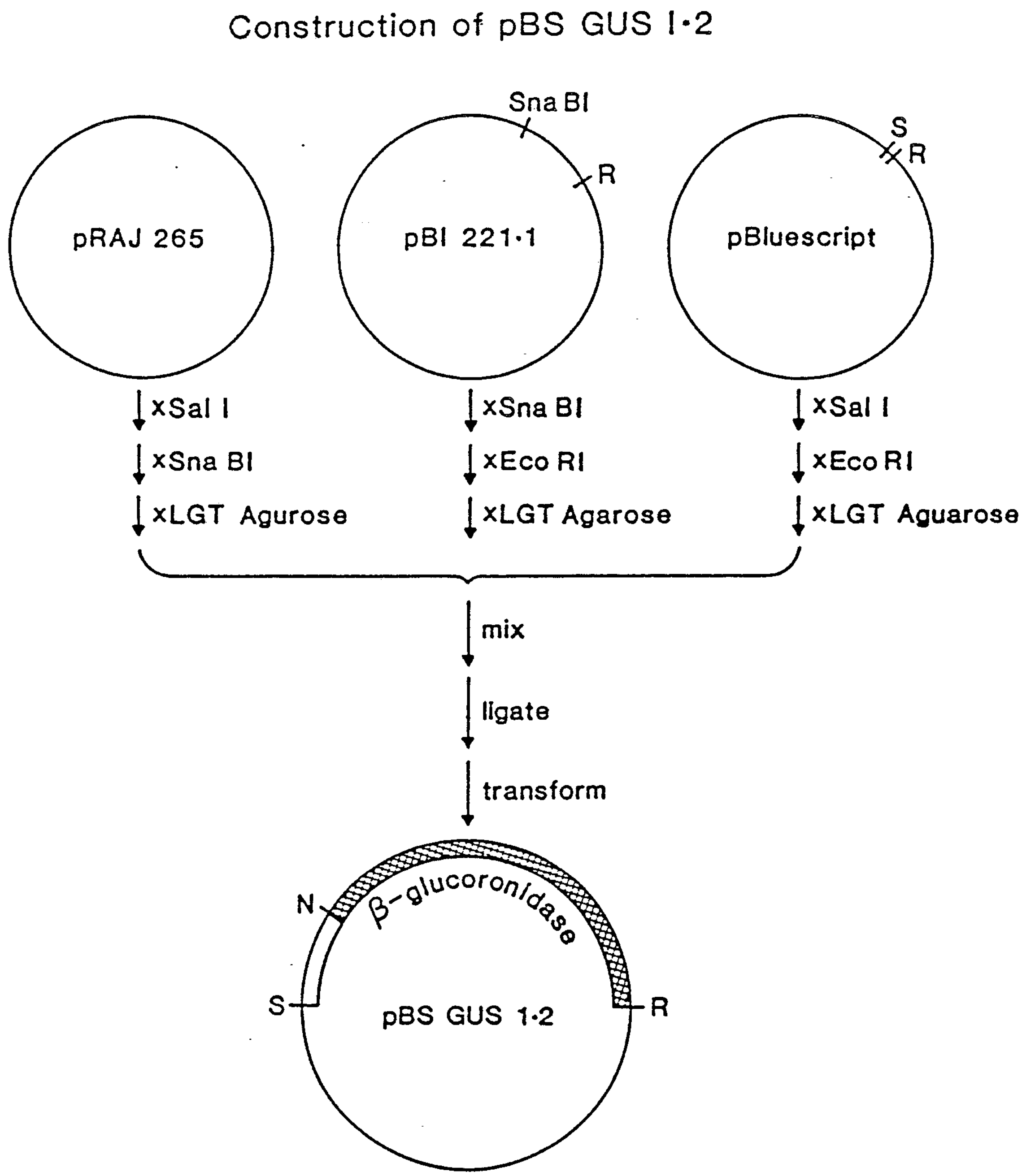
Construction of pBS GUS I·2
Sna BI
R
S
R
pRAJ 265
pBI 221·1
pBluescript
xSal I
xSna BI
xLGT Agurose
xSna BI
xEco RI
xLGT Agarose
xSal I
xEco RI
xLGT Aguarose
mix
ligate
transform
N
β-glucoronidase
S
R
pBS GUS 1·2

FIGURE 12

Construction of pCIB271

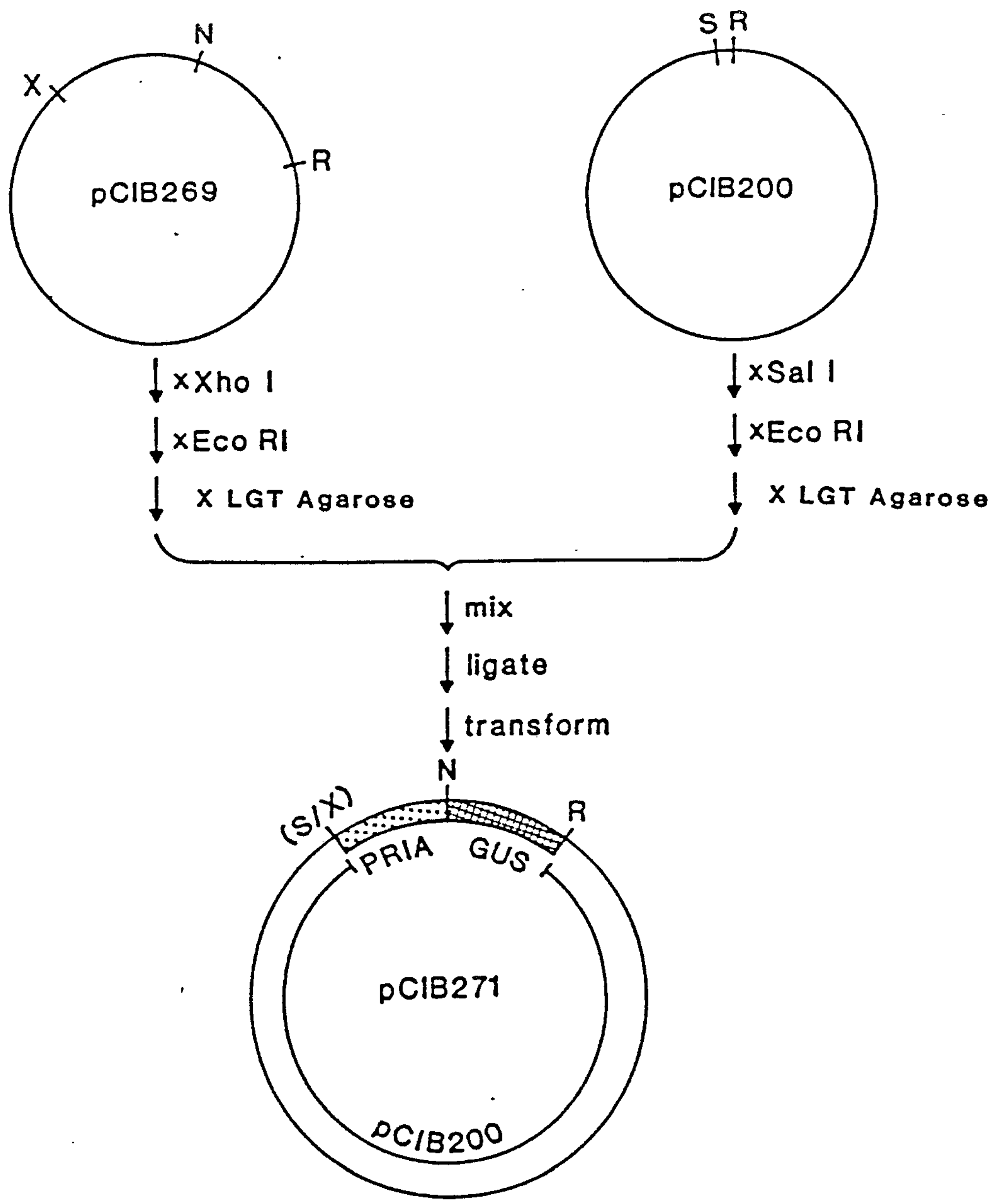


FIGURE 13

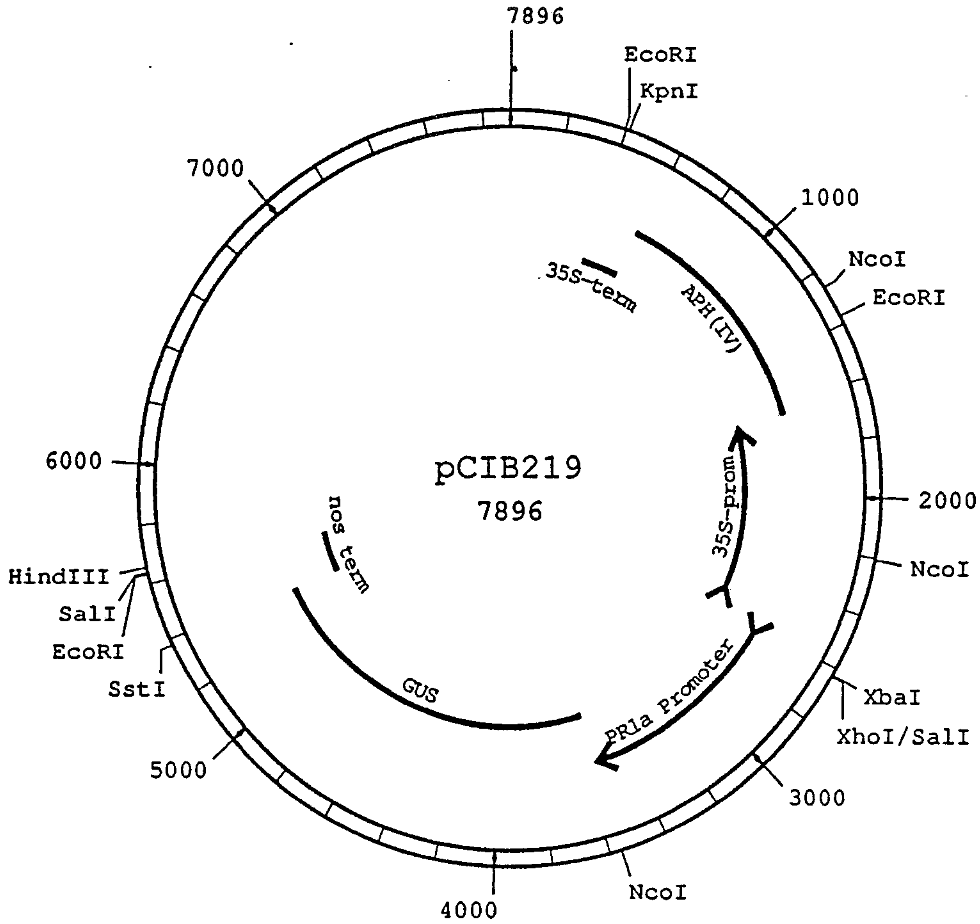
7896
EcoRI
KpnI
7000
1000
NcoI
EcoRI
35S-term
APH(IV)
pCIB219
7896
35S-prom
6000
2000
nos term
NcoI
HindIII
SalI
EcoRI
SstI
GUS
PR1a Promoter
XbaI
XhoI/SalI
5000
3000
4000
NcoI

FIGURE 14

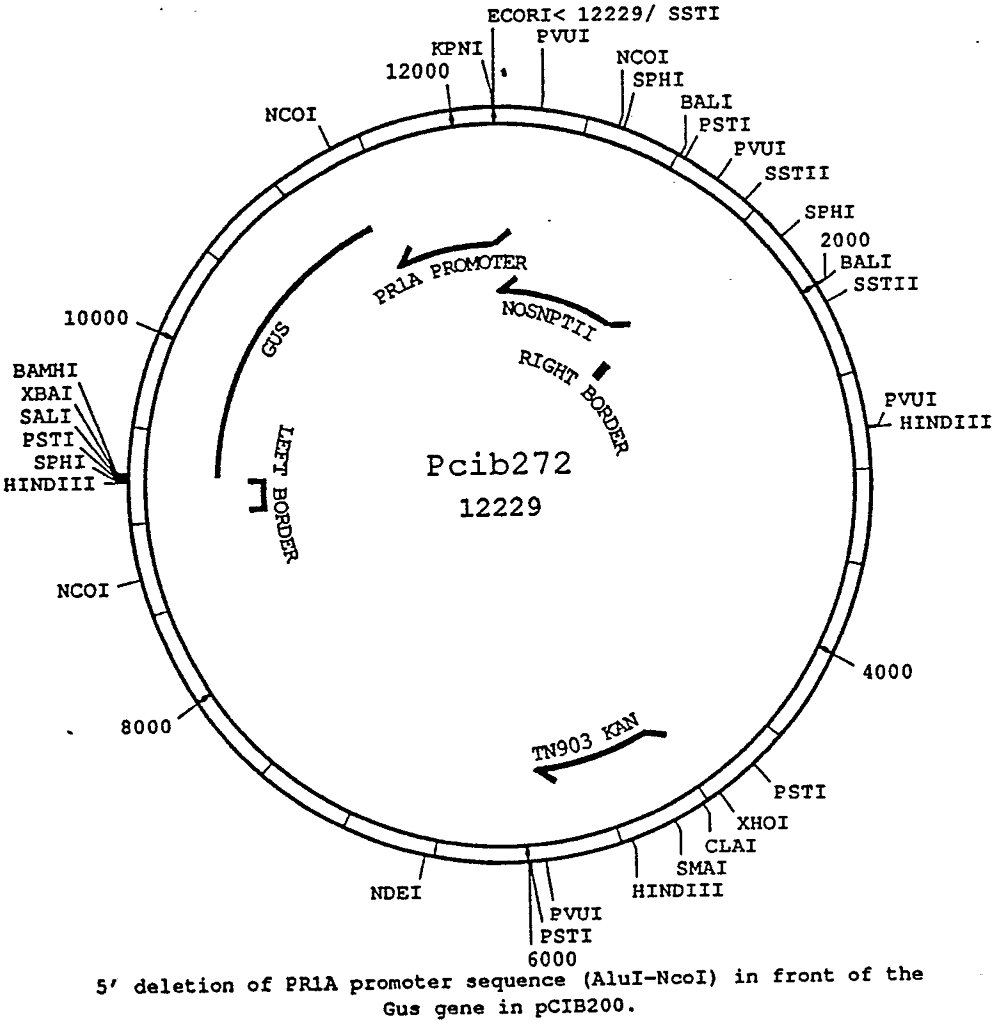


5′ deletion of PR1A promoter sequence (AluI-NcoI) in front of the Gus gene in pCIB200.

FIGURE 15

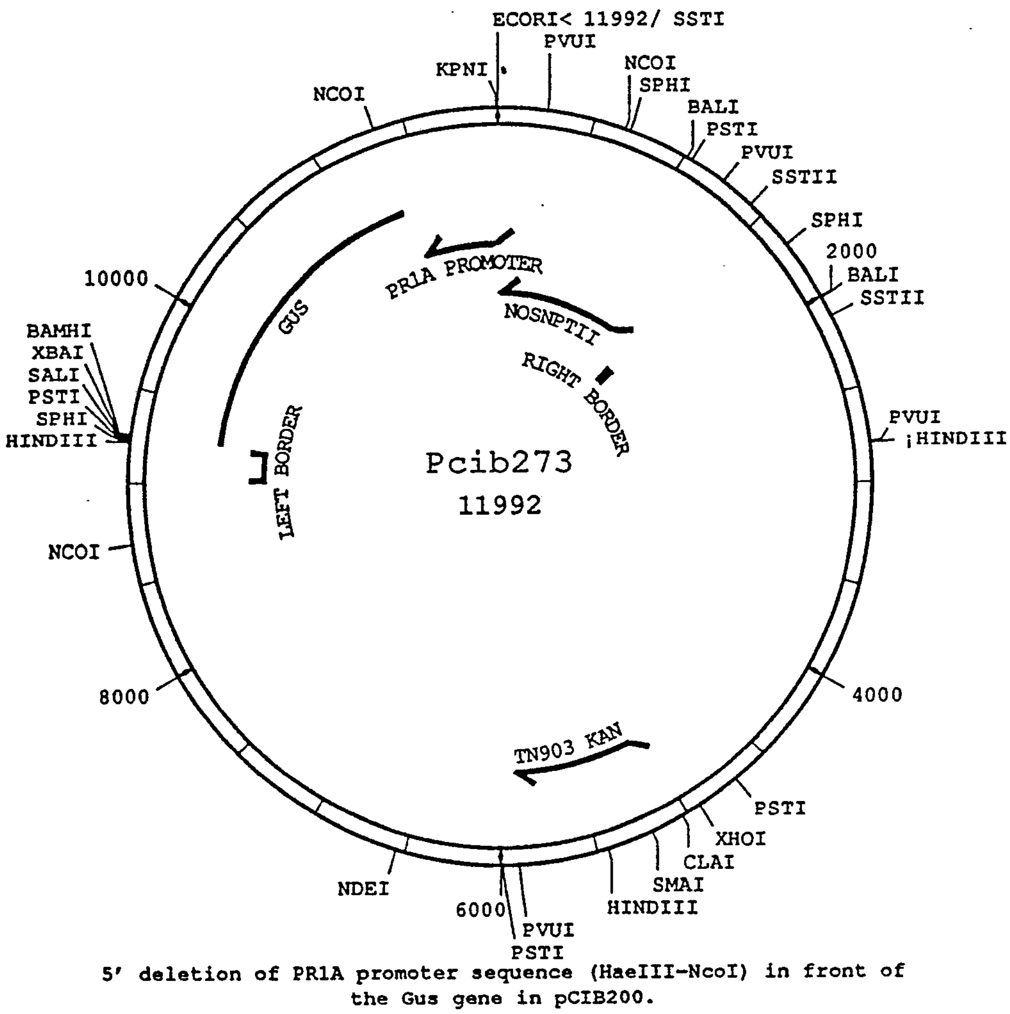


5' deletion of PR1A promoter sequence (HaeIII-NcoI) in front of the Gus gene in pCIB200.

FIGURE 16

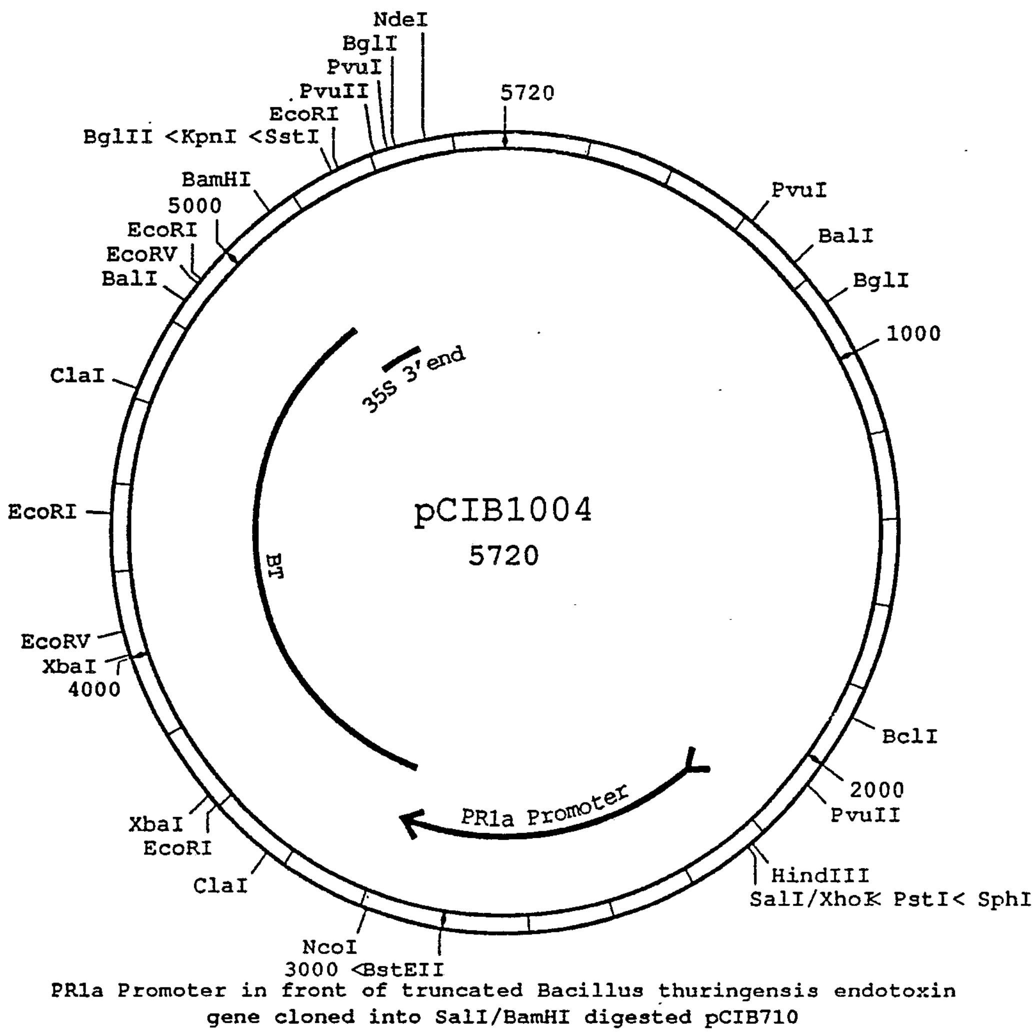


PR1a Promoter in front of truncated Bacillus thuringensis endotoxin gene cloned into SalI/BamHI digested pCIB710

FIGURE 17

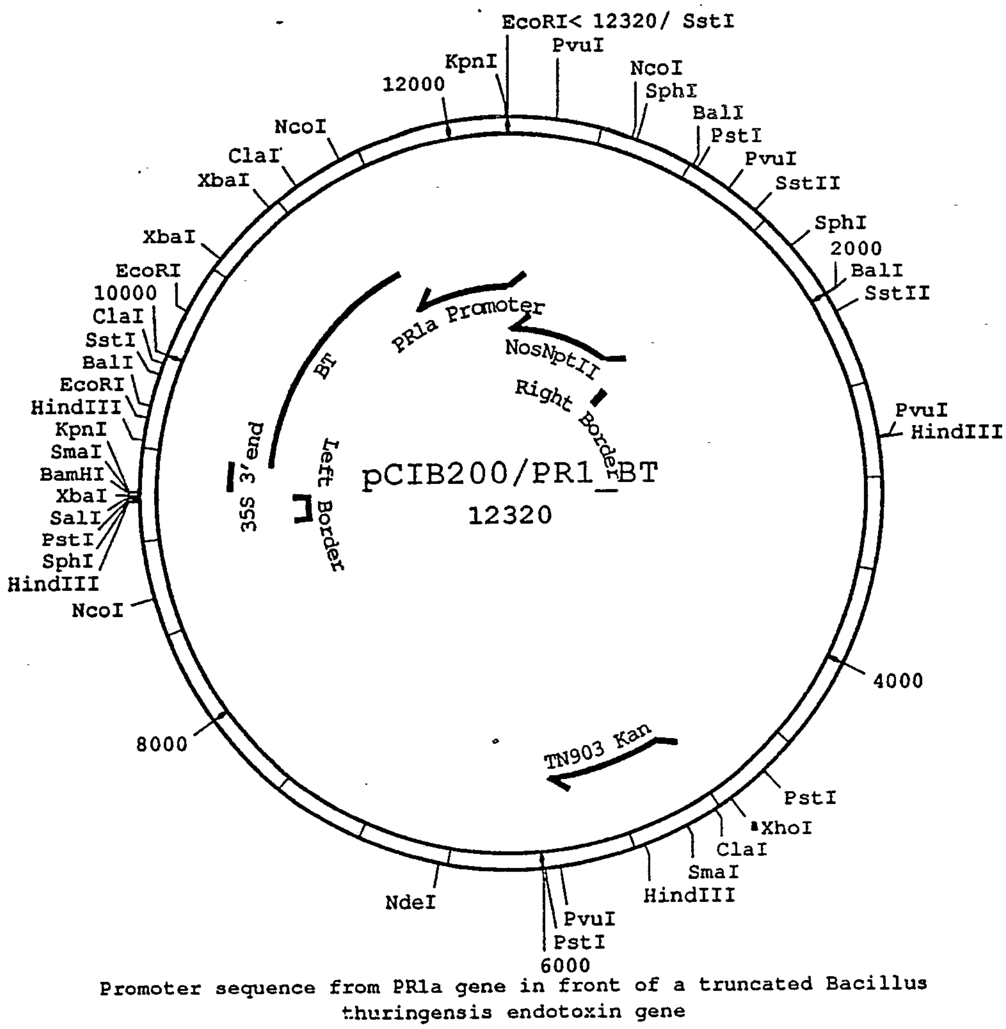

Promoter sequence from PR1a gene in front of a truncated Bacillus thuringensis endotoxin gene

FIGURE 18

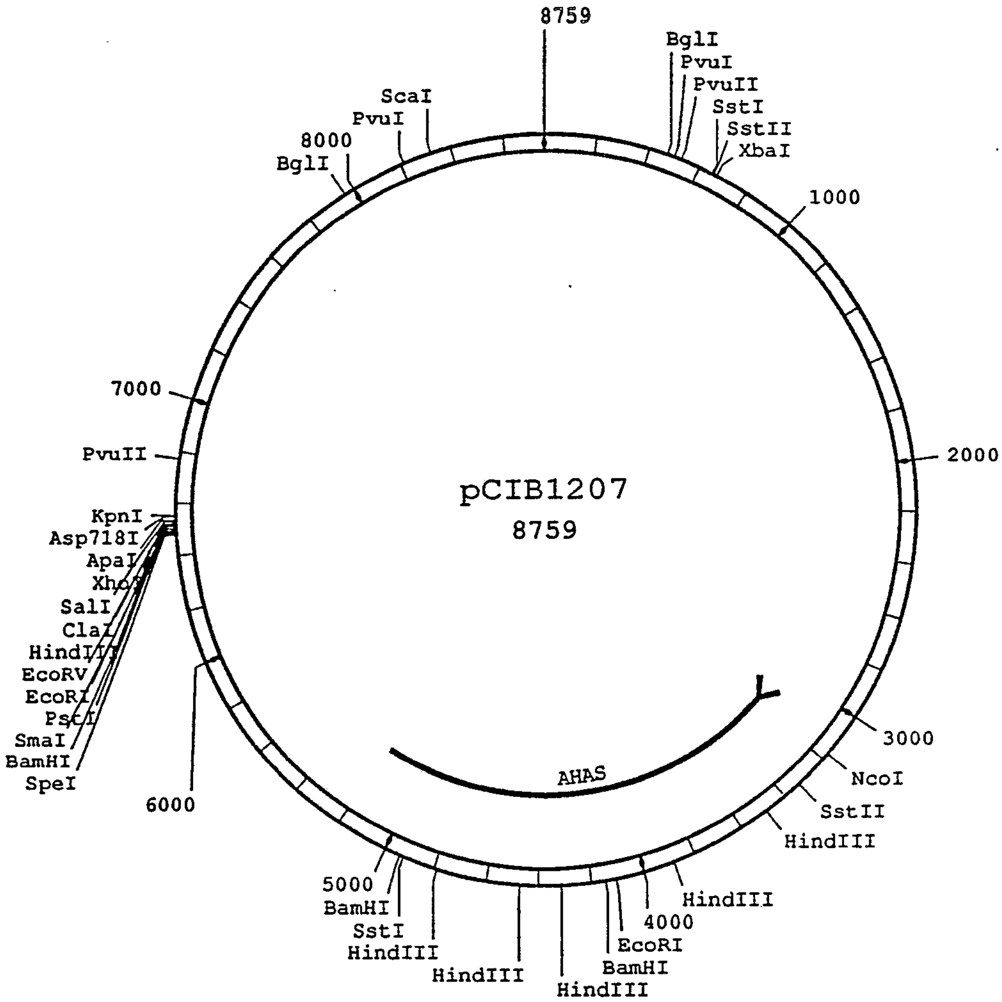
8759
BglI
PvuI
PvuII
SstI
SstII
XbaI
1000
2000
3000
NcoI
SstII
HindIII
HindIII
4000
EcoRI
BamHI
HindIII
HindIII
HindIII
SstI
BamHI
5000
6000
SpeI
BamHI
SmaI
PstI
EcoRI
EcoRV
HindIII
ClaI
SalI
XhoI
ApaI
Asp718I
KpnI
PvuII
7000
BglI
8000
PvuI
ScaI
pCIB1207
8759
AHAS

FIGURE 19

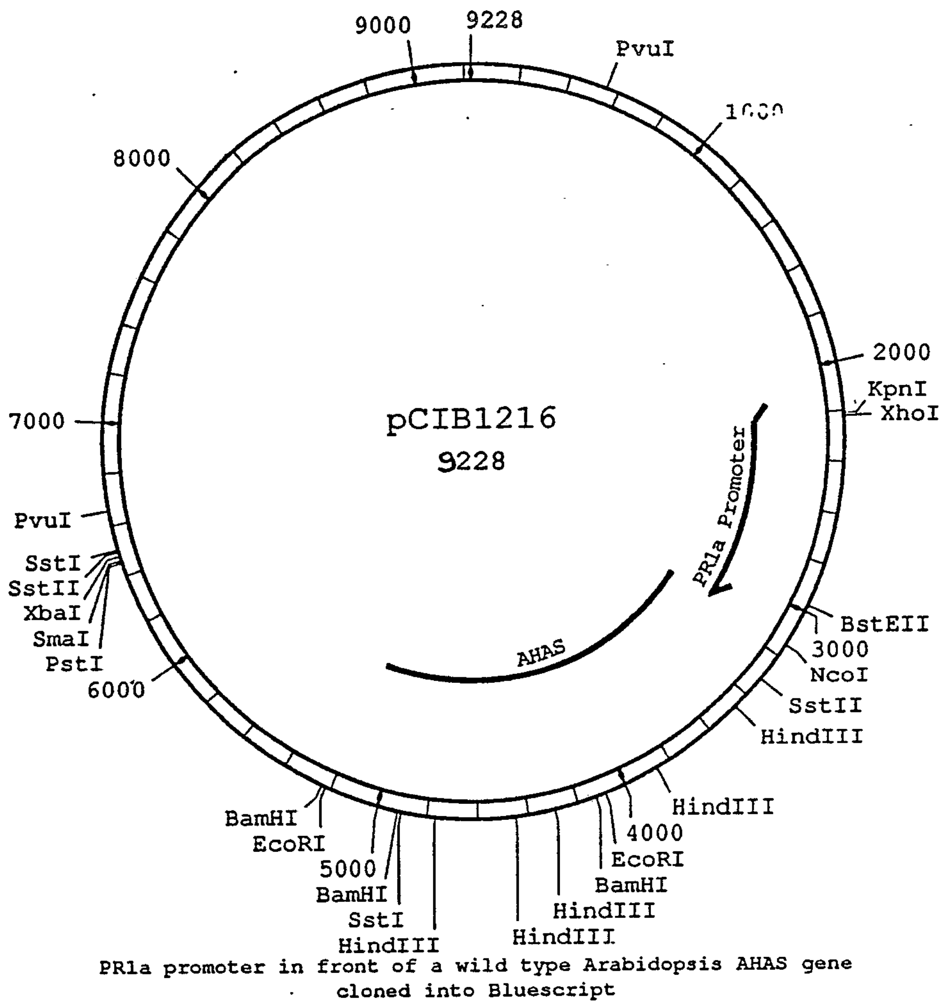

PR1a promoter in front of a wild type Arabidopsis AHAS gene cloned into Bluescript

FIGURE 20

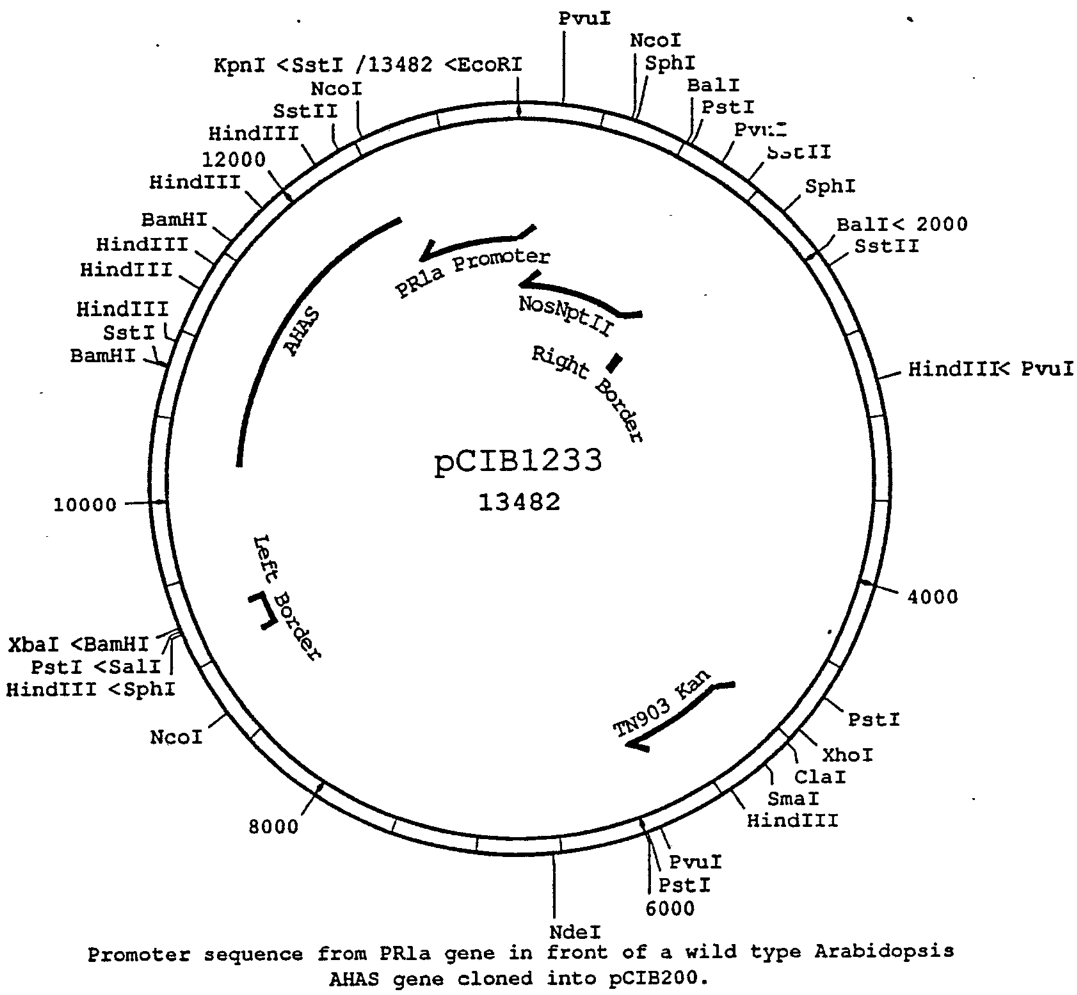


Promoter sequence from PR1a gene in front of a wild type Arabidopsis AHAS gene cloned into pCIB200.

FIGURE 21

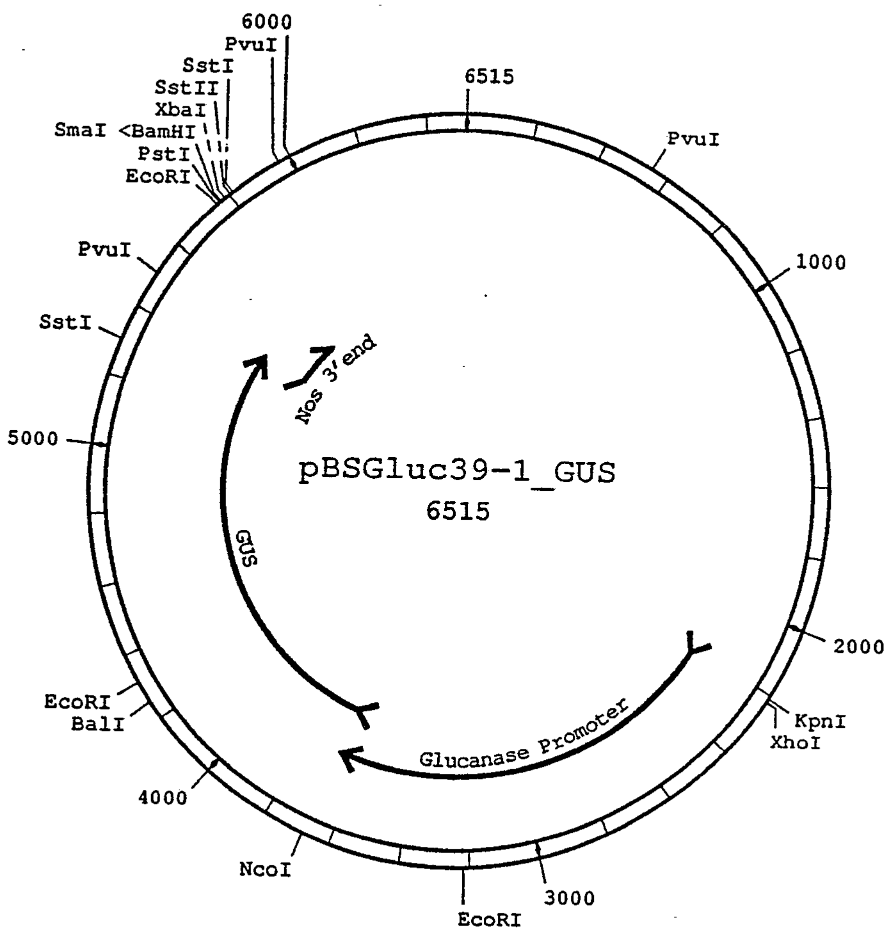
6000
PvuI
SstI
SstII
XbaI
SmaI <BamHI
PstI
EcoRI
6515
PvuI
1000
PvuI
SstI
5000
pBSGluc39-1_GUS
6515
Nos 3' end
GUS
Glucanase Promoter
2000
KpnI
XhoI
EcoRI
BalI
4000
NcoI
EcoRI
3000

FIGURE 22

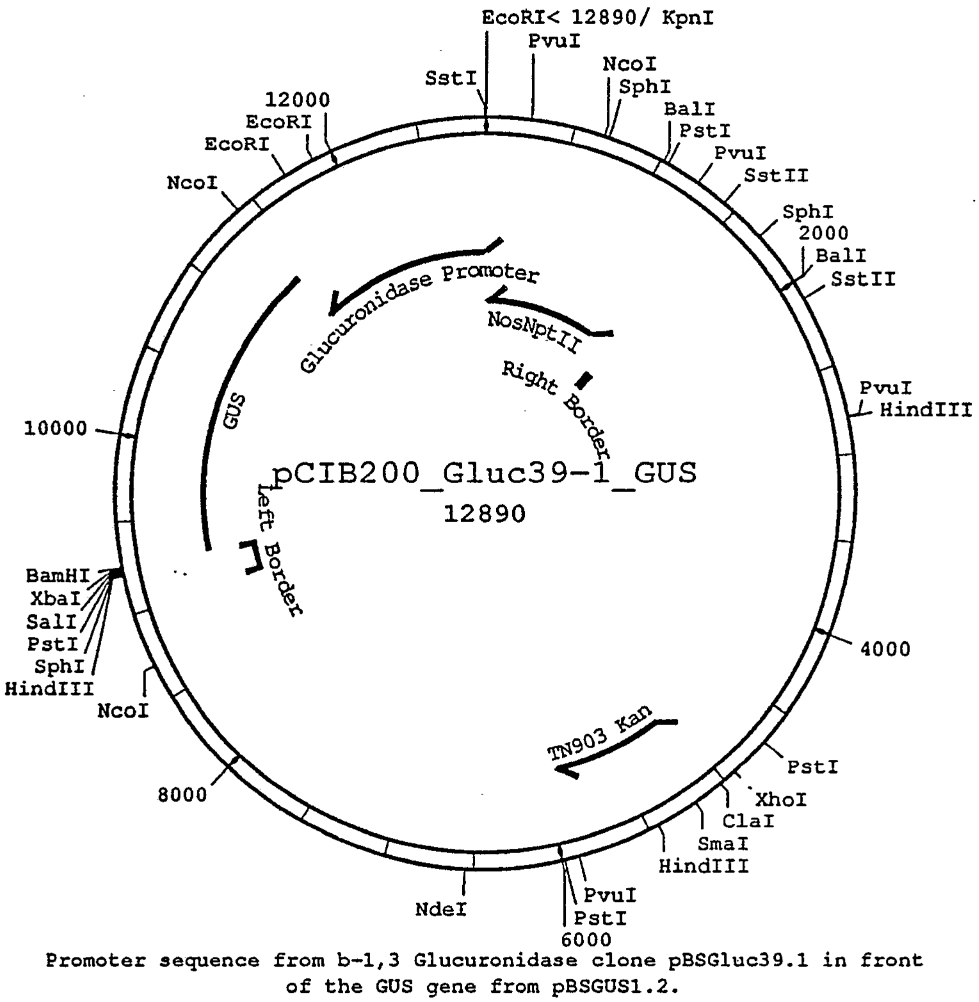


Promoter sequence from b-1,3 Glucuronidase clone pBSGluc39.1 in front of the GUS gene from pBSGUS1.2.

FIGURE 23

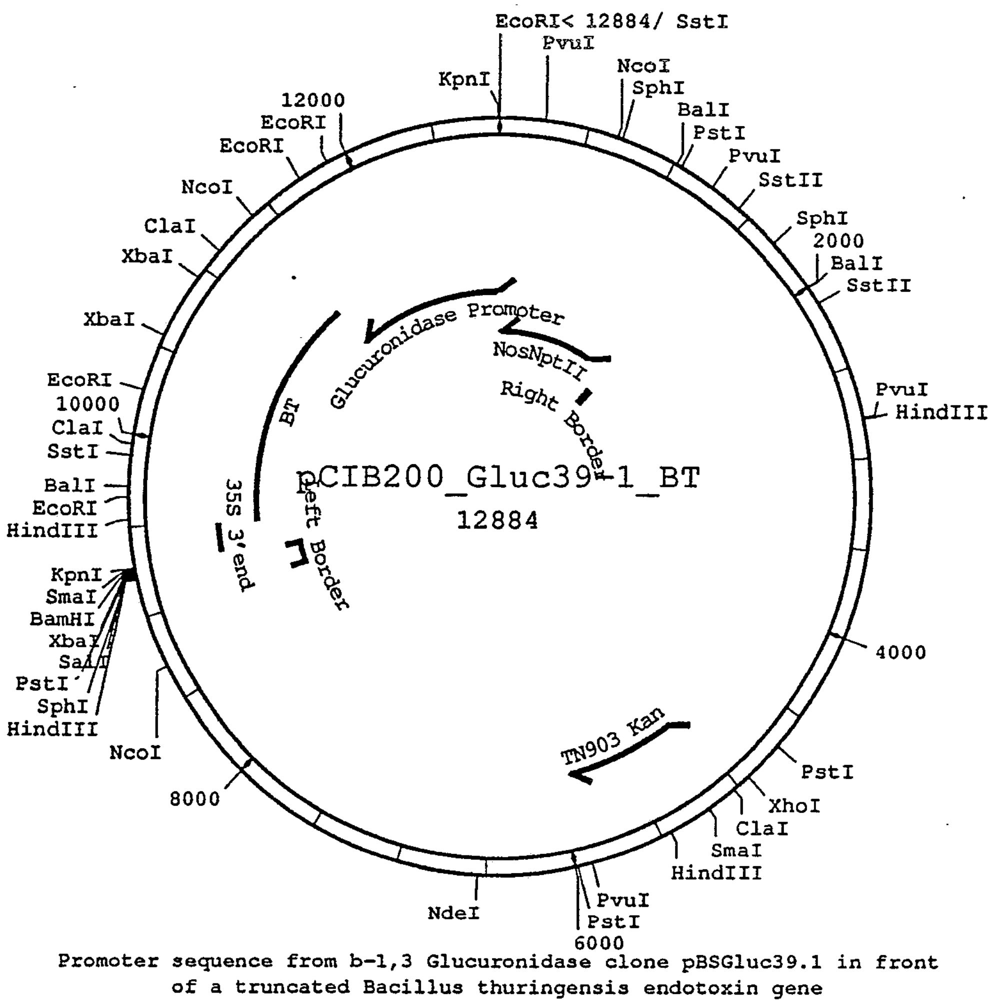


Promoter sequence from b-1,3 Glucuronidase clone pBSGluc39.1 in front of a truncated Bacillus thuringensis endotoxin gene

FIGURE 24

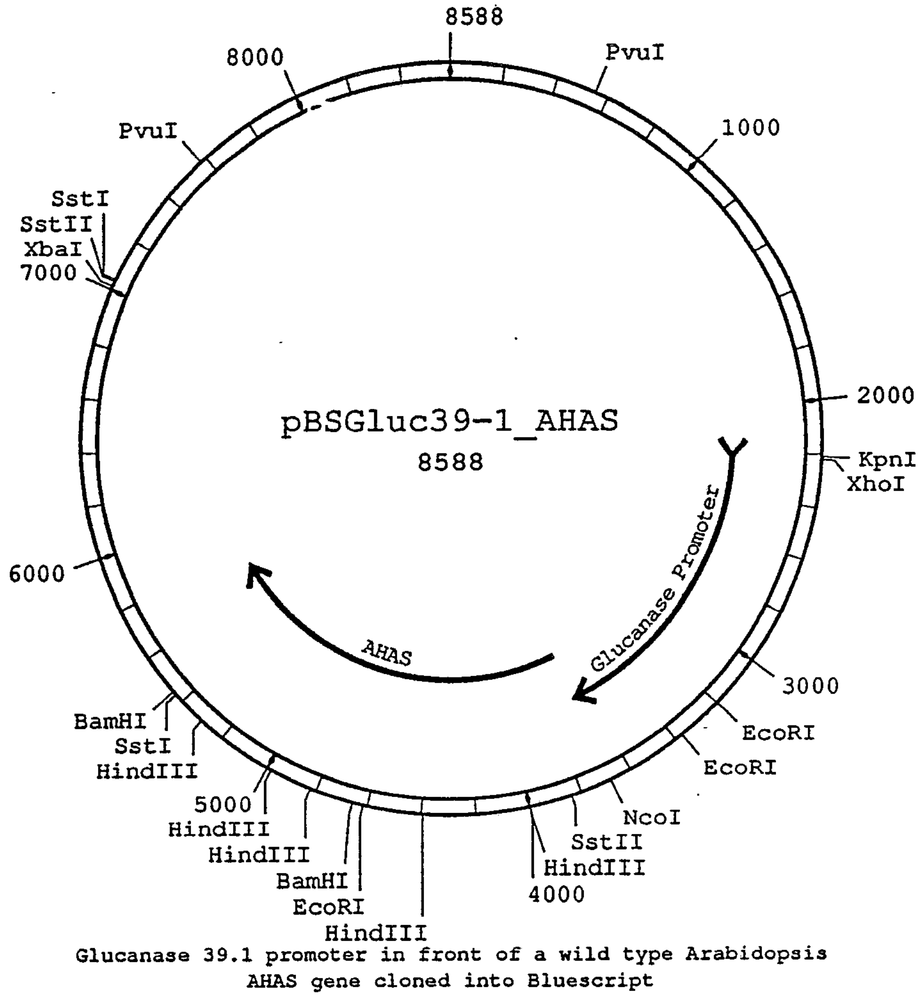


Glucanase 39.1 promoter in front of a wild type Arabidopsis AHAS gene cloned into Bluescript

FIGURE 25

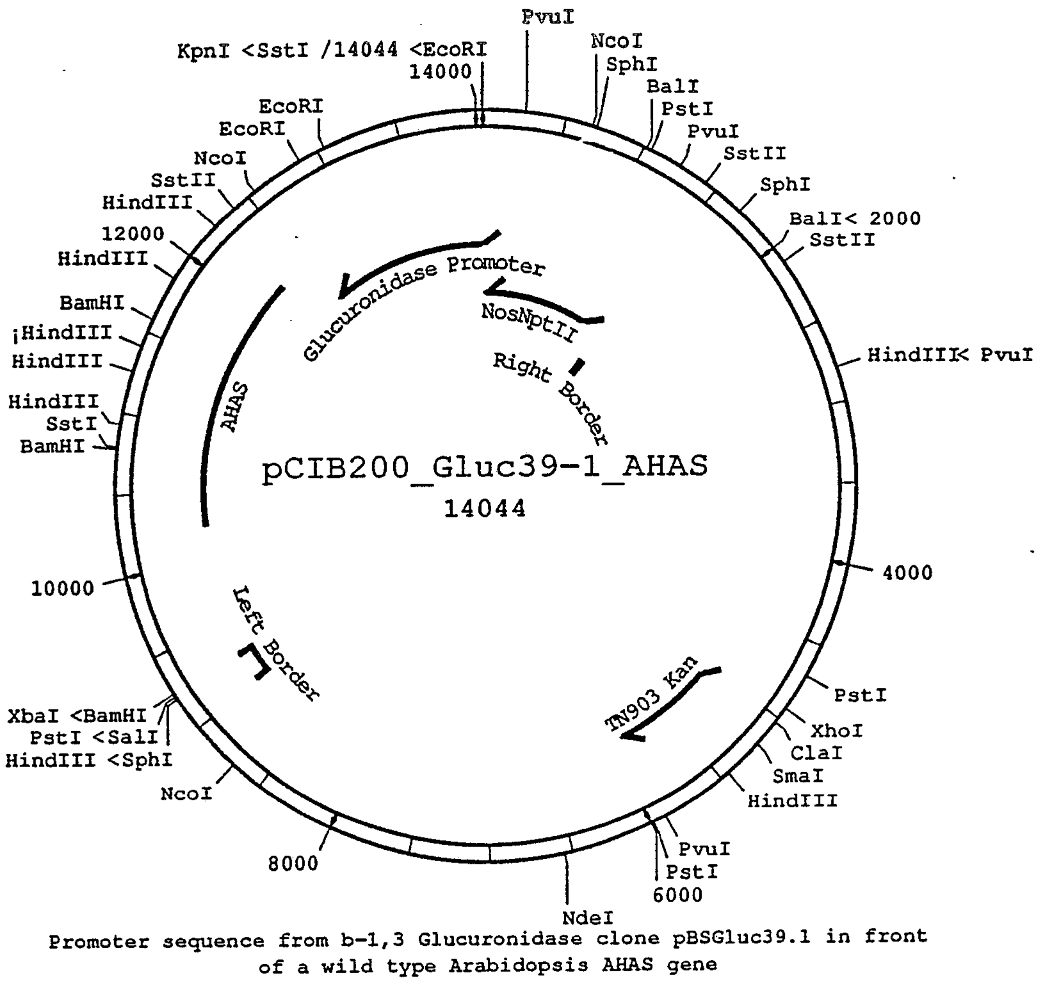


Promoter sequence from b-1,3 Glucuronidase clone pBSGluc39.1 in front of a wild type Arabidopsis AHAS gene

FIGURE 26

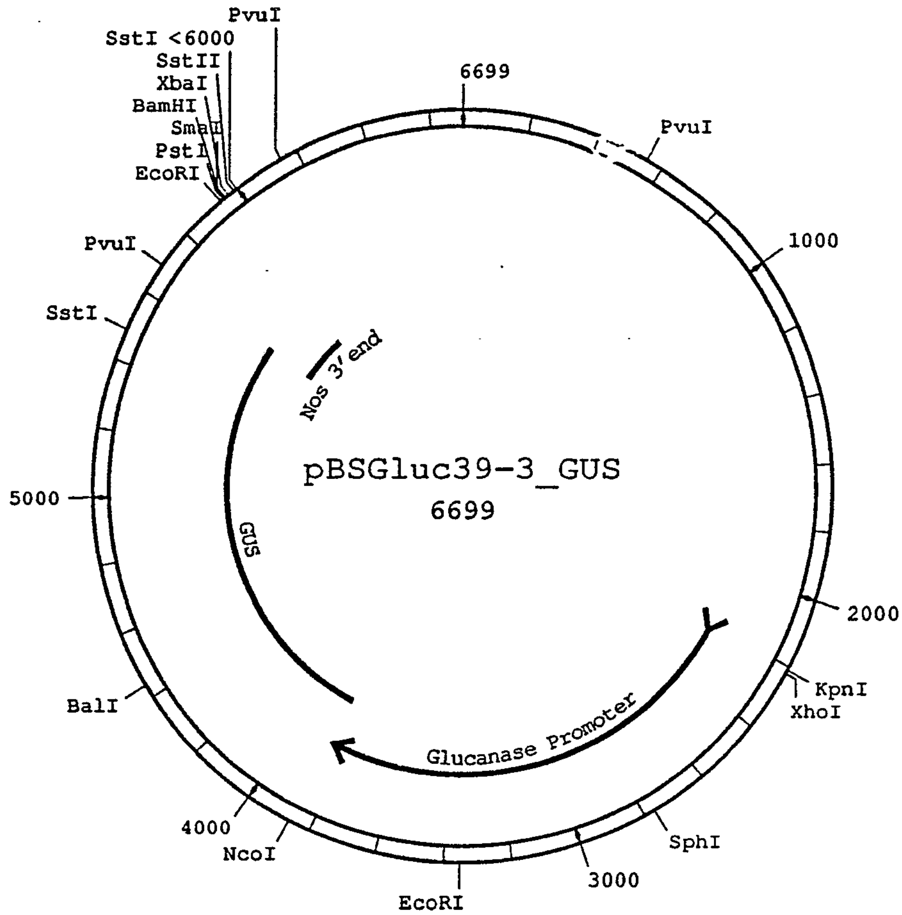
PvuI
SstI <6000
SstII
XbaI
BamHI
SmaI
PstI
EcoRI
6699
PvuI
1000
PvuI
SstI
Nos 3' end
pBSGluc39-3_GUS
6699
5000
GUS
2000
KpnI
XhoI
BalI
Glucanase Promoter
4000
NcoI
EcoRI
3000
SphI

FIGURE 27

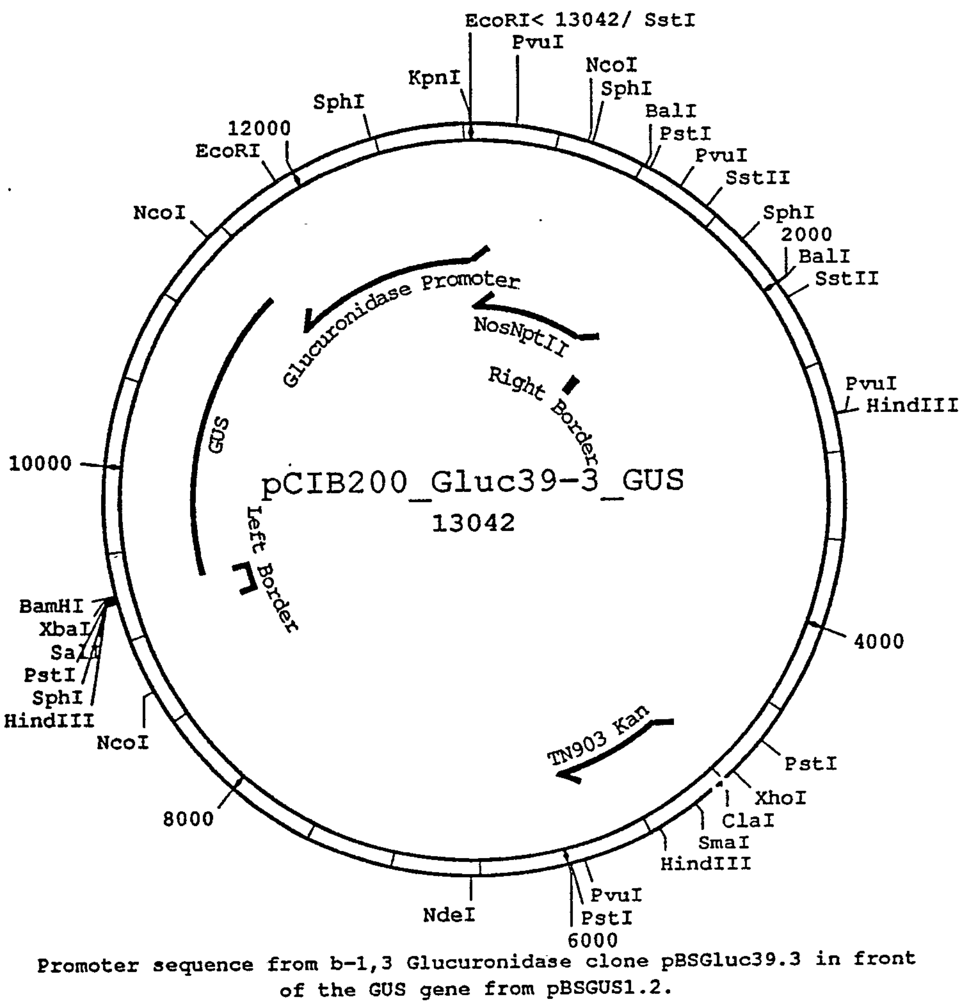

Promoter sequence from b-1,3 Glucuronidase clone pBSGluc39.3 in front of the GUS gene from pBSGUS1.2.

FIGURE 28

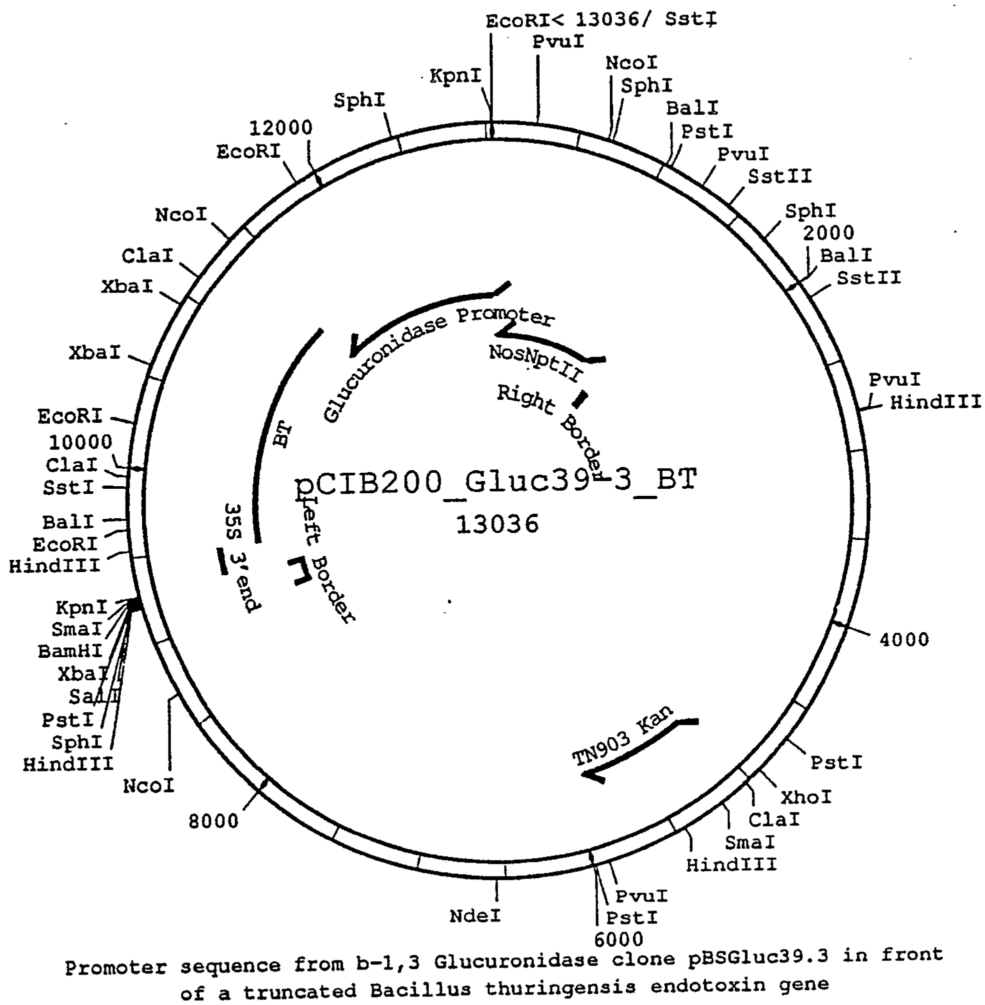


Promoter sequence from b-1,3 Glucuronidase clone pBSGluc39.3 in front of a truncated Bacillus thuringensis endotoxin gene

FIGURE 29

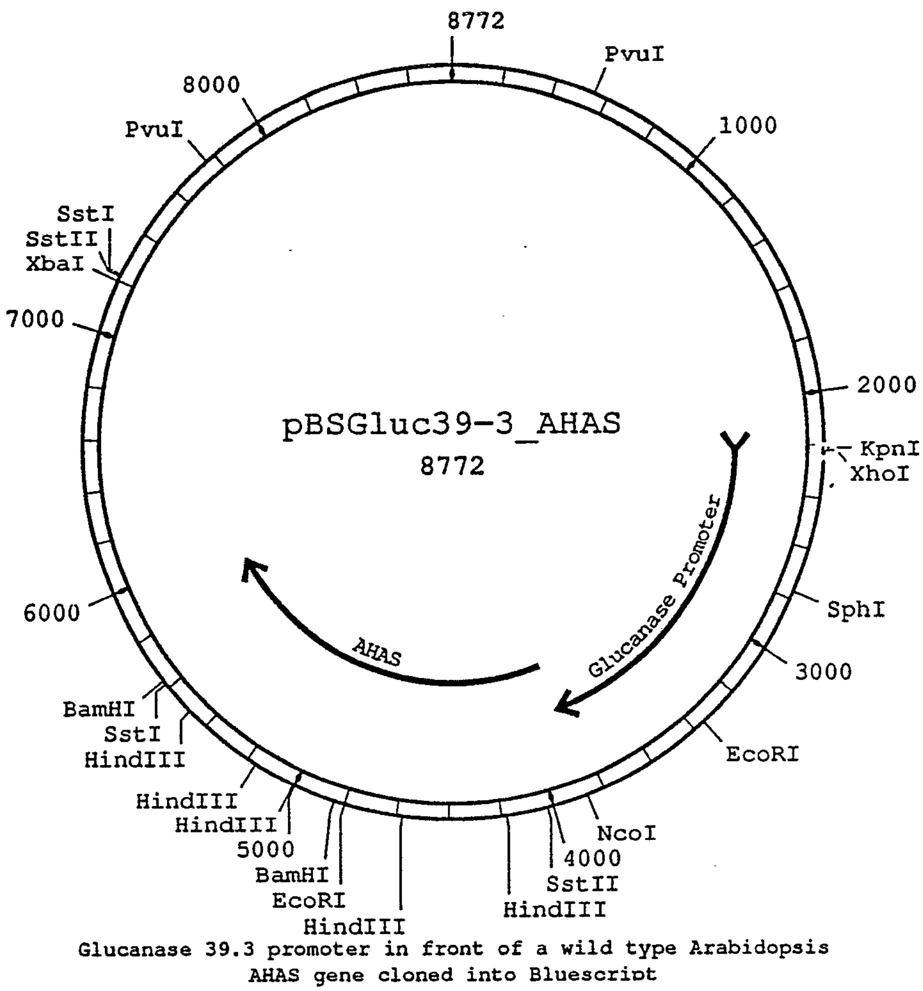


Glucanase 39.3 promoter in front of a wild type Arabidopsis AHAS gene cloned into Bluescript

FIGURE 30

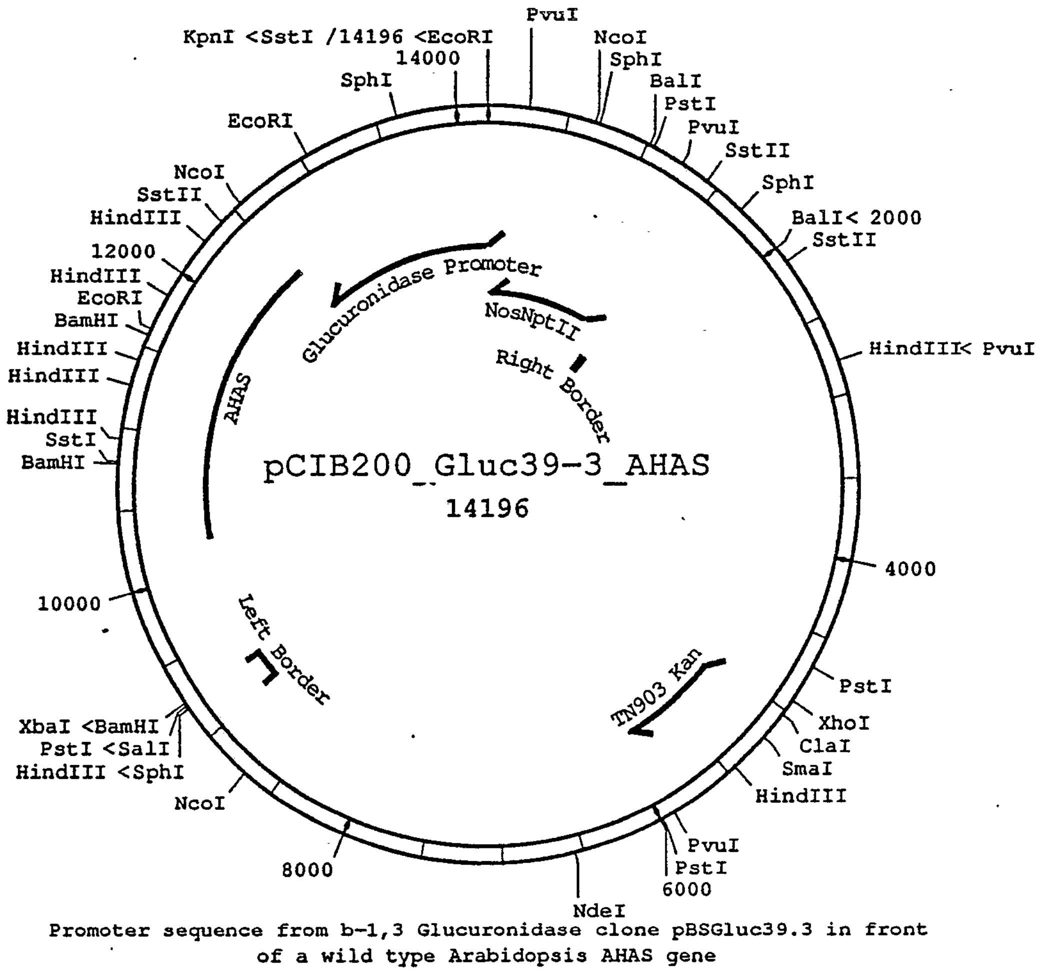


Promoter sequence from b-1,3 Glucuronidase clone pBSGluc39.3 in front of a wild type Arabidopsis AHAS gene

FIGURE 31

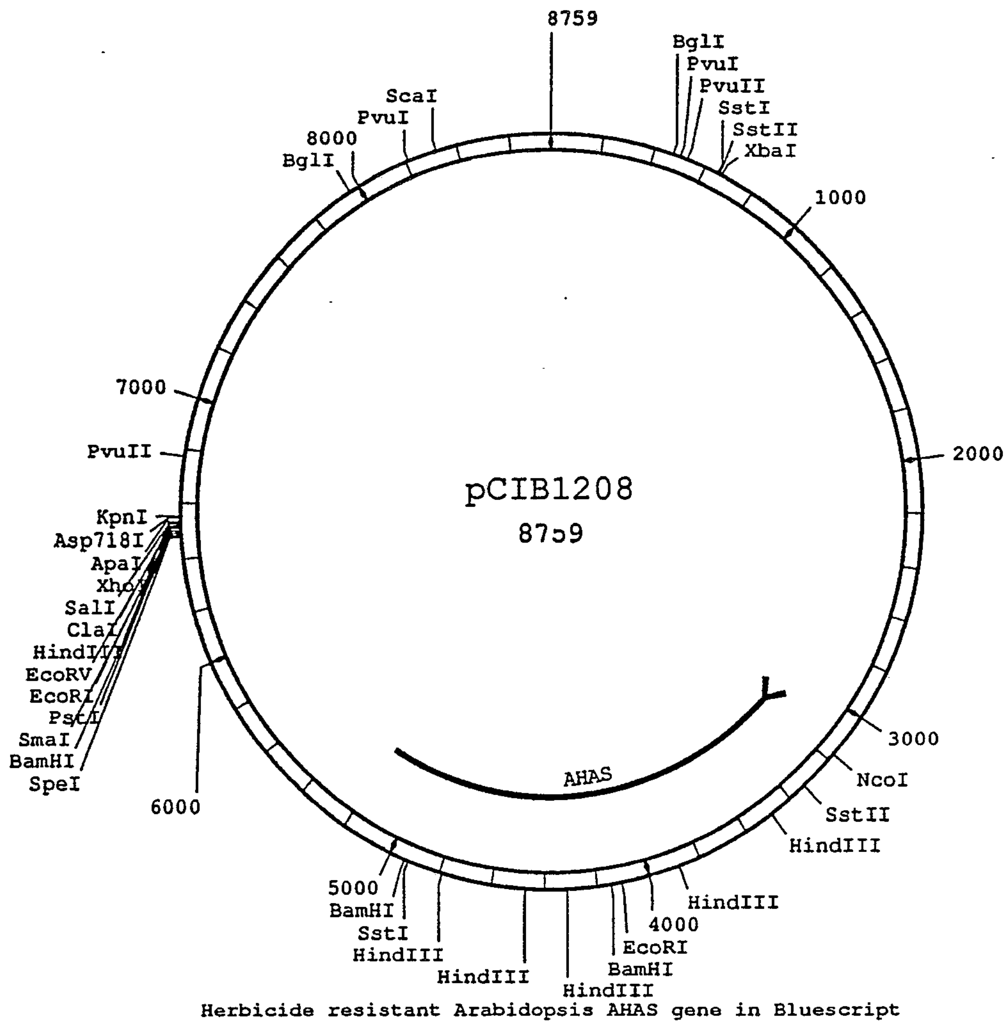

Herbicide resistant Arabidopsis AHAS gene in Bluescript

FIGURE 32

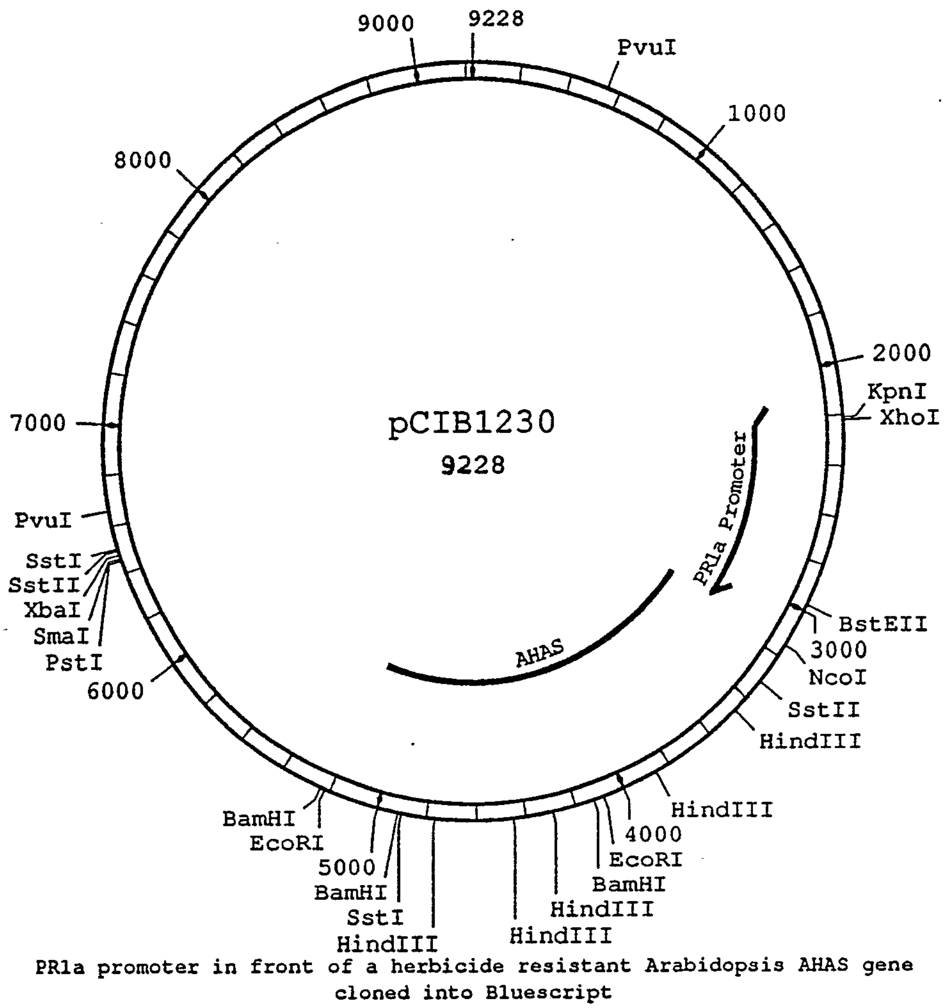


PR1a promoter in front of a herbicide resistant Arabidopsis AHAS gene cloned into Bluescript

FIGURE 33

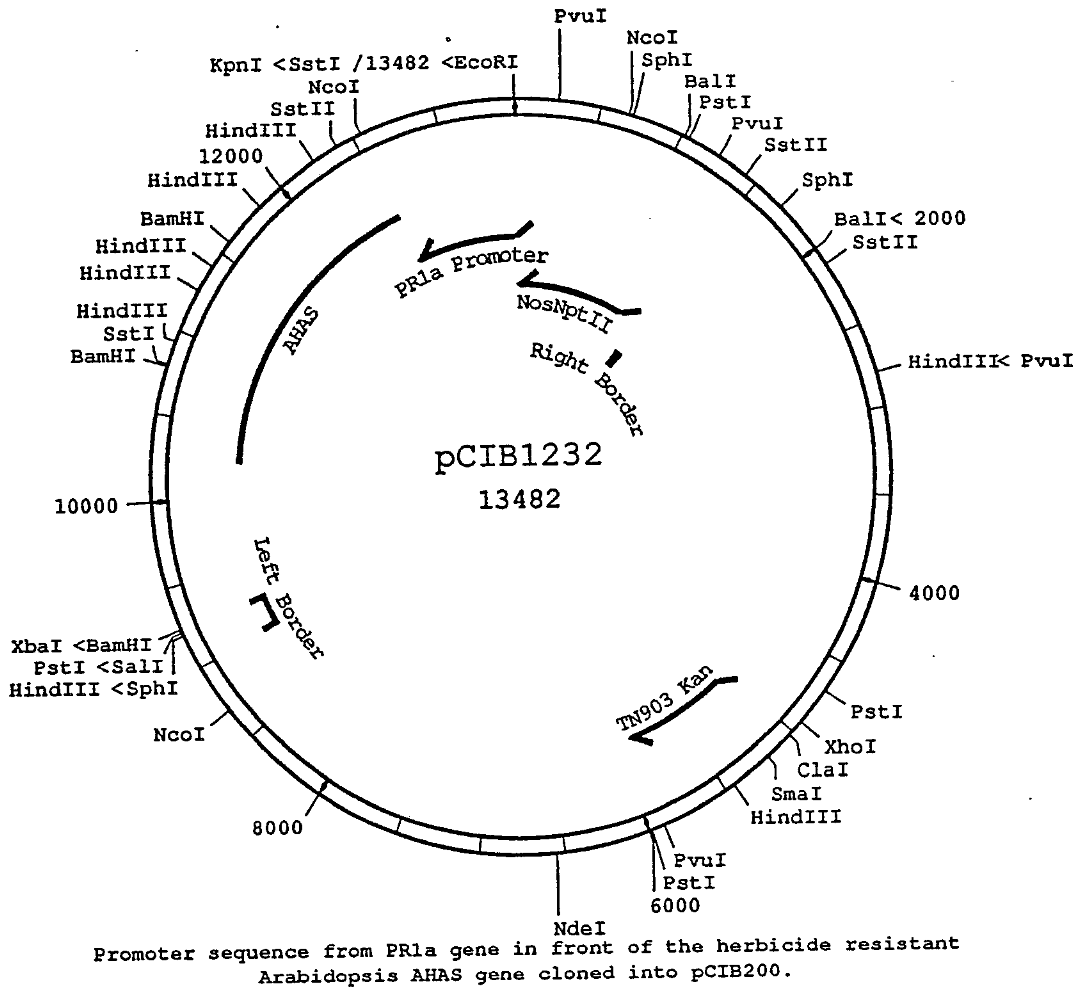


Promoter sequence from PR1a gene in front of the herbicide resistant Arabidopsis AHAS gene cloned into pCIB200.

FIGURE 34

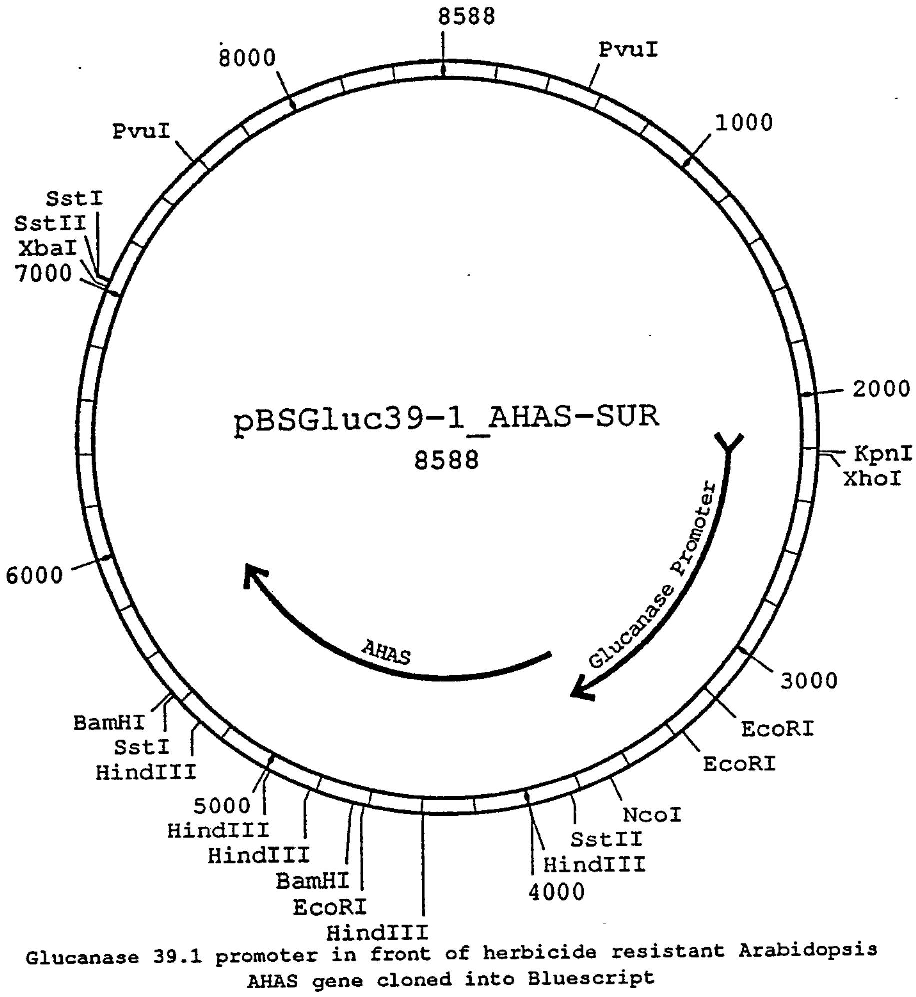


Glucanase 39.1 promoter in front of herbicide resistant Arabidopsis AHAS gene cloned into Bluescript

FIGURE 35

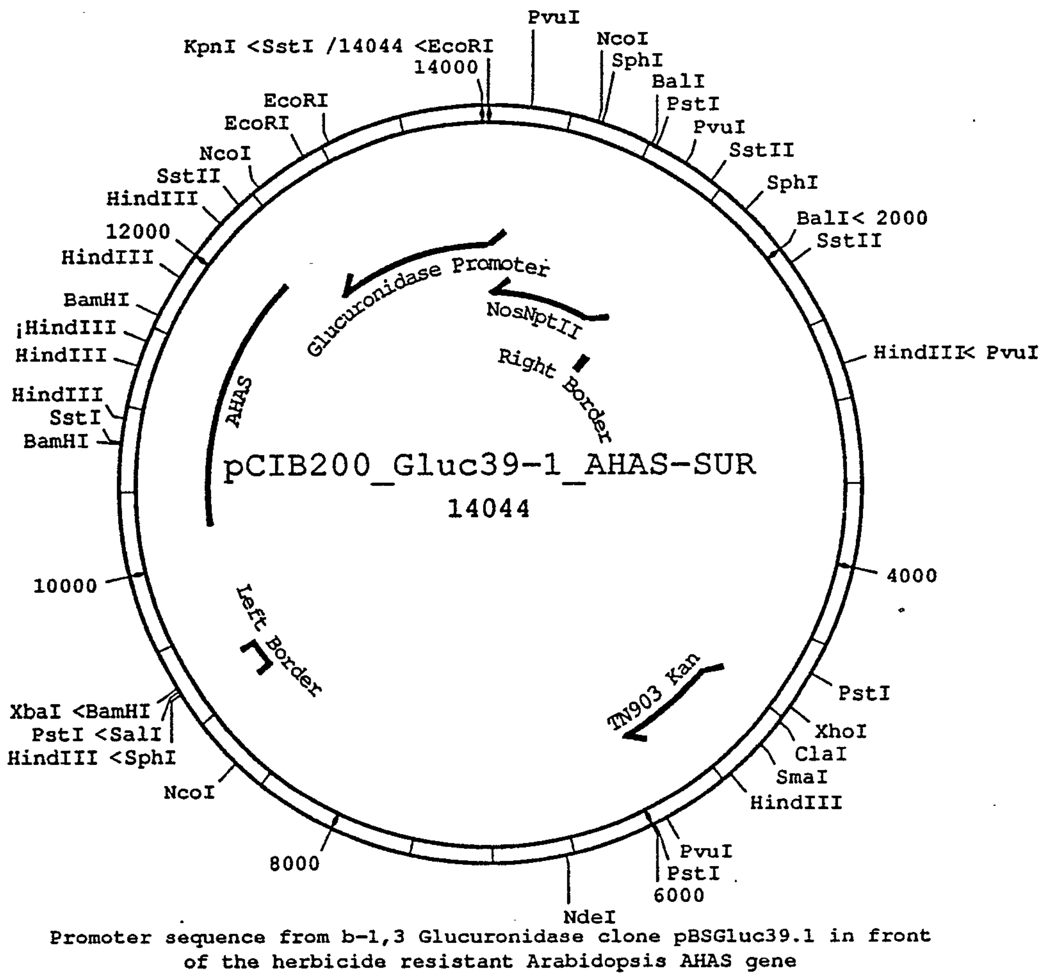


Promoter sequence from b-1,3 Glucuronidase clone pBSGluc39.1 in front of the herbicide resistant Arabidopsis AHAS gene

FIGURE 36

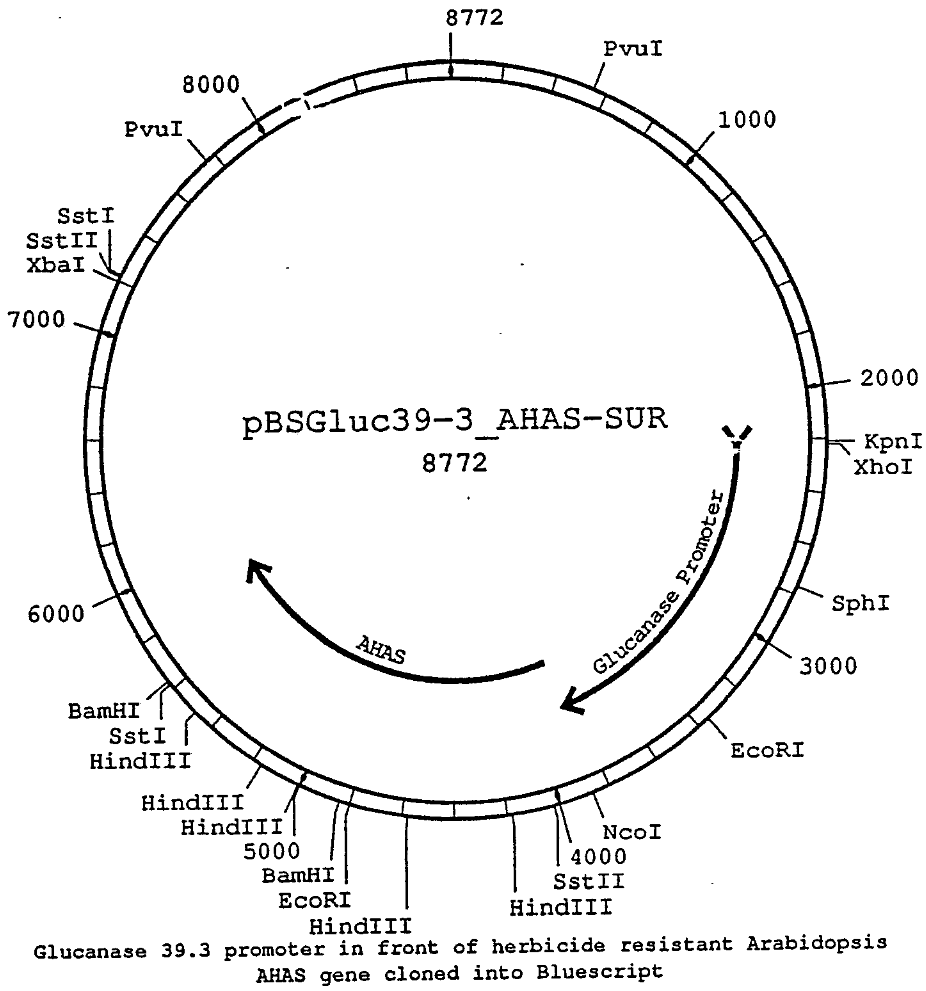


Glucanase 39.3 promoter in front of herbicide resistant Arabidopsis AHAS gene cloned into Bluescript

FIGURE 37

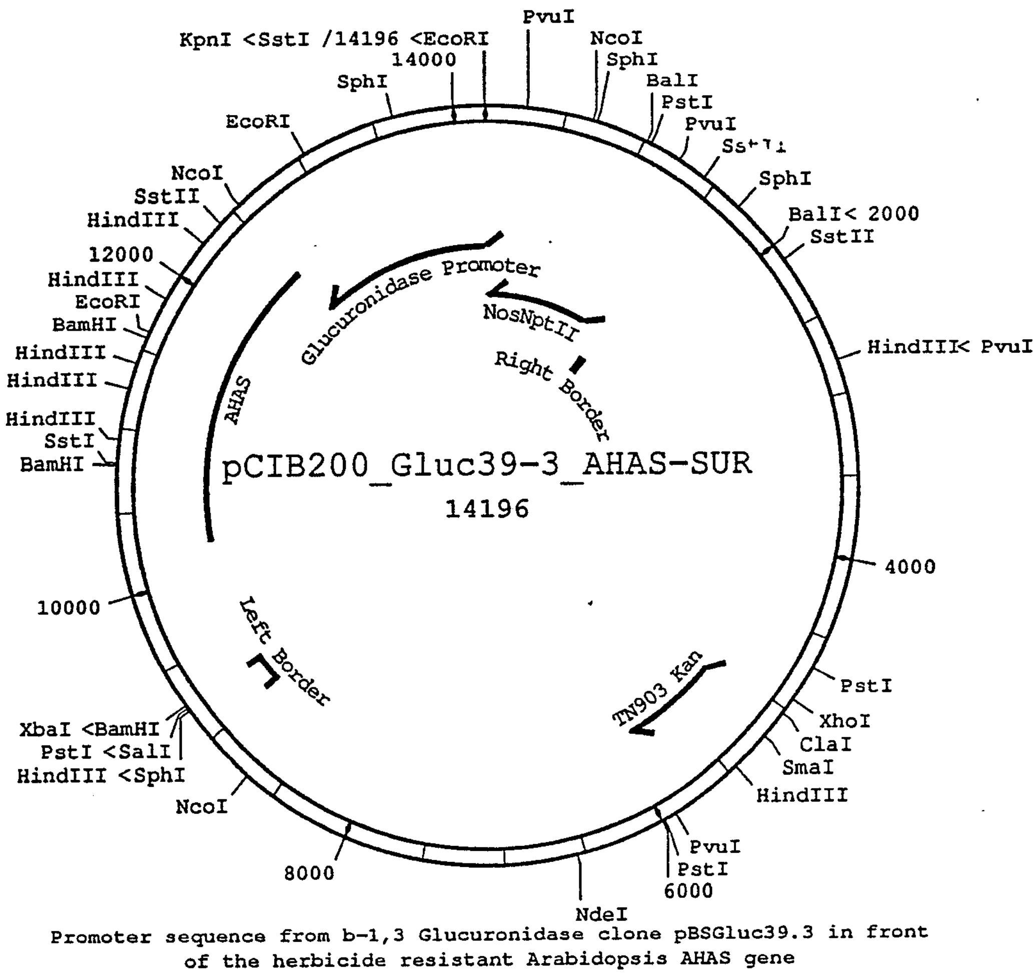


Promoter sequence from b-1,3 Glucuronidase clone pBSGluc39.3 in front of the herbicide resistant Arabidopsis AHAS gene

FIGURE 38